# FASCICULE DE BREVET EUROPEEN

(11) **EP 2 310 371 B1**
(45) Date de publication et mention de la délivrance du brevet: **15.05.2013**
(21) Numéro de dépôt: 09766058.3
(22) Date de dépôt: 26.05.2009
(51) Int. Cl.: C07D 213/64, C07D 213/70, C07D 213/74, C07D 213/643, C07D 213/69, A61K 31/44, A61P 3/10, A61P 3/06

(54) **COMPOSÉS AGONISTES PPAR, PRÉPARATION ET UTILISATIONS POUR LE TRAITEMENT DU DIABÈTE ET/OU DES DYSLIPIDÉMIES**
PPAR-AGONISTENVERBINDUNGEN UND ZUBEREITUNG UND ANWENDUNGEN DAVON ZUR BEHANDLUNG VON DIABETES UND/ODER DYSLIPIDÄMIE
PPAR AGONIST COMPOUNDS AND PREPARATION AND USES THEREOF FOR TREATING DIABETES AND/OR DYSLIPIDEMIA

(30) Priorité: 26.05.2008 FR 0853415
(43) Date de publication de la demande: 20.04.2011
(73) Titulaire: Genfit, 59120 Loos (FR)
(72) Inventeur: MASSON, Christophe, F-28200 Chateaudun (FR); CAUMONT-BERTRAND, Karine, F-59236 Frelinghien (FR)
(74) Mandataire: Tezier Herman, Béatrice
(86) Numéro de dépôt international: PCT/FR2009/050980
(87) Numéro de publication internationale: WO 2009/153496

(56) Documents cités:
- EP-A- 1 266 888
- WO-A1-2005/041959
- WO-A1-2008/066356
- WO-A2-03/084916
- US-A1- 2005 096 336

## Description

La présente invention concerne des composés à intérêt thérapeutique, destinés notamment pour le traitement du diabète et/ou des dyslipidémies. L'invention concerne également des compositions pharmaceutiques comprenant de tels composés.

Le diabète et les dyslipidémies (taux plasmatiques de cholestérol LDL et de triglycérides élevés, cholestérol HDL faible, etc.) font partie des facteurs de risque cardiovasculaire clairement identifiés qui prédisposent un individu à développer une pathologie cardiovasculaire (The Atlas of Heart Disease and Stroke, édité par Mackay J et Mensah M, publié par World Health Organization, 2004). Ces facteurs de risque s'additionnent aux facteurs de risque liés au mode de vie tels que le tabagisme, l'inactivité physique et les régimes alimentaires déséquilibrés. Un effet synergique existe entre ces différents facteurs : la présence concomitante de plusieurs d'entre eux conduit à une aggravation dramatique du risque cardiovasculaire et il convient alors de parler de risque global (« *global risk »)* pour les maladies cardiovasculaires. La prévalence des dyslipidémies atteignait 43,6% de la population en 2004 dans les principaux pays développés. La prévalence du diabète, actuellement en nette augmentation, est en passe de devenir de plus en plus significative dans l'épidémiologie des maladies cardiovasculaires. Elle est estimée à 7,6% de la population pour 2010 (Fox-Tucker J, The Cardiovascular Market Outlook to 2010, Business Insights Reports, 2005).

Selon l'International Atherosclerosis Society, les maladies cardiovasculaires représentent la première cause de mortalité dans les pays industrialisés et deviennent de plus en plus fréquentes dans les pays en voie de développement. Ces maladies sont notamment les maladies coronariennes, l'ischémie cérébrale et les maladies artérielles périphériques. Ces données justifient l'adoption de mesures énergiques pour réduire significativement la morbidité et la mortalité cardiovasculaires. Egalement, la nécessité de trouver des traitements efficaces, apte à agir sur les facteurs de risque des maladies cardiovasculaires et sur leurs conséquences, devient une urgence mondiale, aussi par rapport aux récents résultats décevants des candidatats médicaments (Krause B, 2008).

Entre les différents récepteurs nucléaires qui peuvent être cibles thérapeutiques (Hansen MK et Connolly TM, 2008), l'implication des Peroxysome Proliferator-Activated Receptors (PPARs) dans ce type de pathologies est aujourd'hui très bien établie (Blaschke F et al., 2006 ; Gilde AJ et al., 2006 ; Gervois P et al., 2007). La famille des PPARs comprend trois isoformes, désignés α, γ et δ (encore appelé β), chacun codé par un gène différent. Ces récepteurs, qui font partie de la superfamille des récepteurs nucléaires et des facteurs de transcription, ont un rôle majeur dans la régulation du métabolisme des lipides et des glucides.

PPARα contrôle le métabolisme lipidique (hépatique et musculaire) et l'homéostasie du glucose, et influence le métabolisme intracellulaire des lipides et des sucres par un contrôle direct de la transcription de gènes codant pour des protéines impliquées dans l'homéostasie lipidique. PPARα exerce également des effets anti-inflammatoires et anti-prolifératifs et prévient les effets pro-athérogéniques de l'accumulation du cholestérol dans les macrophages en stimulant l'efflux du cholestérol (Lefebvre P et al., 2006). PPARγ est un régulateur-clé de l'adipogénèse. Il est aussi impliqué dans le métabolisme lipidique des adipocytes matures, dans l'homéostasie du glucose, dans la résistance à l'insuline, dans l'inflammation, dans l'accumulation de cholestérol au niveau des macrophages et dans la prolifération cellulaire (Lehrke M and Lazar MA, 2005). PPARγ joue par conséquent un rôle dans la pathogénèse de l'obésité, de l'insulino-résistance et du diabète. PPARδ est impliqué dans le contrôle du métabolisme lipidique et glucidique, dans la balance énergétique, dans la neurodégénération, dans l'obésité, dans la formation des cellules spumeuses et dans l'inflammation (Gross B et al., 2005).

Ces multiples propriétés font des PPARs des cibles thérapeutiques d'intérêt pour le traitement du diabète et des dyslipidémies, et pour la prévention des maladies cardiovasculaires. Des ligands de PPARs sont déjà connus, certains sont commercialisés et prescrits dans le traitement de certaines des pathologies précédemment évoquées, et leur toxicologie a été étudié (Peraza M et al., 2006). On peut citer des activateurs de PPARα, comme les fibrates (fénofibrate, bézafibrate, ciprofibrate, gemfibrozil), qui sont utilisés en clinique pour traiter certaines dyslipidémies en augmentant les taux plasmatique de HDL (High Density Lipoprotein) et en baissant les triglycérides (Hourton D et al. 2001). Par ailleurs, les thiazolidinediones (Rosiglitazone et Pioglitazone), des ligands de PPARγ, sont utilisés dans le traitement du diabète de type 2. On connaît aussi des ligands de PPARδ (comme L-165041, GW501516 et KD3010). Parmi les documents de l'art antérieur mentionnant des composés proches, les demandes de brevets WO 03/084916, WO 08/152333, WO 05/041959, WO 08/066356, EP 1266888, et US 2005096336 décrivent des agonistes les récepteurs PPAR.

L'invention vise à proposer de nouveaux composés agonistes de PPAR (PPARα et/ou PPARγ et/ou PPARδ), en particulier qui sont adaptés au traitement thérapeutique et/ou prophylactique, du diabète, des dyslipidémies, de l'insulino-résistance, des pathologies associées au syndrome métabolique, de l'athérosclérose, de l'obésité, de l'hypertension et/ou des maladies inflammatoires. Ces composés agonistes PPAR peuvent être aussi particulièrement efficaces pour réduire le risque cardiovasculaire, et prévenir les maladies cardiovasculaires, notamment celles liées aux dérèglements du métabolisme lipidique et/ou glucidique.

Ces buts et d'autres sont atteints par des composés de Formule Générale (I) suivante : 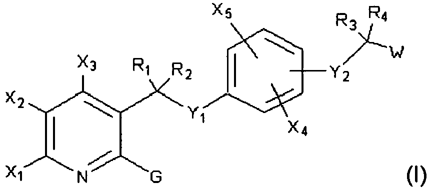 dans laquelle,
G représente :
   - un radical -ORₐ, -SRₐ ; ou
   - un radical -NRₐR_{b} ;
      Rₐ étant choisi parmi un radical alkyle de 1 à 6 atomes de carbone ou alkényle de 2 à 6 atomes de carbone, un cycle de 3 à 14 atomes, un radical phényle, un radical phénylalkyle de partie alkyle ayant 1 à 3 atomes de carbone ;
      R_{b} étant choisi parmi un atome d'hydrogène, un radical alkyle de 1 à 6 atomes de carbone ou alkényle de 2 à 6 atomes de carbone, un cycle de 3 à 14 atomes, un radical phényle, ou un radical phénylalkyle de partie alkyle ayant 1 à 3 atomes de carbone ;
      Rₐ et R_{b} pouvant former, ensemble et avec l'atome d'azote auquel ils sont liés, un hétérocycle de 3 à 8 atomes ;
R₁ et R₂, identiques ou différents, représentent un atome d'hydrogène ou un radical alkyle de 1 à 6 atomes de carbone ou alkényle de 2 à 6 atomes de carbone ;
R₁ et R₂ pouvant former, ensemble et avec l'atome de carbone auquel ils sont liés, un carbocycle de 3 à 6 atomes de carbone ;
Y₁ représente :
   - un atome d'oxygène ou de soufre, ou
   - un groupe -NR-, dans lequel R a la même définition que R_{b};
Y₂ représente :
   - un atome d'oxygène ou de soufre, ou
   - un radical -CR₅R₆- ; avec R₅ et R₆, identiques ou différents, choisis parmi un atome d'hydrogène ou d'halogène, un radical alkyle de 1 à 6 atomes de carbone ou un radical alkényle ou alkynyle de 2 à 6 atomes de carbone, un cycle de 3 à 6 atomes, un radical phénylalkyle de partie alkyle ayant 1 à 3 atomes de carbone,
X₁, X₂, X₃ représentent indépendamment un atome d'hydrogène ou d'halogène, un radical alkyle de 1 à 6 atomes de carbone ou alkényle de 2 à 6 atomes de carbone, un groupement -OR'ₐ, -SR'ₐ, -NR'ₐR'_{b}, un cycle de 5 à 14 atomes, ou un radical phénylalkyle de partie alkyle ayant 1 à 3 atomes de carbone,
avec au moins un des groupements X₁, X₂ et X₃ différent d'un atome d'hydrogène et d'un atome d'halogène,
R'ₐ et R'_{b}, identiques ou différents, ayant les mêmes définitions que Rₐ et R_{b};
X₄ et X₅ représentent indépendamment un atome d'hydrogène ou d'halogène, un radical alkyle de 1 à 6 atomes de carbone ou alkényle de 2 à 6 atomes de carbone, un groupement -OR"ₐ, -SR"ₐ ou -NR"ₐR"_{b}, un cycle de 3 à 14 atomes, un radical phényle, ou un radical phénylalkyle de partie alkyle ayant 1 à 3 atomes de carbone ;
R"ₐ et R"_{b}, identiques ou différents, ayant les mêmes définitions que Rₐ et R_{b};
R₃ et R₄, identiques ou différents, représentent un atome d'hydrogène ou d'halogène, un radical alkyle de 1 à 6 atomes de carbone ou alkényle de 2 à 6 atomes de carbone, un cycle de 3 à 14 atomes, un radical phényle, ou un radical phénylalkyle de partie alkyle ayant 1 à 3 atomes de carbone ;
W représente :
   - un radical carboxyle -COOH ; ou
   - une fonction dérivée de la fonction acide carboxylique, choisie parmi -COOR"'ₐ, -COSR"'ₐ, -CONR"'ₐR"'_{b}, -CSNR"'ₐR"'_{b}, -CONH₂ ; ou
   - un groupement bioisostère du radical carboxyle, choisi parmi :
      - un radical acylsulfonamide (-CONHSO₂R"'ₐ) ;
      - un radical hydrazide (-CONHNR'"ₐR'"_{b});
      - un radical choisi parmi les cycles thiazolidinedione, oxazolidinedione, tétrazole, oxadiazolone, triazolone, triazole, 3-alkyltriazole, ou imidazolidinedione ;
R"'ₐ et R"'_{b}, identiques ou différents, ayant les mêmes définitions que Rₐ et R_{b}.

Dans le cadre de la présente invention, les définitions suivants sont applicables :
- Par radicaux alkyle, alkényle ou alkynyle de n atomes de carbone, on entend selon l'invention des radicaux hydrocarbonés, linéaires, saturés ou insaturés, ramifiés ou non ramifiés, formés de n atomes de carbone au total (atome de carbones de la chaîne principale et atome de carbone des ramifications), comprenant de 1 à 12 atomes de carbone et, plus particulièrement, de 1 à 6 atomes de carbone. Cette définition inclut également les radicaux alkyles, alkényle ou alkynyle substitués par un ou des atomes d'halogènes. Pour les radicaux alkyles de 1 à 6 atomes de carbone, on entend de préférence le radical méthyle, éthyle, n-propyle, isopropyle, n-butyle, isobutyle, tertiobutyle, sec-butyle, pentyle, néopentyle, n-hexyle ou cyclohexyle. Par radical alkyle ayant de 7 à 12 atomes de carbone, on entend de préférence le radical octyle, décyle, ou dodécyle. Par radical alkényle de 1 à 6 atomes de carbone, on entend les radicaux hydrocarbonés présentant au moins une double liaison entre deux atomes de carbone, de type -CH=CH-, par exemple le radical éthényle, propén-1-yle, propén-2-yle, butén-1-yle, butèn-2-yle, pentèn-1-yle, pentèn-2-yle, 3-méthyl-butèn-2-yle. Par radical alkynyle de 1 à 6 atomes de carbone, on entend les radicaux hydrocarbonés présentant au moins une triple liaison entre deux atomes de carbone, de type -C=C-, par exemple le radical éthynyle, propyn-1-yle, propyn-2-yle, butyn-1-yle, butyn-2-yle, pentyn-1-yle ou pentyn-2-yle.
- Par atome d'halogène, on entend un atome de fluor, de chlore, de brome, ou d'iode.
- Par « cycle de n atomes », on entend selon l'invention des radicaux mono- ou polycycliques dont la partie cyclique est formée par n atomes au total (en l'occurrence, n = 3, 4, 5, 6, 7, 8, 9, 10, 11, 12, 13 ou 14) et de preférence comprenant de 3 à 7 atomes de carbone. Ce radical cyclique peut être saturé ou insaturé, éventuellement aromatique. Il peut s'agir d'un carbocycle, c'est-à-dire un cycle dont le corps cyclique est formé exclusivement d'atomes de carbone. Il peut également s'agir d'un hétérocycle; auquel cas, au moins un des atomes du corps cyclique est un hétéroatome, tel que l'azote, l'oxygène ou le soufre. Cette définition des cycles selon l'invention, y compris le phényl, inclut notamment les cycles substitués par un ou plusieurs atomes d'halogène (et/ou par une ou plusieurs fonctions hydroxyle, thiol, cyano, nitro, et/ou par un ou plusieurs radicaux alkyle, alkényle, alkyloxy, alkylthio, ayant 1 à 6 atomes de carbone, et/ou par un ou plusieurs radicaux phényle ou phénylalkyle de partie alkyle ayant 1 à 3 atomes de carbone, lesdits radicaux pouvant eux-mêmes être halogénés (comme les perfluoroalkyles, par exemple -CF₃) et/ou substitués par des groupements alkyle, alkényle, alkyloxy, alkylthio, et/ou par des fonctions hydroxyle, cyano, thiol, nitro. Comme exemples on peut citer :

- des carbocycles saturés tels que cyclopropyle, cyclobutyle, cyclopentyle, cyclohexyle, norbornyle, adamantyle, ou cycloheptyle ;
- des carbocycles insaturés, aromatiques ou partiellement aromatiques, tels que cyclobutadiène, benzène (ou groupe phényl), pentalène, heptalène, naphtalène, ou anthracène ; parmi les groupes carbocycliques aromatiques, le groupe phényle ou naphtyle, substitué ou non, est tout particulièrement préféré ;
- des hétérocycles saturés tels que pyrrolidine, dioxane, morpholine, pipéridine, pipérazine, 2-oxo-pipéridine, ou 2-oxo-pyrrolidine ;
- hétérocycles insaturés, aromatiques ou non aromatiques, tels que pyridine, furane, pyrane, pyrrole, thiophène, isoxazole, oxadiazole, oxazole, benzimidazole, indole, benzofuranne, hexaméthylamine, tétrazole, indoline, isoindole, isoindoline, benzothiophène, quinoline, ou imidazole.
- Les termes « alkyloxy » et « alkylthio » font référence respectivement à une chaîne alkyle liée au reste de la molécule par l'intermédiaire d'un atome d'oxygène (liaison éther) ou à une chaîne alkyle liée au reste de la molécule par l'intermédiaire d'un atome de soufre (liaison thioéther). Le terme « alkyle » répond à la définition précédemment énoncée. A titre d'exemple d'alkoxy, on peut citer les radicaux méthoxy, éthoxy, n-propyloxy, isopropyloxy, n-butyloxy, isobutyloxy, tertio-butyloxy, sec-butyloxy ou hexyloxy. A titre d'exemple d'alkylthio, on peut citer les radicaux méthylthio, éthylthio, n-propylthio, isopropylthio, n-butylthio, isobutylthio, tertio-butylthio, sec-butylthio ou hexylthio.
- Le terme « phénylalkyle » désigne un radical du type alkyle substitué par un groupement phényle, le radical alkyle étant tel que défini ci-dessus. Cette définition inclut notamment les radicaux phénylalkyle dont le groupement phényle est substitué par un ou plusieurs atomes d'halogène et/ou par un ou plusieurs radicaux alkyle de 1 à 6 atomes de carbone ou alkényle de 2 à 6 atomes de carbone, éventuellement eux-mêmes halogénés.
- La notion de « bioisostères du radical carboxyle » fait référence à des groupements chimiques fonctionnellement/biologiquement équivalents à un radical carboxyle (-COOH), c'est-à-dire qu'ils peuvent remplacer le radical carboxyle d'un composé sans modifier de façon significative l'activité biologique globale dudit composé. Les groupements bioisostères sont en général utilisés pour améliorer l'efficacité, la sélectivité, la stabilité, ou la pharmacocinétique des molécules. De nombreux groupements bioisostères du radical carboxyle sont connus et sont largement décrits dans la littérature (Burger A, 1991 ; Lima LM and Barreiro EJ, 2005). C'est le cas notamment des groupements suivants :

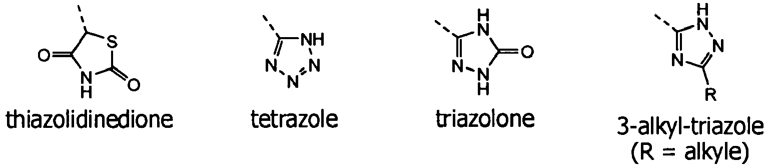

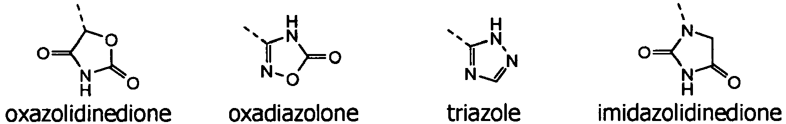

Le radical alkyle R du triazole peut être un radical alkyle tel que défini précédemment.
- La notion de « fonctions dérivées de la fonction acide carboxylique » fait référence à des fonctions sensibles à l'hydrolyse (notamment à l'hydrolyse enzymatique) connu de l'homme de l'art comme étant des précurseurs de la fonction acide carboxylique. Ces fonctions sont largement utilisées pour modifier les propriétés pharmacocinétiques des molécules actives carboxylées. Il s'agit notamment des esters, thioesters, amides et thioamides.

De manière générale, l'invention concerne des composés répondant à la Formule Générale (I) telle précédemment définie, dans laquelle, préférentiellement :
- G est choisi parmi les radicaux méthoxy, éthoxy, *n*₋propyloxy, isopropyloxy, *n*-butyloxy, tertio-butyloxy, sec-butyloxy, *n*-pentyloxy, cyclopentyloxy, *n*-hexyloxy, cyclohexyloxy, phénoxy, méthylamine, diméthylamine, éthylamine, diéthylamine, *n*-propylamine, dipropylamine, isopropylamine, diisopropylamine, *n*-butylamine, dibutylamine, tertio-butylamine, n-hexylamine, dihexylamine, pipéridine, pyrolidine, aniline, méthylthio, éthylthio, *n*-propylthio, isopropylthio, *n*-butylthio, tertio-butylthio, *sec*-butylthio, *n-*pentylthio, cyclopentylthio, *n*-hexylthio, cyclohexylthio, thiophénol ; et/ou

- X₁, X₂, X₃, X₄ et X₅ sont indépendamment choisis parmi l'atome d'hydrogène, les radicaux méthoxy, éthoxy, *n*₋propyloxy, isopropyloxy, *n*-butyloxy, tertio-butyloxy, *sec-*butyloxy, *n*-hexyloxy, cyclohexyloxy, phénoxy, méthylamine, diméthylamine, éthylamine, diéthylamine, *n*-propylamine, dipropylamine, isopropylamine, diisopropylamine, *n*-butylamine, dibutylamine, tertio-butylamine, *n*-hexylamine, dihexylamine, pipéridine, pyrolidine, aniline, méthylthio, éthylthio, *n*-propylthio, isopropylthio, *n*-butylthio, tertio-butylthio, *sec*-butylthio, *n*-pentylthio, cyclopentylthio, *n-*hexylthio, cyclohexylthio, thiophénol, phényle, benzyle, phénéthyle, 2-méthylphényle, 3-méthylphényle, 4-méthylphényle, 2-méthoxyphényle, 3-méthoxyphényle, 4-méthoxyphényle, 2-fluorophényle, 3-fluorophényle, 4-fluorophényle, 2-chlorophényle, 3-chlorophényle, 4-chlorophényle, 2-bromo-phényle, 3-bromophényle, 4-bromophényle, 2-iodophényle, 3-iodophényle, 4-iodo-phényle, 2-(trifluorométhyl)phényle, 3-(trifluorométhyl)phényle, 4-(trifluorométhyl)phényle, 2-(trifluorométhoxy)phényle, 3-(trifluorométhoxy)phényle, 4-(trifluorométhoxy)phényle, 2,6-diméthylphényle, 3,6-diméthylphényle, 4,6-diméthylphényle, 5,6-di-méthylphényle, 2,6-fluorophényle, 3,6-fluorophényle, 4,6-fluorophényle, 5,6-fluoro-phényle, 2,6-dichlorophényle, 3,6-dichlorophényle, 4,6-dichlorophényle, 5,6-di-chlorophényle, 2,6-bromophényle, 3,6-bromophényle, 4,6-bromophényle, 5,6-bromophényle, 2,6-iodophényle, 3,6-iodophényle, 4,6-iodophényle, 5,6-iodo-phényle, iodo, bromo, chloro, fluoro, nitro, cyano, méthyle, éthyle, *n*-propyle, isopropyle, cyclopropyle, *n*-butyle, isobutyle, tertio-butyle, *sec*-butyle, cyclobutyle, pentyle, néopentyle, cyclopentyle, n-hexyle, cyclohexyle, pyridyle, furyle, thiényle ; et/ou
- R₁ et R₂ peuvent être indépendamment choisis parmi l'atome d'hydrogène, les radicaux méthyle, trifluorométhyle, éthyle, *n*-propyle, isopropyle, cyclopropyle, *n*-butyle, isobutyle, tertio-butyle, *sec*-butyle, cyclobutyle, pentyle, néopentyle, cyclopentyle, n-hexyle, cyclohexyle ; R₁ et R₂ peuvent aussi former, ensemble et avec l'atome de carbone auquel ils sont liés, un cycle du type cyclopropyle, cyclobutyle, cyclopentyle ou cyclohexyle ; et/ou
- R₃ et R₄ peuvent être indépendamment choisis parmi l'atome d'hydrogène, les radicaux iodo, bromo, chloro, fluoro, méthyle, trifluorométhyle, éthyle, *n*-propyle, isopropyle, cyclopropyle, *n*-butyle, isobutyle, tertio-butyle, *sec*-butyle, cyclobutyle, pentyle, néopentyle, cyclopentyle, *n*-hexyle, cyclohexyle, phényle, benzyle, phénéthyle ; R₃ et R₄ peuvent aussi former, ensemble et avec l'atome de carbone auquel ils sont liés, un cycle du type cyclopropyle, cyclobutyle, cyclopentyle, ou cyclohexyle ; et/ou

- Y₁ peut être choisi entre un atome d'oxygène, de soufre ou de sélénium, ou un -NR auquel cas, R peut être choisi parmi l'atome d'hydrogène, les radicaux méthyle, trifluorométhyle, éthyle, *n*-propyle, isopropyle, cyclopropyle, *n*-butyle, isobutyle, tertiobutyle, *sec*-butyle, pentyle, néopentyle, *n*-hexyle, phényle, benzyle, phénéthyle, 2-méthylphényle, 3-méthylphényle, 4-méthyl-phényle, 2-méthoxyphényle, 3-méthoxyphényle, 4-méthoxyphényle, 2-fluorophényle, 3-fluorophényle, 4-fluorophényle, 2-chlorophényle, 3-chlorophényle, 4-chloro-phényle, 2-bromophényle, 3-bromophényle, 4-bromophényle, 2-iodophényle, 3-iodophényle, 4-iodophényle, 2-(trifluorométhyl)phényle, 3-(trifluorométhyl)-phényle, 4-(trifluorométhyl)phényle, 2,6-diméthylphényle, 3,6-diméthylphényle, 4,6-diméthylphényle, 5,6-diméthylphényle, 2,6-fluorophényle, 3,6-fluoro-phényle, 4,6-fluorophényle, 5,6-fluorophényle, 2,6-dichlorophényle, 3,6-dichloro-phényle, 4,6-dichlorophényle, 5,6-dichlorophényle, 2,6-bromophényle, 3,6-bromo-phényle, 4,6-bromophényle, 5,6-bromophényle, 2,6-iodophényle, 3,6-iodophényle, 4,6-iodo-phényle, et 5,6-iodophényle, et/ou
- Y₂ peut être choisi entre un atome d'oxygène, de soufre ou de sélénium, ou un -CR₅R₆ auquel cas, R₅ et R₆ peuvent être indépendamment choisis parmi l'atome d'hydrogène, les radicaux iodo, bromo, chloro, fluoro, méthyle, trifluorométhyle, éthyle, *n*-propyle, isopropyle, cyclopropyle, *n*-butyle, isobutyle, tertio-butyle, *sec*-butyle, cyclobutyle, pentyle, néopentyle, cyclopentyle, *n*-hexyle, cyclohexyle, phényle, benzyle, phénéthyle, 2-méthylphényle, 3-méthylphényle, 4-méthylphényle, 2-méthoxyphényle, 3-méthoxyphényle, 4-méthoxyphényle, 2-fluorophényle, 3-fluorophényle, 4-fluorophényle, 2-chlorophényle, 3-chlorophényle, 4-chlorophényle, 2-bromophényle, 3-bromophényle, 4-bromophényle, 2-iodophényle, 3-iodophényle, 4-iodophényle, 2-(trifluorométhyl)phényle, 3-(trifluorométhyl)phényle, 4-(trifluorométhyl)phényle, 2,6-diméthylphényle, 3,6-diméthylphényle, 4,6-diméthylphényle, 5,6-diméthylphényle, 2,6-fluorophényle, 3,6-fluorophényle, 4,6-fluorophényle, 5,6-fluorophényle, 2,6-dichlorophényle, 3,6-dichloro-phényle, 4,6-dichlorophényle, 5,6-dichlorophényle, 2,6-bromophényle, 3,6-bromo-phényle, 4,6-bromophényle, 5,6-bromophényle, 2,6-iodophényle, 3,6-iodophényle, 4,6-iodo-phényle, 5,6-iodophényle, pyridyle, furyle, thiényle, méthoxy, éthoxy, *n*₋propyloxy, isopropyloxy, *n*-butyloxy, tertio-butyloxy, *sec-*butyloxy, *n*-hexyloxy, cyclohexyloxy, phénoxy, méthylamine, diméthylamine, éthylamine, diéthylamine, *n*-propylamine, dipropylamine, isopropylamine, diisopropylamine, *n*-butylamine, dibutylamine, tertio-butylamine, *n*-hexylamine, dihexylamine, pipéridine, pyrolidine, aniline, méthylthio, éthylthio, *n*-propylthio, isopropylthio, *n*-butylthio, tertio-butylthio, *sec*-butylthio, *n*-pentylthio, cyclopentylthio, n-hexylthio, cyclohexylthio, et thiophénol ; R₅ et R₆ peuvent aussi former, ensemble et avec l'atome de carbone auquel ils sont liés, un cycle du type cyclopropyle, cyclobutyle, cyclopentyle ou cyclohexyle ; et/ou
- W est un radical carboxyle, une fonction dérivée (notamment l'ester méthylique, l'ester éthylique, l'ester propylique, l'ester isobutylique, l'ester tertio-butylique) ou un groupement bioisostère de la fonction acide carboxylique.

L'invention concerne plus préférentiellement des composés de Formule Générale (I) telle que précédemment définie et dans laquelle au moins l'une des conditions ci-après énumérées est satisfaite :
- Y₂ se trouve en méta ou en para de Y₁ et plus préférentiellement encore Y₂ se trouve en para de Y₁ ; et/ou
- X₃ désigne un atome d'hydrogène et plus préférentiellement encore X₃ et X₂ désignent simultanément un atome d'hydrogène ou X₃ et X₁ désignent simultanément un atome d'hydrogène; et/ou
- X₄ et X₅ désignent indépendamment un atome d'hydrogène, un radical alkyle ayant 1 à 6 atomes de carbone, ou un groupement -OR"ₐ ou -SR"ₐ (R"ₐ étant un radical alkyle ayant 1 à 6 atomes de carbone) ; et/ou
- R₁, R₂, R₃, R₄ désignent indépendamment un atome d'hydrogène ou un radical méthyle, éthyle, propyle, butyle, isopropyle ou tertio-butyle ; et/ou
- X₃, R₁ et R₂ désignent simultanément des atomes d'hydrogène ; et/ou
- X₁ et/ou X₂ désigne(nt) un cycle de 5 à 14, de préférence de 5 à 10, atomes, non substitué ou substitué par un groupement -CF₃, et plus préférentiellement encore un radical phényle, furanyle ou naphtalényle, non substitués ou substitués par un groupement -CF₃ ; et/ou
- G désigne un radical -ORₐ ou -SRₐ, avec Rₐ choisi parmi un radical alkyle ayant 1 à 6 atomes de carbone, un cyclohexyle ou un radical phényle ; ou bien alors G désigne un radical -NRₐR_{b}, Rₐ et R_{b} formant ensemble, et avec l'atome d'azote auquel ils sont liés, un hétérocycle de 3 à 8 atomes (notamment, un radical pipéridinyle) ; et/ou
- G désigne un radical -ORₐ avec Rₐ choisi parmi un radical méthyle, éthyle, propyle, butyle, isopropyle ou tertio-butyle.

Un premier aspect particulier de l'invention concerne les composés de Formule Générale (I) dans laquelle Y₁ désigne un atome d'oxygène ou soufre et simultanément Y₂ désigne un atome d'oxygène, un atome de soufre ou un groupement -CR₅R₆ dans lequel R₅ et R₆, identiques ou différents, sont choisis parmi un atome d'hydrogène, un radical alkyle de 1 à 6 atomes de carbone, un radical alkényle ou alkynyle de 2 à 6 atomes de carbone, et un cycle de 3 à 6 atomes, le cycle étant de préférence le phényle.

Un deuxième aspect particulier de l'invention concerne les composés de Formule Générale (I) dans laquelle Y₁ désigne un groupe aminé -NH. Selon ce deuxième aspect particulier de l'invention, Y₂ représente de préférence un atome d'oxygène, de soufre ou un radical -CR₅R₆-, dans lequel R₅ et R₆, identiques ou différents, sont choisis parmi un atome d'hydrogène, un radical alkyle de 1 à 6 atomes de carbone, un radical alkényle ou alkynyle de 2 à 6 atomes de carbone, et un cycle de 3 à 6 atomes, le cycle étant de préférence le phényle.

Selon ce premier ou deuxième aspect particulier de l'invention, X₁ désigne de préférence un radical phényle non substitué ou un radical phényle substitué par un groupement -CF₃, ledit groupement -CF₃ étant de préférence en para du radical pyridinyle, et/ou G désigne un groupement -OCH₃ ou -OC(CH₃)₃. Plus spécifiquement, X₁ désigne avantageusement un radical phényle substitué par un groupement -CF₃ en para du radical pyridinyle, G désigne un groupement -OCH₃, et X₂ désigne un atome d'hydrogène.Selon une variante de réalisation de ce premier aspect particulier de l'invention, X₁ désigne avantageusement un radical phényle non substitué, G désigne un groupement -OC(CH₃)₃, et X₂ désigne un atome d'hydrogène. Qu'il s'agisse de composés appartenant audit premier aspect particulier de l'invention ou audit deuxième aspect particulier de l'invention, avantageusement R₁, R₂, R₃ et R₄ désignent simultanément des atomes d'hydrogène.

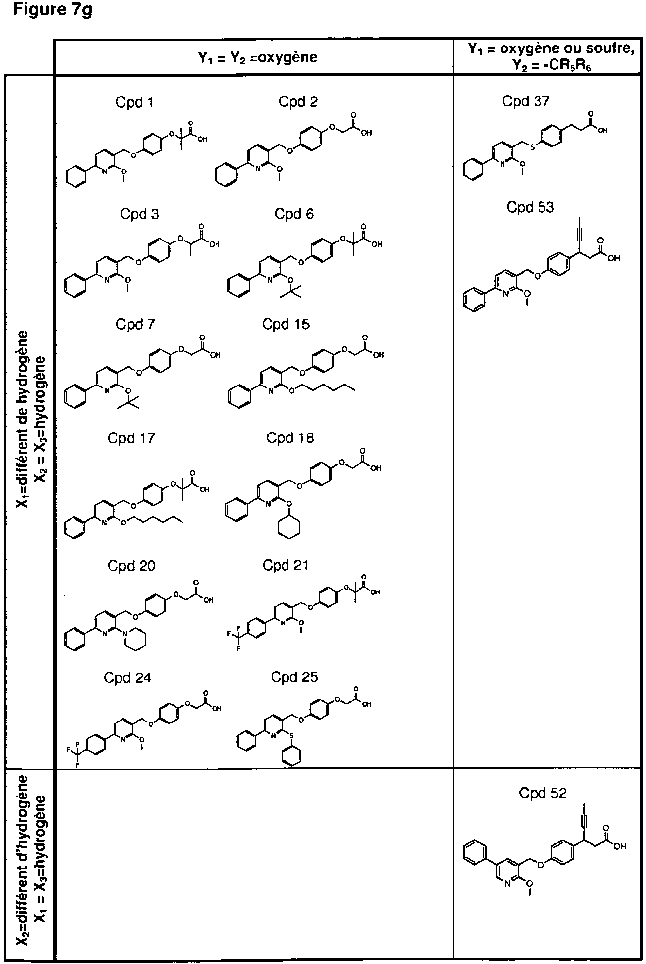

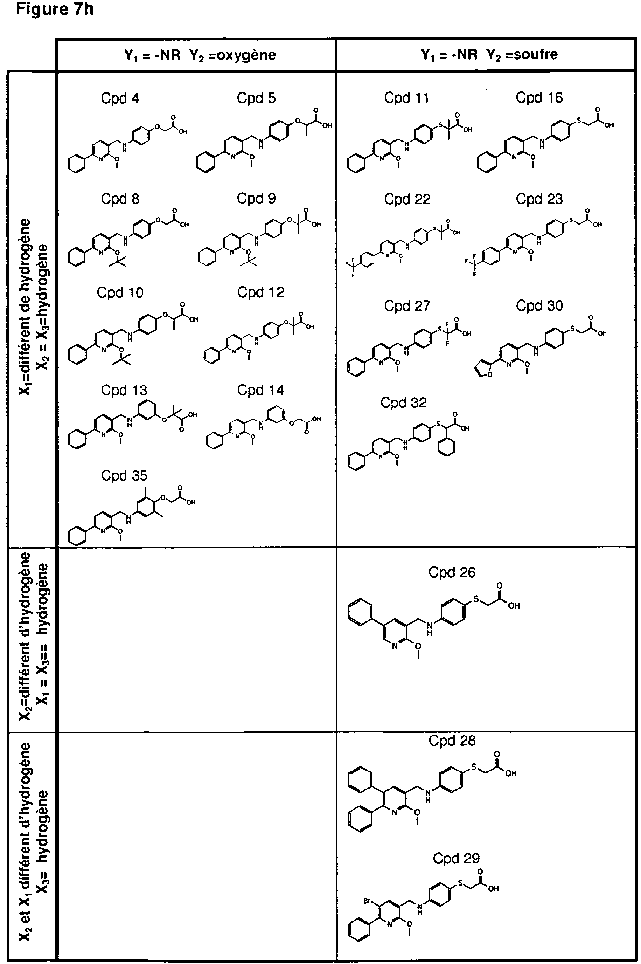

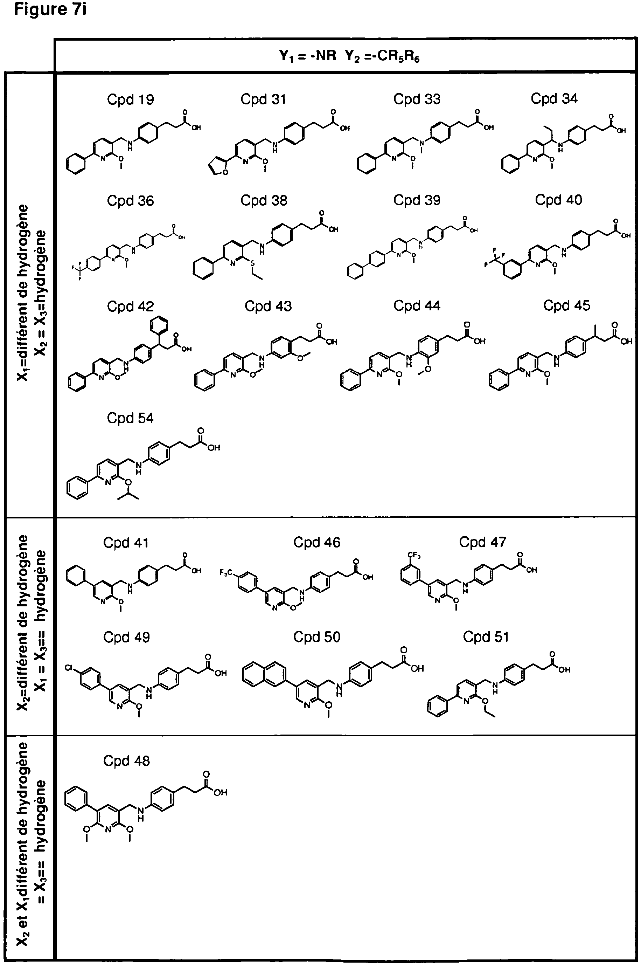

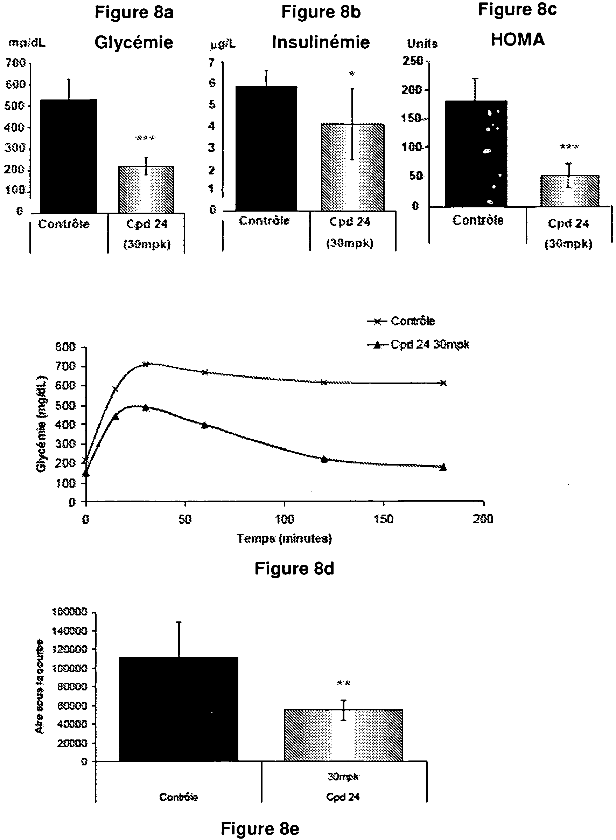

Un troisième aspect particulier de l'invention concerne les composés de Formule Générale (I) qui
- sont identifiés et regroupés sur la base des caractéristiques structurales comme définies dans les Figures 7g, 7h et 7i ; et/ou
- présentent les activités comme établies dans les Exemples 8 à 12, et plus spécifiquement dans le Tableau 8-1 et les Figures 8 à 12.

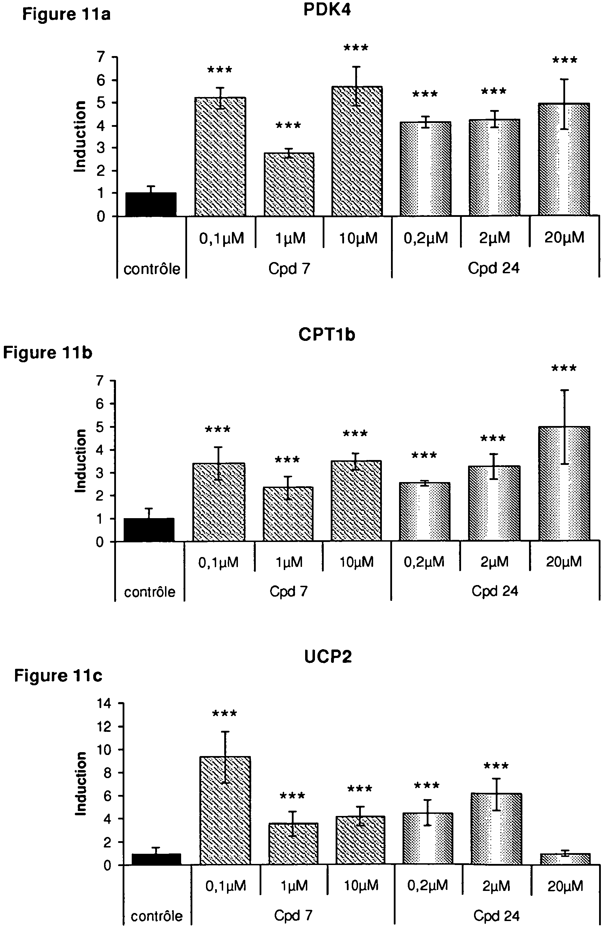

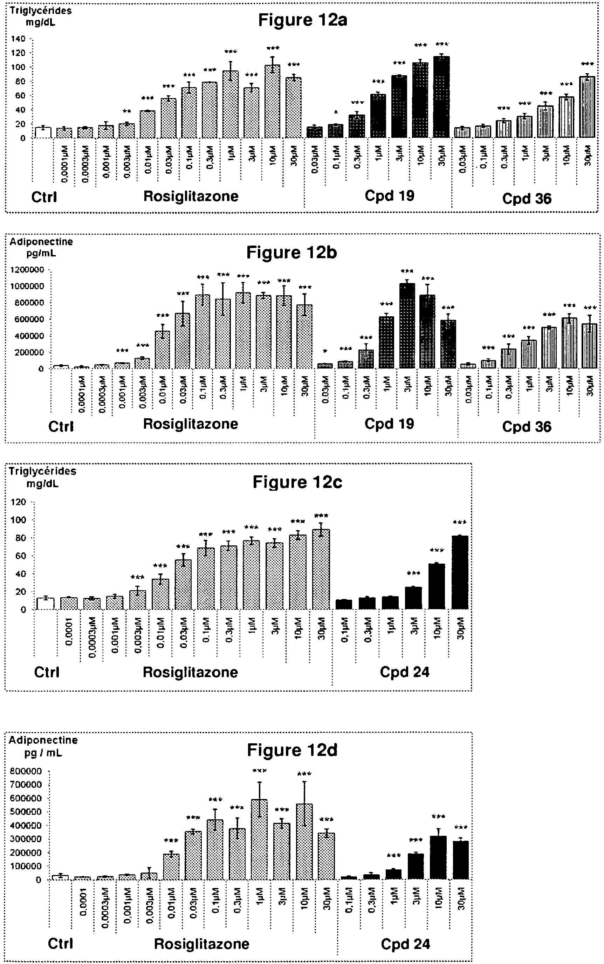

La présente invention concerne des composés activateurs des PPARs. Ces composés répondent aux critères pharmacologiques cités dans la littérature pour ce genre de composés, par mesure de différents paramètres comme les propriétés activatrices de PPARs humains in vitro et en modèles cellulaires, et le caractère anti-diabétique ou hypolipémiant in vivo dans des modelés murines. Ces résultats montrent comment les composés de Formule Générale (I) ayant des groupements spécifiques ont des propriétés supérieures et inattendues par rapport aux documents de l'art antérieur mentionnant des composés proches, comme WO 03/084916, WO 08/152333, WO 05/041959, WO 08/066356, EP 1266888, et US 2005096336. Par exemple, les propriétés activatrices de PPARδ ont été confirmées *in vivo* et *in vitro* pour Cpd 24 et Cpd 7 (voir Tableau 8-1, Exemple 11, et Figure 11), qui font partie du même groupement de composés selon l'invention (voir Figure 7g, dans laquelle Cpd 2 qui a été évalué dans Exemple 9). Egalement, les propriétés activatrices de PPARγ ont été confirmées *in vivo* et *in vitro* pour Cpd 19 et Cpd 36 (voir Tableau 8-1, Exemple 12, et Figure 12), qui font partie du même groupement de composés selon l'invention (voir Figure 7i).

De manière préférée, les composés selon l'invention sont choisis parmi :

| | |
|---|---|
| Cpd 1 : | acide 2-(4-((2-méthoxy-6-phénylpyridin-3-yl)méthoxy)phénoxy)-2-méthyl-propanoïque |
| Cpd 2 : | acide 2-(4-((2-méthoxy-6-phénylpyridin-3-yl)méthoxy)phénoxy)éthanoïque |
| Cpd 3 : | acide 2-(4-((2-méthoxy-6-phénylpyridin-3-yl)méthoxy)phénoxy)propanoïque |
| Cpd 4 : | acide 2-(4-(((2-méthoxy-6-phénylpyridin-3yl)méthyl)amino)phénoxy) éthanoïque |
| Cpd 5 : | acide 2-(4-(((2-méthoxy-6-phénylpyridin-3yl)méthyl)amino)phénoxy) propanoïque |
| Cpd 6 : | acide 2-(4-((2-tertio-butyloxy-6-phénylpyridin-3-yl)méthoxy)phénoxy)-2-méthyl-propanoïque |
| Cpd 7 : | acide 2-(4-(((2-tertio-butyloxy-6-phénylpyridin-3-yl)méthoxy)phénoxy) éthanoïque |
| Cpd 8 : | acide 2-(4-((2-tertio-butytoxy-6-phénylpyridin-3-yl)méthyl)amino)phénoxy) éthanoïque |
| Cpd 9 : | acide 2-(4-(((2-tertio-butyloxy-6-phénylpyridin-3-yl)méthyl)amino)phénoxy)-2-méthyl-propanoïque |
| Cpd 10 : | acide 2-(4-(((2-tertio-butyloxy-6-phénylpyridin-3-yl)méthyl)amino)phénoxy)-propanoïque |
| Cpd 11 : | acide 2-(4-(((2-méthoxy-6-phénylpyridin-3-yl)méthyl)amino)phénylthio)-2-méthyl-propanoïque |
| Cpd 12 : | acide 2-(4-(((2-méthoxy-6-phénylpyridin-3-yl)méthyl)amino)phénoxy)-2-méthyl-propanoïque |
| Cpd 13 : | acide 2-(3-(((2-méthoxy-6-phénylpyridin-3-yl)méthyl)amino)phénoxy)-2-méthyl-propanoïque |
| Cpd 14 : | acide 2-(3-(((2-méthoxy-6-phénylpyridin-3-yl)méthyl)amino)phénoxy) éthanoïque |
| Cpd 15 : | acide 2-(4-((2-hexyloxy-6-phénylpyridin-3-yl)méthoxy)phénoxy)éthanoïque |
| Cpd 16 : | acide 2-(4-(((2-méthoxy-6-phénylpyridin-3-yl)méthyl)amino)phénylthio)-éthanoïque |
| Cpd 17 : | acide 2-(4-((2-hexyloxy-6-phénylpyridin-3-yl)méthoxy)phénoxy)-2-méthyl-propanoïque |
| Cpd 18 : | acide 2-(4-((2-cyclohexyloxy-6-phénylpyridin-3-yl)méthoxy)phénoxy)éthanoïque |
| Cpd 19 : | acide 3-(4-(((2-méthoxy-6-phénylpyridin-3-yl)méthyl)amino)phényl) propanoïque |
| Cpd 20 : | acide 2-(4-((6-phényl-2-(pipéridin-1-yl)pyridin-3-yl)méthoxy)phénoxy)-éthanoïque |
| Cpd 21 : | acide 2-(4-((2-méthoxy-6-(4-(trifluorométhyl)phényl)pyridin-3-yl)méthoxy)-phénoxy)-2-méthylpropanoïque |
| Cpd 22 : | acide 2-(4-(((2-méthoxy-6-(4-(trifluorométhyl)phényl)pyridin-3-yl)méthyl)amino) -phénylthio)-2-méthylpropanoïque |
| Cpd 23 : | acide 2-(4-(((2-méthoxy-6-(4-(trifluorométhyl)phényl)pyridin-3-yl)méthyl)amino) -phénylthio)éthanoïque |
| Cpd 24 : | acide 2-(4-((2-méthoxy-6-(4-(trifluorométhyl)phényl)pyridin-3-yl)méthoxy)-phénoxy)éthanoïque |
| Cpd 25 : | acide 2-(4-((2-phénylthio-6-(phényl)pyridin-3-yl)méthoxy)phénoxy)éthanoïque |
| Cpd 26 : | acide 2-(4-(((2-méthoxy-5-phénylpyridin-3-yl)méthyl)amino)phénylthio) éthanoïque |
| Cpd 27 : | acide 2-(4-(((2-méthoxy-6-phénylpyridin-3-yl)méthyl)amino)phénylthio)-2,2-difluoroéthanoïque |
| Cpd 28 : | acide 2-(4-(((2-méthoxy-5,6-diphénylpyridin-3-yl)méthyl)amino)phénylthio)-éthanoïque |
| Cpd 29 : | acide 2-(4-(((2-méthoxy-5-bromo-6-phénylpyridin-3-yl)méthyl)amino) phénylthio)-éthanoïque |
| Cpd 30 : | acide 2-(4-(((2-méthoxy-6-furylpyridin-3-yl)méthyl)amino)phénylthio)-éthanoïque |
| Cpd 31 : | acide 3-(4-(((2-méthoxy-6-furylpyridin-3-yl)méthyl)amino)phényl)propanoïque |
| Cpd 32 : | acide 2-(4-(((2-méthoxy-6-phénylpyridin-3-yl)méthyl)amino)phénylthio)-2-phényl-éthanoïque |
| Cpd 33 : | acide 3-(4-(((2-méthoxy-6-phénylpyridin-3-yl)méthyl)(méthyl)amino)phényl)-propanoïque |
| Cpd 34 : | acide 3-(4-(1-((2-méthoxy-6-phénylpyridin-3-yl)propyl)amino)phényl)-propanoïque |
| Cpd 35 : | acide 2-(4-(((2-méthoxy-6-phénylpyridin-3-yl)méthyl)amino)-2,6-diméthyl-phénoxy)éthanoïque |
| Cpd 36 : | acide 3-(4-(((2-méthoxy-6-(4-(trifluorométhyl)phényl)pyridin-3-yl)méthyl)amino) -phényl)-propanoïque |
| Cpd 37 : | acide 3-(4-((2-méthoxy-6-phénylpyridin-3-yl)méthylthio)phényl)propanoïque |
| Cpd 38 : | acide 3-(4-(((2-(éthylthio)-6-phénylpyridin-3-yl)méthyl)amino)phényl)-propanoïque |
| Cpd 39 : | acide 3-(4-(((2-méthoxy-6-(parabiphényl)pyridin-3-yl)méthyl)amino)phényl)-propanoïque |
| Cpd 40 : | acide 3-(4-(((2-méthoxy-6-(3-(trifluorométhyl)phényl)pyridin-3-yl)méthyl)amino)-phényl)propanoïque |
| Cpd 41 : | acide 3-(4-(((2-méthoxy-5-phénylpyridin-3-yl)méthyl)amino)phényl)-propanoïque |

| | |
|---|---|
| Cpd 42 : | acide 3-(4-((2(-méthoxy-6-phénylpyridin-3-yl)méthyl)amino)phényl)-3-phényl-propanoïque |
| Cpd 43 : | acide 3-(2-méthoxy-4-(((2-méthoxy-6-phénylpyridin-3-yl)méthyl)amino)-phényl)-propanoïque |
| Cpd 44 : | acide 3-(3-méthoxy-4-(((2-méthoxy-6-phénylpyridin-3-yl)méthyl)amino)-phényl)-propanoïque |
| Cpd 45 : | acide 3-(4-(((2-méthoxy-6-phénylpyridin-3-yl)méthyl)amino)phényl)butanoïque |
| Cpd 46 : | acide 3-(4-(((2-méthoxy-5-(4-(trifluorométhyl)phényl)pyridin-3-yl)méthyl)amino)phényl)propanoïque |
| Cpd 47 : | acide 3-(4-(((2-méthoxy-5-(3-(trifluorométhyl)phényl)pyridin-3-yl)méthyl)amino) -phényl)propanoïque |
| Cpd 48 : | acide 3-(4-(((2,6-diméthoxy-5-phénylpyridin-3-yl)méthyl)amino)phényl)-propanoïque |
| Cpd 49 : | acide 3-(4-(((5-(4-chlorophényl)-2-méthoxypyridin-3-yl)méthyl)amino)phényl)-propanoïque |
| Cpd 50 : | acide 3-(4-(((2-méthoxy-5-(naphthalèn-2-yl)pyridin-3-yl)méthyl)amino)phényl)-propanoïque |
| Cpd 51 : | acide 3-(4-(((2-éthoxy-6-phénylpyridin-3-yl)méthyl)amino)phényl)propanoïque |
| Cpd 52 : | acide 3-(4-((2-méthoxy-5-phénylpyridin-3-yl)méthoxy)phényl)hex-4-ynoïque |
| Cpd 53 : | acide 3-(4-((2-méthoxy-6-phénylpyridin-3-yl)méthoxy)phényl)hex-4-ynoïque |
| Cpd 54 : | acide 3-(4-(((2-isopropyloxy-6-phénylpyridin-3-yl)méthyl)amino)phényl)-propanoïque. |

Même si les composés selon l'invention peuvent être générés et purifiés selon des procédés et avec des composés déjà connus par l'homme de l'art et tels que ceux décrits dans la littérature, l'invention concerne les procédés de préparation des composés de Formule Générale (I).

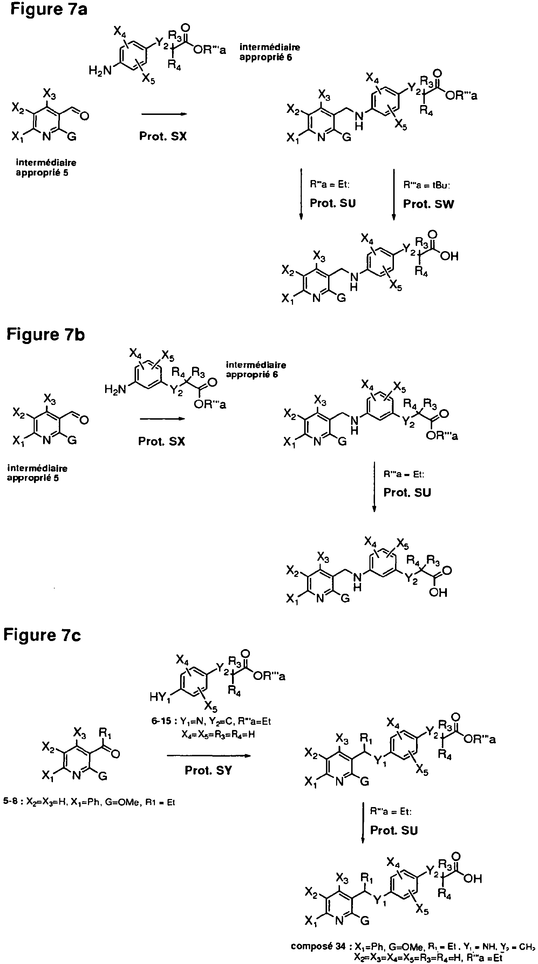

Selon une première variante du procédé de préparation (Figure 7a, 7b et 7c), les composés de Formule Générale (I) peuvent être obtenus par une série de réactions consistant à faire réagir des intermédiaires de type phénol, thiophénol, ou aniline selon l'invention avec un des intermédiaires de type pyridine 3-carboxaldéhyde ou cétone selon l'invention.

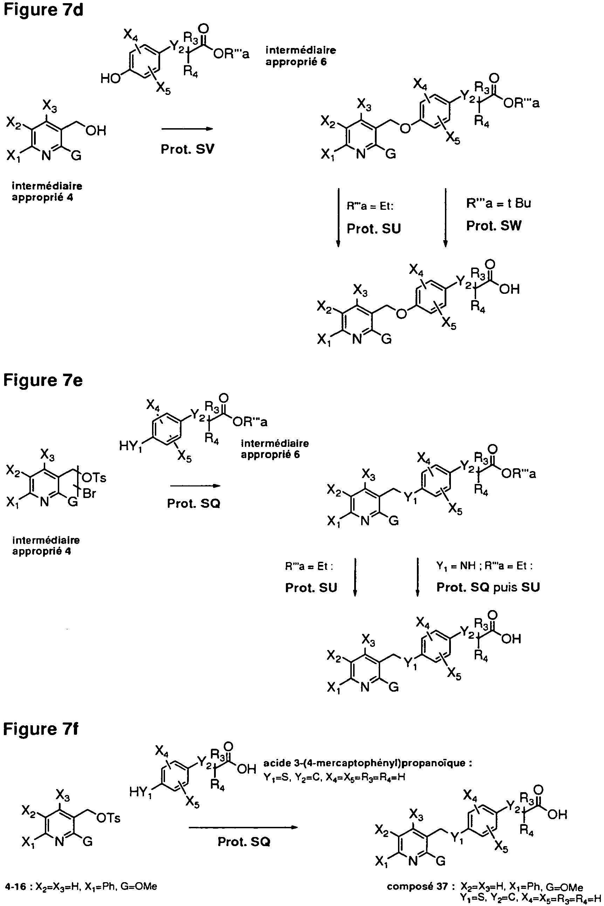

Selon une deuxième variante du procédé de préparation (Figure 7d, 7e et 7f), les composés de Formule Générale (I) peuvent être obtenus par une série de réactions consistant à faire réagir des intermédiaires de type phénol, thiophénol, ou aniline selon l'invention avec un des intermédiaires de type 3-hydroxyméthyl-, 3-halométhyl- ou 3-arylsulfonylméthyl-pyridine selon l'invention.

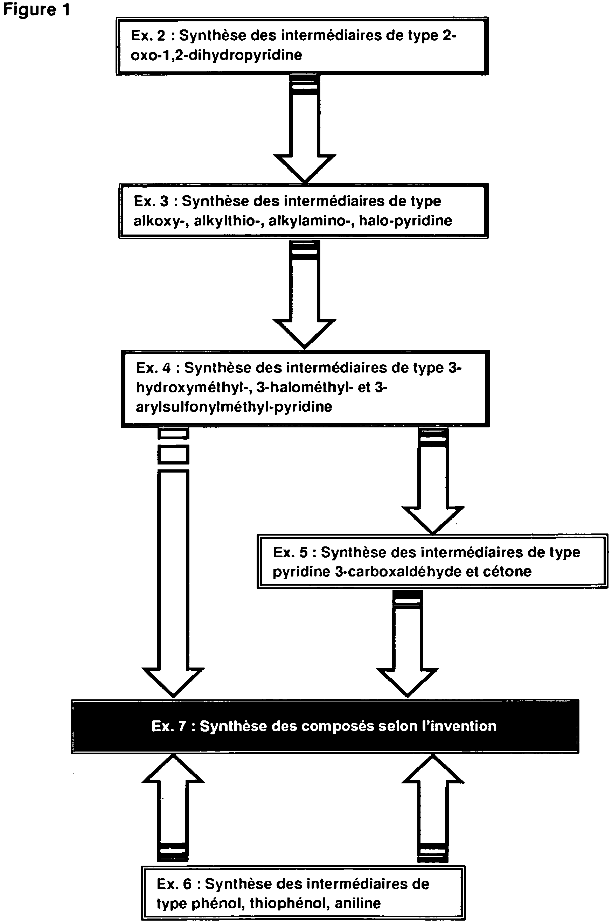

Les détails sur les méthodes générales de synthèse et de purification des bruts réactionnels obtenus sont définis dans Exemple 1. Plus particulièrement, les Exemples 1 à 7 montrent comment différentes séries de composés selon l'invention, et les correspondants intermédiaires réactionnels, peuvent être synthétisés et purifiés à partir de composés déjà connus. Un schéma général de synthèse des composés de Formule Générale (I) est présenté dans Figure 1 mais l'homme de l'art pourra synthétiser lesdites composés avec d'autres procédés et composés déjà connus, notamment pour obtenir des intermédiaires réactionnels et/ou composés avec des groupements G, R, R₁-R₆, R/'/"/"'ₐ, R/'/"/"'_{b}, X₁-X₆ et/ou Y₁, Y₂ spécifiques.

Les groupes fonctionnels éventuellement présents dans les intermédiaires réactionnels utilisés dans les procédés peuvent être protégés, soit sous forme permanente, soit sous forme temporaire, par des groupes protecteurs qui assurent une synthèse univoque des composés attendus. Les réactions de protection et déprotection sont effectuées selon des techniques bien connues de l'homme de l'art ou telles que celles décrites dans la littérature, comme dans le livre « Greene's Protective Groups in Organic Synthesis » (4ème édition, 2007 ; édité par Wuts PG et Greene TW ; publié par John Wiley et Sons).

Les composés selon l'invention peuvent contenir un ou plusieurs centres asymétriques. La présente invention inclut les stéréoisomères (diastéréoisomères, énantiomères), purs ou en mélange, ainsi que les mélanges racémiques et les isomères géométriques, ou les tautomères. Quand un mélange énantiomériquement pur (ou enrichi) est souhaité, il pourra être obtenu soit par purification du produit final ou d'intermédiaires chiraux, soit par synthèse asymétrique suivant des méthodes connues de l'homme du métier (utilisant par exemple des réactifs et catalyseurs chiraux). Certains composés selon l'invention peuvent avoir différentes formes tautomères stables et toutes ces formes ainsi que leurs mélanges sont inclus dans l'invention. Les techniques pour obtenir et caractériser les stéréoisomères, purs ou en mélange, ainsi que les mélanges racémiques et les isomères géométriques, ou les tautomères ont été décrites dans la littérature, comme dans le livre « Chirality in Drug Design and Development» (2004 ; édité par Reddy IK et Mihvar R ; Publié par CRC Press).

Les composés de Formule Générale (I) peuvent exister à l'état de bases ou de sels d'addition à des acides. Ces sels peuvent être préparés, sélectionnés et utilisés selon des techniques bien connues de l'homme de l'art ou telles que celles décrits dans la littérature comme dans le livre « Handbook of Pharmaceutical Salts: Properties, Selection, and Use » (2002 ; édité par Stahl PH et Wermuth GH ; publié par. VHCA Switzerland et Wiley-VCH Germany). Notamment, la présente invention concerne les sels « pharmaceutiquement acceptables » des composés selon l'invention. D'une manière générale, ce terme désigne les sels peu ou non toxiques obtenus à partir de bases ou d'acides, organiques ou inorganiques.

Ces sels peuvent être obtenus lors de l'étape de purification finale du composé selon l'invention ou par incorporation du sel sur le composé déjà purifié. Ces sels peuvent être préparés avec des acides pharmaceutiquement acceptables mais les sels d'autres acides utiles pour la purification ou l'isolement des composés de Formule Générale (I) font également partie de l'invention. En particulier, lorsque les composés selon l'invention se présentent sous forme d'un sel, il s'agit d'un sel d'un métal alcalin, en particulier, un sel de sodium ou de potassium, ou d'un sel d'un métal alcalino-terreux, en particulier le magnésium ou le calcium, ou encore d'un sel avec une amine organique, plus particulièrement, avec un acide aminé tel que l'arginine ou la lysine.

Plus particulièrement, le groupement W tel que décrit précédemment peut avoir un caractère acide. Les sels correspondants sont choisis parmi les sels métaux (par exemple, aluminium, zinc, chrome), les sels alcalins (par exemple, lithium, sodium, potassium) ou alcalino-terreux (par exemple, calcium, magnésium). Il peut s'agir par exemple de sels organiques tels que des dérivés ammonium et amines non toxiques : ammonium, ammonium quaternaire (par exemple, tétraméthylammonium, tétraéthylammonium), alkylamines (par exemple, méthylamine, diméthylamine, triméthylamine, triéthylamine, éthylamine, etc.), hydroxyalkylamines (par exemple, 2-hydroxyéthylamine, bis-(2-hydroxyéthyl)amine, tri-(2-hydroxyéthyl)amine, etc.), cycloalkylamines (par exemple, bicyclohexylamine, glucamine, etc.), pyridines et analogues (comme collidine, quinine, quinoline, etc.) de sels d'acides aminés à caractère basique (par exemple, lysine, arginine, etc.).

Le noyau pyridine, les groupes G et/ou Y₁ tels que décrits précédemment peuvent avoir un caractère basique. Les sels correspondants sont choisis avantageusement parmi les acides minéraux (chlorhydrique, bromhydrique, sulfurique, borique, nitrique, phosphorique, etc.) ou les acides organiques (par exemple, les acides carboxyliques ou sulfoniques tels que l'acide formique, acétique, méthylsulfonique, propionique, toluènesulfonique, valérique, oléique, palmitique, stéarique, lactique, laurique, oxalique, citrique, maléique, succinique, glycolique, tartrique, etc.) ou encore les sels obtenus à partir d'acides aminés à caractère acide tels que l'acide glutamique.

Certains composés selon l'invention peuvent être isolés sous forme de zwitterions et chacune de ces formes est incluse dans l'invention ainsi que leurs mélanges. Certains composés selon l'invention et leurs sels peuvent être stables sous plusieurs formes solides. La présente invention inclut toutes les formes solides des composés selon l'invention ce qui inclut les formes amorphes, polymorphes, mono- et poly-cristallines.

Les composés de Formule Générale (I) peuvent exister sous forme libre ou sous forme solvatée, à savoir sous forme d'associations ou de combinaisons avec une ou plusieurs molécules d'un solvant, par exemple avec des solvants pharmaceutiquement acceptables tels que l'eau (hydrates) ou l'éthanol. La présente description divulgue également les prodrogues des composés selon l'invention qui, après administration chez un sujet, se transforment en composés tels que décrits dans l'invention ou en leurs métabolites qui présentent des activités thérapeutiques comparables aux composés selon l'invention.

Les composés selon l'invention marqués par un ou plusieurs isotopes sont également inclus dans l'invention : ces composés sont structurellement identiques mais diffèrent par le fait qu'au moins un atome de la structure est remplacé par un isotope (radioactif ou non). Des exemples d'isotopes pouvant être inclus dans la structure des composés selon l'invention peuvent être choisis parmi l'hydrogène, le carbone, l'azote, l'oxygène, le soufre tels que ²H, ³H, ¹³C, ¹⁴C, ¹⁵N, ¹⁸O, ¹⁷O, ³⁵S respectivement. Les isotopes radioactifs ³H et ¹⁴C sont particulièrement préférés car faciles à préparer et à détecter dans le cadre d'études de biodisponibilité *in vivo* des substances. Les isotopes lourds (tels que ²H) sont particulièrement préférés car ils sont utilisés comme standards internes dans des études analytiques.

La présente invention a aussi pour objet les composés tels que décrits ci-avant, à titre de médicaments. Notamment la présente invention a pour objet l'utilisation d'un composé selon l'invention dans la fabrication d'un médicament destiné au traitement thérapeutique et/ou prophylactique du diabète, des dyslipidémies, de l'insulino-résistance, des pathologies associées au syndrome métabolique, de l'athérosclérose, et des maladies cardiovasculaires (notamment celles liées aux dérèglements du métabolisme lipidique et/ou glucidique), de l'obésité, de l'hypertension et/ou des maladies inflammatoires.

La présente invention a également pour objet une composition pharmaceutique comprenant, dans un support acceptable sur le plan pharmaceutique, au moins un composé tel que décrit ci-dessus, éventuellement en association avec un ou plusieurs autres principes actifs thérapeutiques et/ou cosmétiques. Il s'agit avantageusement d'une composition pharmaceutique pour le traitement thérapeutique et/ou prophylactique du diabète, des dyslipidémies, de l'insulino-résistance, des pathologies associées au syndrome métabolique, de l'athérosclérose, des maladies cardiovasculaires, de l'obésité, de l'hypertension, des maladies inflammatoires, etc. Les pathologies inflammatoires désignent de manière particulière l'asthme. Il s'agit préférentiellement d'une composition pharmaceutique pour prévenir et/ou traiter les facteurs de risque cardiovasculaire liés aux dérèglements du métabolisme lipidique et/ou glucidique (hyperlipidémie, diabète de type 2, obésité etc.) en permettant la diminution du risque global, avec des composés activateurs PPAR comme décrit dans la littérature (Gilde AJ et al., 2006; Blaschke F et al., 2006).

La présente description concerne une composition nutritionnelle comprenant au moins un composé tel que décrit ci-dessus.

Dans le cadre de la présente invention et de façon générale, on entend par « support acceptable sur le plan pharmaceutique » des substances telles que les excipients, les véhicules, les adjuvants, les tampons qui sont classiquement utilisés, en combinaison avec le(s) principe(s) actif(s), pour la préparation d'un médicament. Le choix de tels supports dépend essentiellement de la voie d'administration envisagée.

Un autre objet de l'invention réside dans l'utilisation d'au moins un composé tel que décrit ci-avant pour la préparation de compositions pharmaceutiques destinées au traitement thérapeutique et/ou prophylactique de diverses pathologies, notamment liées à des troubles du métabolisme parmi lesquelles on peut citer le diabète (en particulier, le diabète de type 2), les dyslipidémies, l'insulino-résistance, les pathologies associées au syndrome X, métabolique, l'athérosclérose, les maladies cardiovasculaires, l'obésité, l'hypertension, les maladies inflammatoires.

A titre d'exemple, les composés selon l'invention, à l'instar des composés insulinosécréteurs et des composés activateurs PPAR actuellement commercialisés pour le traitement des maladies métaboliques, pourront de manière avantageuse être administrés en combinaison avec un ou plusieurs autres agents thérapeutiques et/ou cosmétiques, commercialisés ou en développement, tels que :
- des anti-diabétiques : les insulinosécréteurs (comme les sulfonylurées et glinides ... ), les inhibiteurs de l'alpha-glucosidase, les agonistes PPARγ (comme les thiazolidinediones...), les agonistes mixtes PPARα/PPARγ, les pan-PPAR (composés activant simultanément les 3 isoformes PPAR), les biguanides, les inhibiteurs de la Dipeptidyl Peptidase IV, les agonistes du Glucagon-Like Peptide-1 (comme exenatide), les inhibiteurs d'alpha-glucosidase, ainsi que l'insuline et les analogues de l'insuline ;
- des molécules hypolipémiantes et/ou hypocholestérolémiantes : les fibrates (comme fénofibrate et gemfibrozil), les inhibiteurs de la hydroxylméthylglutaryl Coenzyme A réductase (comme les statines), les inhibiteurs de l'absorption du cholestérol (comme ézétimibe et phytostérols), les inhibiteurs de la Cholesteryl Ester Transfer Protein, les inhibiteurs de l'Acyl-CoA:Cholesterol O-Acyl Transferase (ACAT), les inhibiteurs de la Microsomal Triglycéride Transfer Protein, les agents séquestrants des acides biliaires, la vitamine E, les acides gras poly-insaturés, les acides gras oméga 3, les dérivés de type acide nicotinique (niacine) ;
- des agents anti-hypertenseurs et les agents hypotenseurs : les inhibiteurs de Angiotensin-Converting Enzyme (comme captopril et enalapril), les antagonistes du récepteur de l'angiotensine Il (comme losartan et irbésartan), les bêta-bloquants (comme atenolol et propranolol), les diurétiques thiazidiques et non thiazidiques, les vasodilatateurs, les bloquants des canaux calciques (comme nifédipine et vérapamil) ;
- des agents anti-plaquettaires (comme aspirine et clopidogrel) ;
- des agents anti-obésité : sibutramine, les inhibiteurs de lipases (orlistat), les agonistes et antagonistes PPARδ, les antagonistes du récepteur cannabinoïde CB1, les agonistes de la dopamine, les agonistes des récepteurs de leptine, les inhibiteurs de la recapture de la sérotonine, les agonistes béta-3, les agonistes CCK-A, les inhibiteurs de NPY, les agonistes des récepteurs MC4, les antagonistes des récepteurs Melanin Concentrating Hormone, les antagonistes de l'orexine, les inhibiteurs de phosphodiesterases, les inhibiteurs de la 11-β-hydroxy steroid deshydrogenase, les inhibiteurs de dipeptidyl peptidase IV, les antagonistes (ou agonistes inverses) de l'histamine H3, les dérivés du Ciliary Neurotrophic Factor, les agonistes des récepteurs Growth Hormone Secretagogue, les modulateurs de la ghréline, les inhibiteurs de la diacyglycerol acyltransferase ;
- des agents anti-inflammatoires : les corticoïdes (comme prednisone et hydrocortisone), les Anti-Inflammatoires Non Stéroidiens (dérivés de l'indole, du groupe des arylcarboxyliques, dérivés de l'oxicam, ou du groupe des fénamates, les inhibiteurs sélectifs de la COX2 (comme célécoxib ou rofécoxib) ;
- des agents anti-oxydants : par exemple le probucol ;
- des agents utilisés dans le traitement de l'insuffisance cardiaque : les diurétiques thiazidiques ou non thiazidiques, les inhibiteurs de l'Angiotensin Converting Enzyme, les digitaliques, les bêta-bloquants, les inhibiteurs de phosphodiestérases ;
- des agents utilisés pour le traitement de l'insuffisance coronaire : les bêta-bloquants, les bloquants des canaux calciques, les agents donneurs de NO, l'Amiodarone,
- des anti-asthmatiques : les bronchodilatateurs (agonistes des récepteurs bêta 2), des corticoïdes, le cromoglycate, les antagonistes du récepteur aux leucotriènes ;
- des corticoïdes utilisés dans le traitement des pathologies de la peau telles que le psoriasis et les dermatites ;
- des vasodilatateurs et/ou des agents anti-ischémiques.

La présente description divulgue également une méthode de traitement thérapeutique et/ou prophylactique du diabète, des dyslipidémies, de l'insulino-résistance, des pathologies associées au syndrome métabolique, de l'athérosclérose, des maladies cardiovasculaires (notamment celles liées aux dérèglements du métabolisme lipidique et/ou glucidique), de l'obésité, de l'hypertension et/ou des maladies inflammatoires, comprenant l'administration à un sujet, notamment humain, d'une quantité efficace d'un composé ou d'une composition pharmaceutique tels que définis ci-avant. Au sens de l'invention le terme «une quantité efficace » se réfère à une quantité du composé suffisante pour produire le résultat biologique désiré, de préférence non toxique. Au sens de l'invention le terme « sujet » signifie un mammifère et plus particulièrement un humain.

Le terme « traitement » désigne le traitement curatif, symptomatique, et/ou prophylactique. Les composés de l'invention peuvent ainsi être utilisés chez des sujets (en particulier humains) atteints d'une maladie déclarée. Les composés de l'invention peuvent aussi être utilisés pour retarder ou ralentir la progression ou prévenir une progression plus en avant de la maladie, améliorant ainsi la condition des sujets. Les composés de l'invention peuvent enfin être administrés aux personnes non malades, mais qui pourraient développer normalement la maladie ou qui ont un risque important de développer la maladie.

Selon un autre aspect de l'invention, le composé de Formule Générale (I), ou un de ses sels d'addition à un acide pharmaceutiquement acceptable ou un de ses solvats ou hydrates et un autre agent thérapeutique peuvent être administrés de manière simultanée (dans une seule et même forme pharmaceutique), séparée (avec l'administration, en même temps, des deux composés mais chacun compris dans une forme pharmaceutique distincte) ou étalée dans le temps (avec l'administration, en temps différents, des deux composés, généralement pendant un laps de temps qui n'excède pas 24 heures).

Les compositions pharmaceutiques selon l'invention comprennent avantageusement un ou plusieurs excipients ou véhicules, acceptables sur le plan pharmaceutique. On peut citer par exemple des solutions salines, physiologiques, isotoniques, tamponnées, etc., compatibles avec un usage pharmaceutique et connues de l'homme du métier. Les compositions peuvent contenir un ou plusieurs agents ou véhicules choisis parmi les agents dispersants, solubilisants, stabilisants, conservateurs, émulsionnants, anti-oxydants, émollients, hydratants, mouillants, pour l'amélioration de la saveur, pour réguler l'hydratation ou le pH, etc. Des agents ou véhicules utilisables dans des formulations (liquides et/ou injectables et/ou solides) sont notamment la méthylcellulose, l'hydroxyméthylcellulose, la carboxyméthylcellulose, le polysorbate 80, le mannitol, la gélatine, le lactose, certaines huiles végétales ou animales, l'acacia, le dextrose, le saccharose, la gélatine, l'agar, l'acide stéarique, les liposomes, etc. Les compositions peuvent être formulées sous forme de suspensions injectables, gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons. A titre d'exemple, une forme unitaire d'administration d'un composé selon l'invention sous forme de comprimé peut comprendre les composants suivants : mannitol, croscarmellose sodique, amidon de maïs, hydroxypropyl-méthylcellulose, stéarate de magnésium.

Les composés ou compositions selon l'invention peuvent être administrés de différentes manières et sous différentes formes. Ainsi, ils peuvent être par exemple administrés de manière systémique, par voie orale, parentérale, topique, oculaire, rectale, perlinguale, par inhalation ou par injection, comme par exemple par voie intraveineuse, intra-musculaire, sous-cutanée, trans-dermique, intra-artérielle, etc. Pour les injections, les composés sont généralement conditionnés sous forme de suspensions liquides, qui peuvent être injectées au moyen de seringues ou de perfusions. Par voie orale, la composition peut se présenter sous forme de comprimés, de gélules, de dragées, de sirops, de suspensions, de solutions, de poudres, de granulés, d'émulsions, de suspensions de microsphères ou nanosphères ou de vésicules lipidiques ou polymériques permettant une libération contrôlée. Par voie parentérale, la composition peut se présenter sous forme de solutions ou suspensions pour perfusion ou pour injection.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée, comme aussi expliqué dans la littérature, par exemple dans le livre «Pharmaceutical Dosage Forms and Drug Delivery » (2007 ; édité par Mahato R ; publié par CRC Press).

Il est entendu que le débit et/ou la dose injectée peuvent être adaptés par l'homme du métier en fonction du sexe, l'âge et le poids du patient, de la pathologie, du mode d'administration, ou des traitements éventuellement associés. Typiquement, les composés sont administrés à des doses pouvant varier entre 1 µg et 2 g par administration, préférentiellement de 0,1 mg à 1 g par administration. Les administrations peuvent être quotidiennes voire répétées plusieurs fois par jour, le cas échéant. D'autre part, les compositions selon l'invention peuvent comprendre, en outre, d'autres agents ou principes actifs. Les composés sont utilisés à une concentration généralement comprise entre 0,001% et 10% en poids, de préférence entre 0,01% et 1% en poids, par rapport au poids de la composition.

### ABREVIATIONS

- ACO : Acyl-CoenzymeA oxidase
- ApoCIII : Apolipoprotein CIII
- Cpd : Composé
- CPT1b : Carnitine Palmitoyl Transferase 1 b
- Ctrl : Contrôle
- Et : Ethyle
- Etp.: Etape
- Ex. Exemple
- G : Groupement dans la Formule Générale (I)
- HDL-cholesterol : High Density Lipoprotein cholesterol
- HOMA : Homeostatic Model Assessment
- Int.: Intermédiaire
- mpk : mg /kg/jour
- OMe : O-Méthyle
- OtBu : O-Tertiobutyle
- PDK4 : Pyruvate Deshydrogenase Kinase, isoforme 4
- Ph : Phényle
- Prot. Protocole
- PPAR : Peroxisome proliferator-activated receptor
- R, R₁-R₆, R/'/"/'"ₐ, R/'/"/"'_{b} Groupements dans la Formule Générale (I)
- SEt: S-Ethyle
- tBu : Tertiobutyle
- TG : Triglycérides
- UCP2 : Uncoupling Protein 2
- X₁-X₆ : Groupements dans la Formule Générale (I)
- Y₁, Y₂: Groupements dans la Formule Générale (I)

### ANALYSES STATISTIQUES

Les analyses statistiques des différents expériences pharmacologiques consistent en un test de Student (test-t). Les résultats sont exprimés par rapport au groupe contrôle selon la valeur du paramètre p : p<0,05 (noté *) ; p<0,01 (noté **) ; p<0,001 (noté ***).

### LEGENDES DES FIGURES

**Figure 1** **-** Schéma général pour la synthèse des composés selon l'invention_{.}
   Sauf pour des composés spécifiques qui sont indiqués dans les Exemples de 1 à 7, les intermédiaires générés en Exemple 2 (Figure 2) ont été utilisés pour synthétiser les intermédiaires de l'Exemple 3 (Figure 3). Ces derniers ont été utilisés pour synthétiser les intermédiaires de l'Exemple 4 (Figure 4), qui ont été ensuite utilisés pour synthétiser les intermédiaires de l'Exemple 5 (Figure 5). Les intermédiaires de l'Exemple 6 (Figure 6) ont été générés séparement. Comme décrit dans l'Exemple 7 (Figure 7) , les composés selon l'invention ont été synthétisés en utilisant soit les intermédiaires de l'Exemple 4 et de l'Exemple 6 soit les intermédiaires de l'Exemple 5 et de l'Exemple 6.

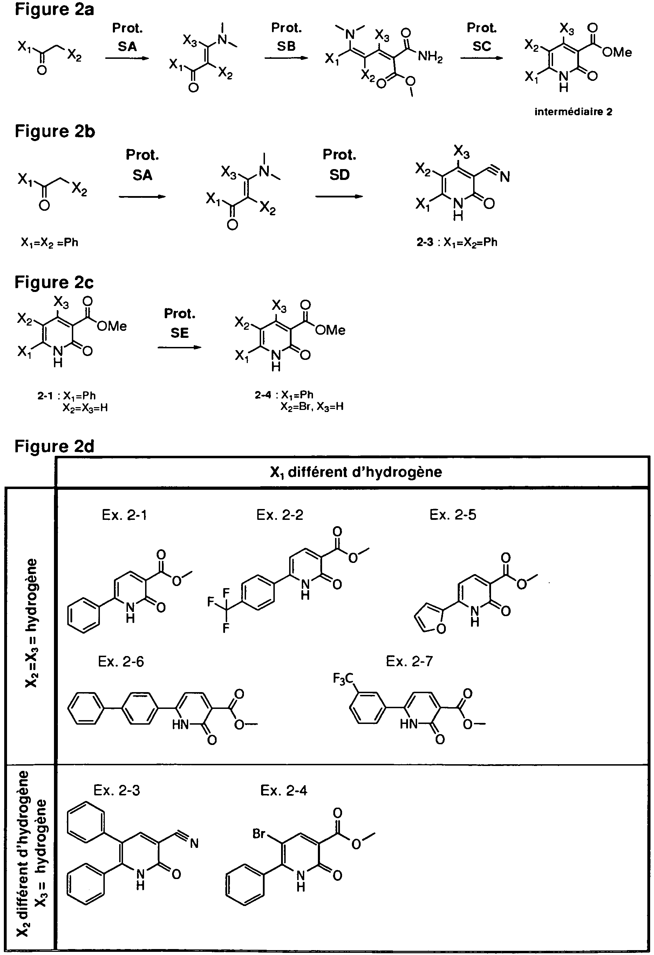

**Figure 2** - Intermédiaires de type 2-oxo-1,2-dihydropyridine
   Schéma réactionnel pour la synthèse des intermédiaires de l'Exemple 2: Ex. 2-1, 2-2 et 2-5 à 2-7 (Figure 2a) ; 2-3 (Figure 2b); 2-4 (Figure 2c). Ces intermédiaires peuvent être classés selon leurs groupements X₁, X₂ et X₃ (Figure 2d).

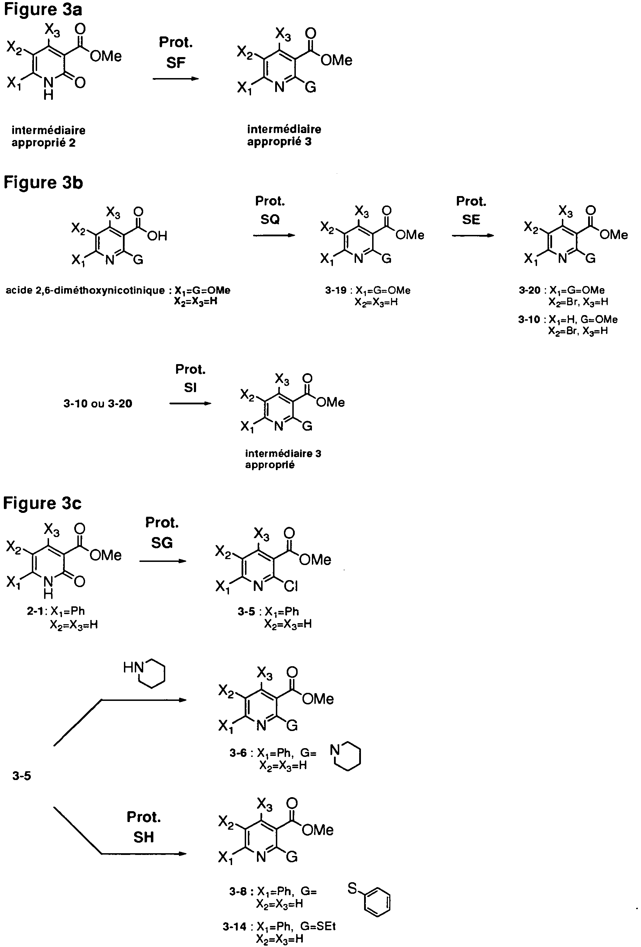

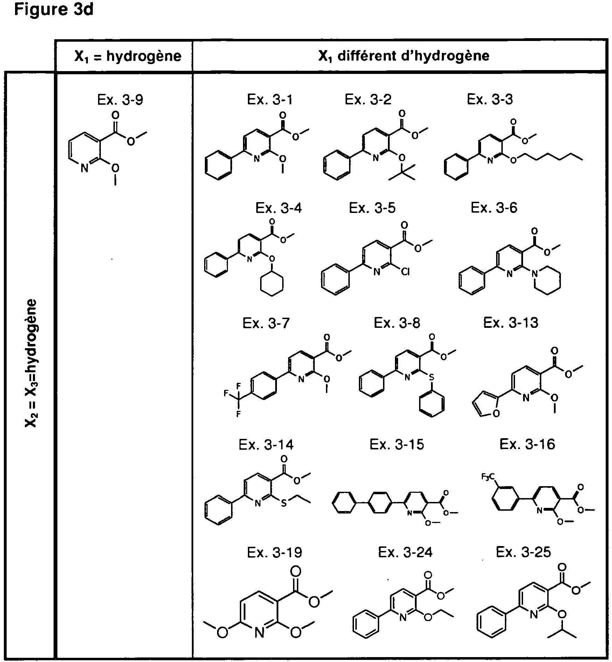

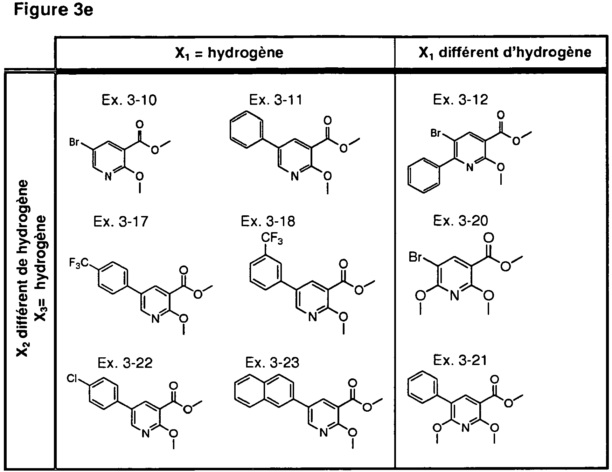

**Figure 3** - Intermédiaires de type alkoxy, alkylthio-, alkylamino-, halo-pyridine
   Schéma réactionnel pour la synthèse des intermédiaires de l'Exemple 3: Ex. 3-1 à 3-4, 3-7, 3-9, 3-12, 3-13, 3-15, 3-16, 3-24 et 3-25 (Figure 3a) ; 3-10, 3-11, 3-17, 3-18, 3-19 à 3-23 (Figure 3b) ; 3-5, 3-6, 3-8 et 3-14 (Figure 3c). Ces intermédiaires peuvent être classés selon leurs groupements X₁, X₂ et X₃ (Figures 3d et 3e).

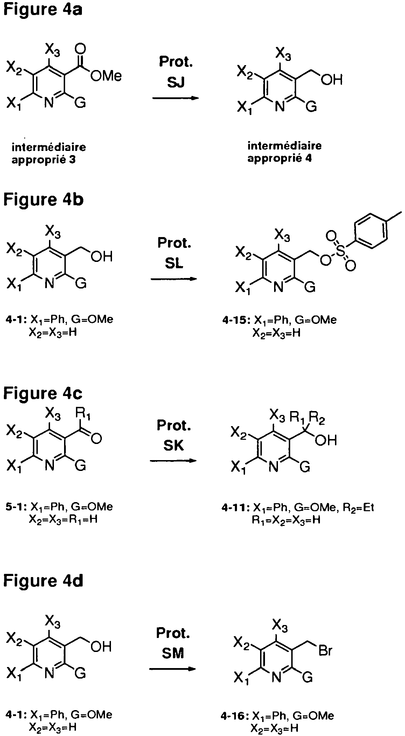

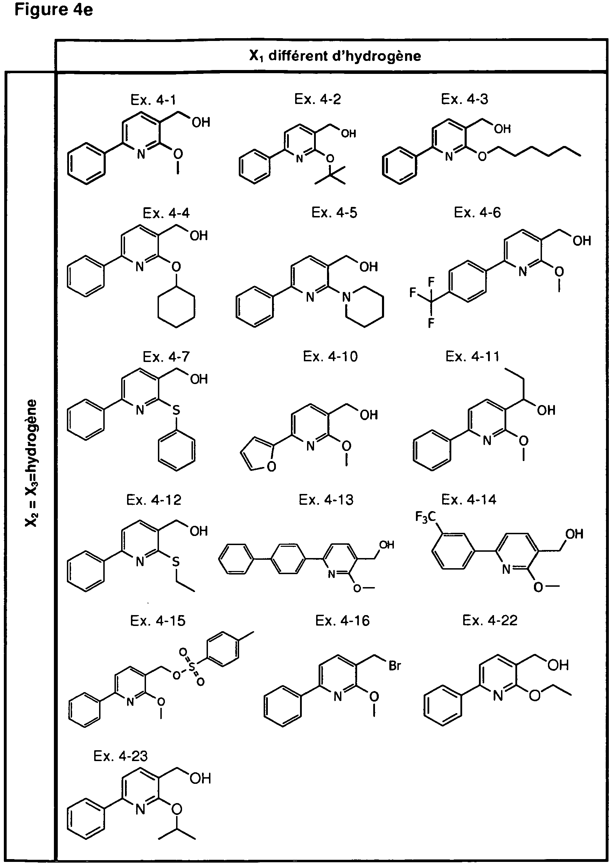

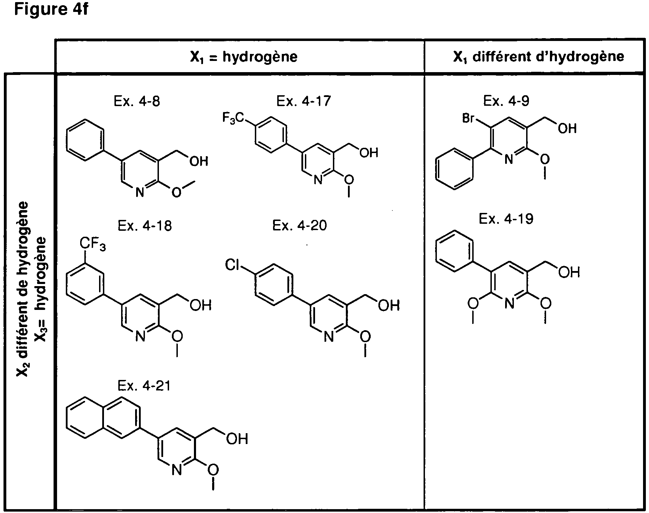

**Figure 4** - Intermédiaires de type 3-hydroxyméthyl-, 3-halométhyl- et 3-arylsulfonylméthylpyridine
   Schéma réactionnel pour la synthèse des intermédiaires de l'Exemple 4: Ex. 4-1 à 4-10, 4-12, 4-13, 4.14, 4-17 à 4-23 (Figure 4a) ; 4-15 (Figure 4b) ; 4-11 (Figure 4c) ; 4-16 (Figure 4d). Ces intermédiaires peuvent être classés selon leurs groupements X₁, X₂ et X₃ (Figures 4e et 4f).

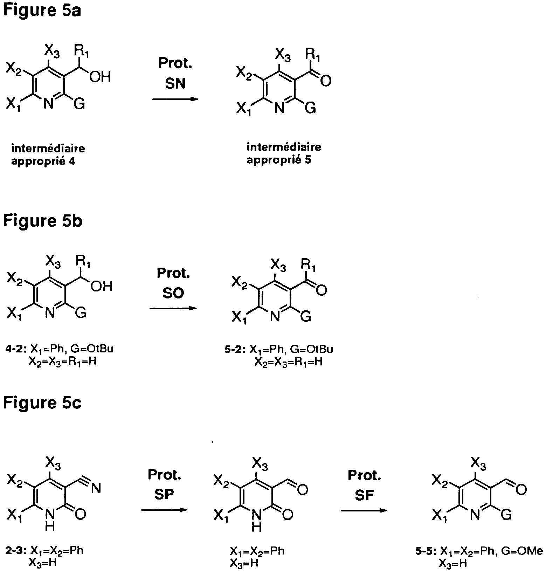

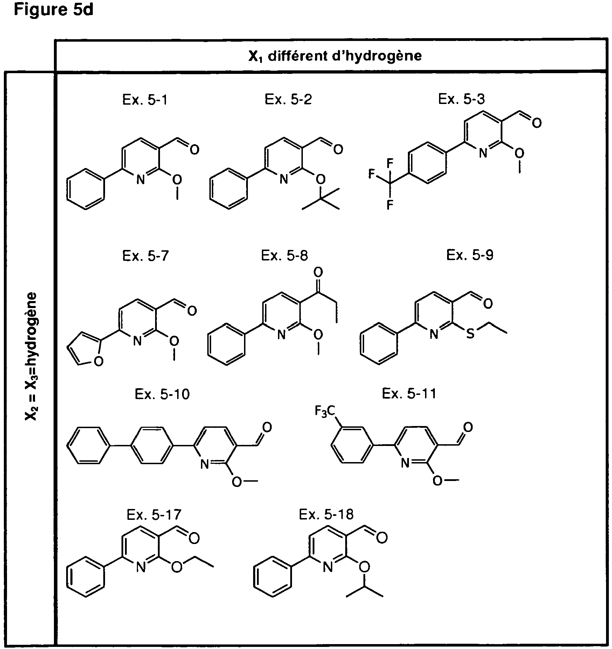

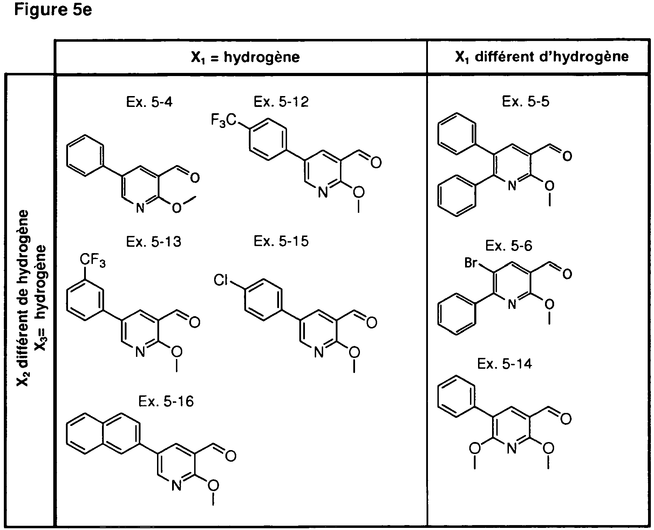

**Figure 5** - Intermédiaires de type Pyridine 3-carboxaldéhyde et cétone
   Schéma réactionnel pour la synthèse des intermédiaires de l'Exemple 5: Ex.5-1, 5-3, 5-4, 5-6 à 5-18 (Figure 5a) ; 5-2 (Figure 5b) ; 5-5 (Figure 5c). Ces intermédiaires peuvent être classés selon leurs groupements X₁, X₂ et X₃ (Figure 5d et 5e).

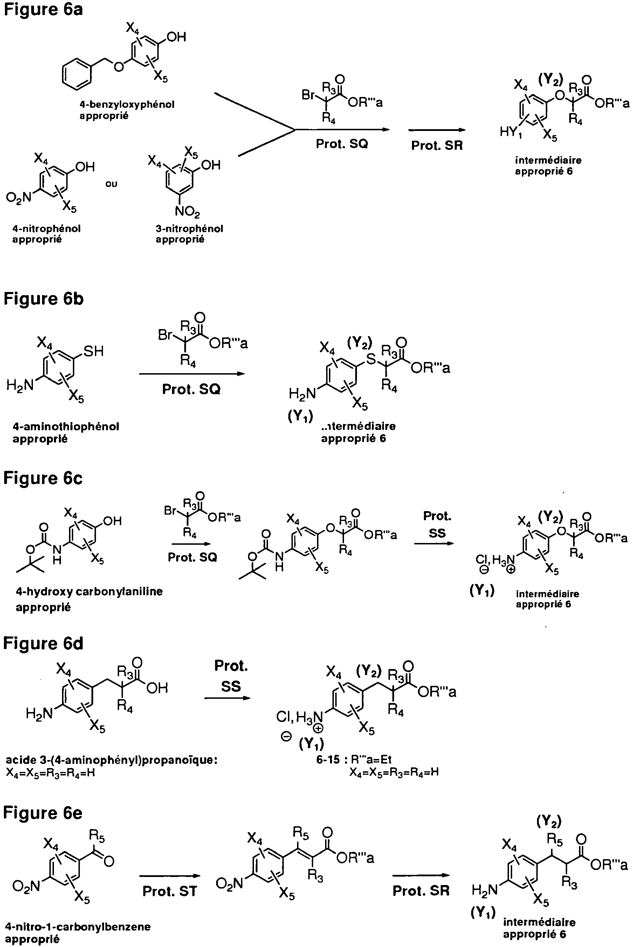

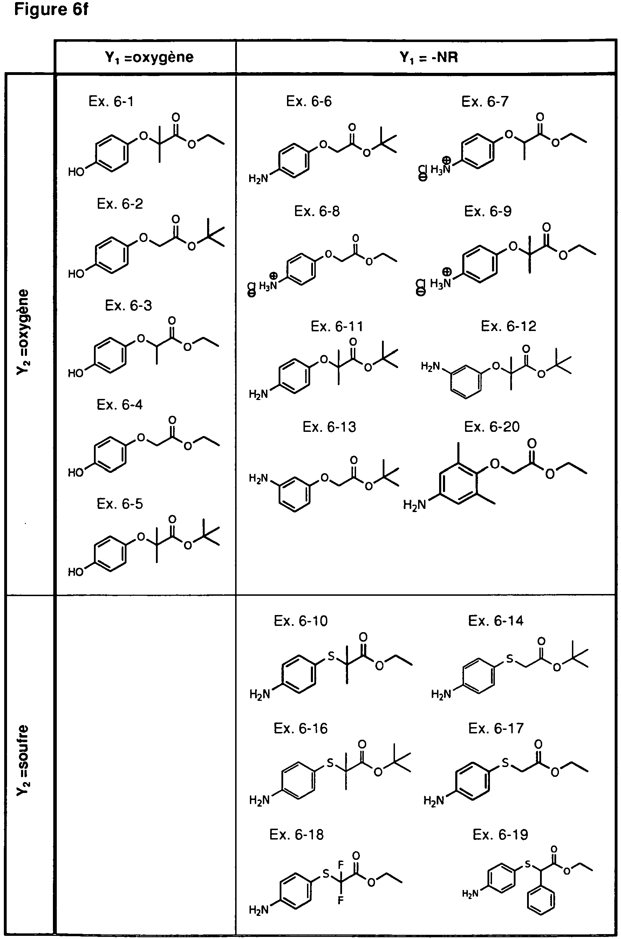

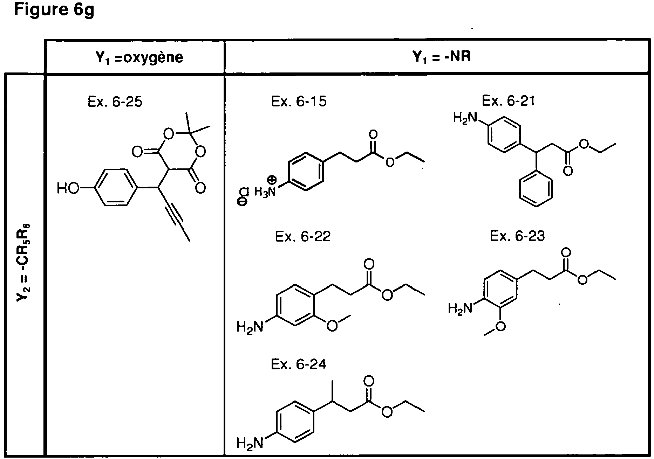

**Figure 6** - Intermédiaires de type phénol, thiophénol et aniline
   Schéma réactionnel pour la synthèse des intermédiaires de l'Exemple 6 : Ex.6-1 à 6-6, 6-11 à 6-13, et 6-20 (Figure 6a) ; 6-10, 6-14, 6-16 à 6-19 (Figure 6b) ; 6-7 à 6-9 (Figure 6c) ; 6-15 (Figure 6d) ; 6-21 à 6-24 (Figure 6e). Ces intermédiaires peuvent être classés selon leurs groupements Y₁ et Y₂ (Figure 6f et 6g).
**Figure 7** - Composés selon l'invention
   Schéma réactionnel pour la synthèse des composés selon l'invention : Cpd 4, Cpd 8-12, Cpd 16, Cpd 19, Cpd 22, Cpd 23, Cpd 26-32, Cpd 35, Cpd 36, Cpd 38-51, et Cpd 54 (Figure 7a) ; Cpd 13 et Cpd 14 (Figure 7b) ; Cpd 34 (Figure 7c) ; Cpd 2, Cpd 6, Cpd 7, Cpd 15, Cpd 17, Cpd 18, Cpd 20, Cpd 21, Cpd 24, Cpd 25, Cpd 52, Cpd 53 (Figure 7d) ; Cpd 1, Cpd 3, Cpd 5, Cpd 33 (Figure 7e) ; Cpd 37 (Figure 7f). Les composés selon l'invention peuvent être classés selon leurs groupements X₁, X₂, X₃, Y₁ et Y₂ (Figures 7g, 7h, et 7i).
**Figure 8** - Caractère antidiabétique des composés selon l'invention
   L'effet de Cpd 24 a été évalué *in vivo* chez la souris db/db. Les taux plasmatique de glucose (Figure 8a), d'insuline (Figure 8b), et l'index HOMA (Figure 8c) ont été mesurés après 8 jours de traitement avec Cpd 24 administré à 30 mpk chez la souris db/db. La différence mesurée témoigne de l'effet de Cpd 24 sur les paramètres d'insulino-résistance. Une diminution de l'index HOMA, calculé à partir de ces paramètres plasmatiques, est aussi le reflet d'une amélioration de la sensibilité à l'insuline.
   L'effet de Cpd 24 a été aussi évalué par la mesure de la tolérance au glucose. La glycémie a été mesurés chez la souris db/db traitée avec Cpd 24, administré à 30 mpk pendant 9 jours, après administration orale d'une dose unique de glucose (jour 0), pour obtenir les courbes cinétiques de glycémie (Figure 8d ; les aires sous les courbes du test de tolérance au glucose sont représentées dans Figure 8e). L'action corrective de Cpd 24 sur l'insulino-résistance se traduit par une amélioration de la tolérance au glucose. Les taux sont comparés à ceux obtenus chez des animaux contrôles (non traités).

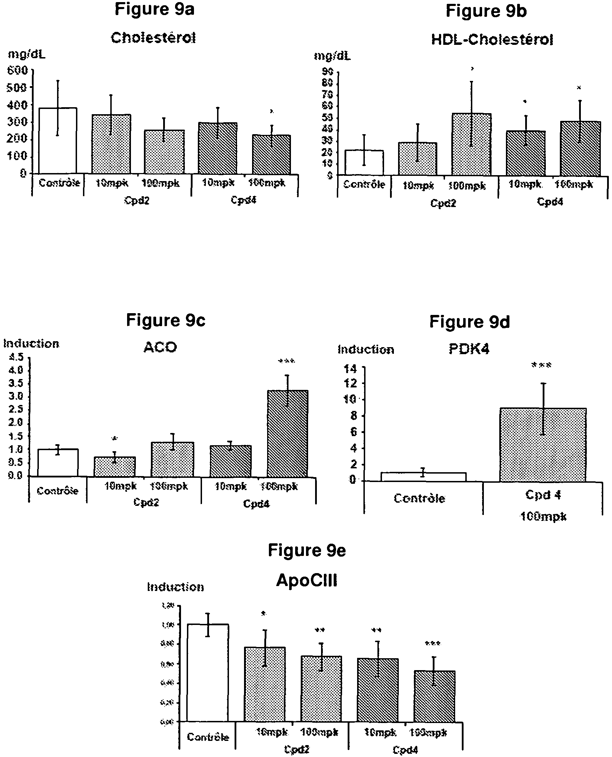

**Figure 9** - Propriétés hypolipémiantes et stimulatrices de la synthèse du HDL-cholestérol des composés selon l'invention
   Les taux plasmatiques de cholestérol total (Figure 9a) et de HDL-cholestérol (Figure 9b) ont été mesurés chez la souris dyslipidémique ApoE2/E2 après 7 jours de traitement par voie orale avec les Cpd 2 ou Cpd 4, administrés à 10 et 100 mpk. Ces paramètres sont comparés à ceux obtenus chez des animaux contrôles (non traités) : la différence mesurée témoigne de l'effet hypolipémiant des composés selon l'invention.
   L'effet de Cpd 2 et Cpd 4 a été aussi évaluée dans le tissu hépatique de la souris ApoE2/E2 par la mesure de l'expression de gènes impliqués dans le métabolisme lipidique et/ou glucidique comme ACO (Figure 9c), PDK4 (Figure 9d) et Apo CIII (Figure 9e). Les niveaux d'expression de chaque gène sont normalisés par rapport au niveau d'expression du gène de référence 36B4. Ces paramètres sont comparés à ceux obtenus chez des animaux contrôles (non traités) : le facteur d'induction, c'est-à-dire le rapport entre le signal relatif (induit par le composé selon l'invention) et la moyenne des valeurs relatives du groupe contrôle, est ensuite calculé. Plus ce facteur est élevé, plus le composé a un caractère activateur d'expression génique. Le résultat final est représenté comme moyenne des valeurs d'induction dans chaque groupe expérimental.

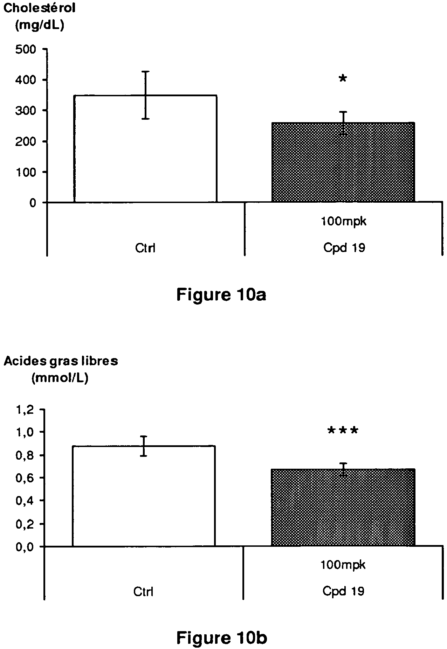

**Figure 10** - Propriétés hypolipémiantes des composés selon l'invention
   Les taux plasmatiques de cholestérol total (Figure 10a) et d'acides gras libres (Figure 10b) ont été mesurés chez la souris dyslipidémique E2/E2 après 7 jours de traitement par voie orale avec Cpd 19, administré à 100 mpk. Ces paramètres sont comparés à ceux obtenus avec des animaux contrôles (non traités) : la différence mesurée témoigne de l'effet hypolipémiant des composés selon l'invention.
**Figure 11** - Propriétés activatrices de PPARδ des composés selon l'invention
   Les effets stimulateurs du métabolisme lipidique, glucidique et de la dépense énergétique dans le muscle squelettique des composés selon l'invention ont été évalués par la mesure de l'expression de PDK4 (Figure 11a), CPT1b (Figure 11b), et UCP2 (Figure 11c) dans des myocytes murins traités pendant 24 heures avec Cpd 7 ou Cpd 24 à différentes concentrations (0,1, 1, 10µM et 0,2, 2 et 20µM respectivement). L'expression de ces gènes dans ce type cellulaire est directement la conséquence de l'activation de PPARδ par les composés selon l'invention. Plus l'expression des gènes est augmentée, plus le composé selon l'invention est activateur de PPARδ et donc stimulateur du métabolisme dans les cellules musculaires. Les niveaux d'expression présentés sont normalisés par rapport au niveau d'expression du gène de référence 36B4.
**Figure 12** - Propriétés activatrices de PPARγ des composés selon l'invention
   Les effets stimulateurs de l'adipogénèse de Cpd 19, Cpd 24 et Cpd36 ont été évalués par la mesure de l'accumulation de TG (Figures 12a et 12c) et de la sécrétion d'adiponectine (Figures 12b et 12d) dans un modèle *in vitro* de pré-adipocytes murins. Après 9 jours de traitement par les composés en dose réponse de 30nM à 30µM, les taux ont été comparés à ceux mesurés dans des cellules non traitées : plus les concentrations de TG et d'Adiponectine sont augmentées, plus les composés ont un caractère adipogénique et sont donc activateurs de PPARγ. L'activité des composés selon l'invention est aussi comparée aux effets d'une molécule de référence agoniste des PPARγ (Rosiglitazone). Plus l'accumulation de triglycérides et la sécrétion d'adiponectine sont augmentées, plus le composé selon l'invention est activateur de PPARγ et donc stimulateur du métabolisme dans les cellules adipocytaires.

### EXEMPLES

### Exemple 1 : Protocoles généraux

Les composés de l'invention sont préparés selon les méthodes générales et les protocoles généraux de synthèse SA à SY ci-après. Les composés selon l'invention et les correspondants intermédiaires réactionnels ont été caractérisé au niveau structrurale par RMN ¹H (300MHz ; CDCl₃, CDCl₃+D₂O, ou DMSO-d₆, la dernière conditions notamment pour les composés selon l'invention; δ en ppm).

### Protocole SA :

La cétone adequate (Acétophénone pour Ex. 2-1 ; 4-trifluoroacétophénone pour Ex. 2-2 ; 2-phénylacétophénone pour Ex. 2-3 ; 2-acétylfurane pour Ex. 2-5 ; para-acétylbiphényle pour Exemple 2-6 ; 3-trifluorométhylacétophénone 2.7) est solubilisée dans de l'acétal diméthylique de N,N-diméthylformamide (1,2 à 1,5 éq.). Le milieu est maintenu sous agitation à reflux. Le temps de réaction estimé varie entre 24 et 48 heures. Des résultats satisfaisants ont été obtenus en 24 heures. Protocole SB :

La prop-2-én-1-one de l'étape précédente (voir Exemples 2-1, 2-2, 2-5, 2-6, et 2-7) est solubilisés dans du méthanol (0,3 à 1,5 mol/L). Le milieu est maintenu sous agitation à reflux. Le temps de réaction estimé varie entre 18 et 48 heures. Des résultats satisfaisants ont été obtenus en 18 heures.

### Protocole SC :

L'ester méthylique de l'étape précédente (voir Exemples 2-1, 2-2, 2-5, 2-6, 2-7) est solubilisé dans du toluène (0,15 à 0,5 mol/L) puis de l'acide acétique (1 à 1,5 éq.) est ajouté. Le mélange est maintenu sous agitation à reflux. Le temps de réaction estimé varie entre 12 et 48 heures. Des résultats satisfaisants ont été obtenus en 18 heures. Après retour à température ambiante, le milieu réactionnel est refroidi à 0°C et le précipité formé est filtré puis, lavé par de l'acétone.

### Protocole SD :

L'aminopropénone de l'étape précédente, le cyanoacétamide (1,1 éq.) et le méthanol (2 éq.) sont solubilisés dans du N,N-diméthylformamide (0,3 mol/L). Cette solution est additionnée à une suspension de NaH (2 éq.) dans du N,N-diméthylformamide (3 mol/L). L'ensemble est maintenu sous agitation à 95°C pendant 18 heures.

### Protocole SE :

La pyridinone de l'étape précédente (Ex. 3-9 pour Ex. 3-10 ; Ex. 3-19 pour Ex. 3-20) et la N-bromosuccinimide (1 à 1,1 éq.) sont solubilisées dans du N,N-diméthylformamide (0,1 à 0,4 mol/L). Le milieu réactionnel est maintenu sous agitation à reflux. Pour un rendement d'au moins 30-80 %, le temps de réaction estimé varie entre 4 et 16 heures. Des résultats satisfaisants ont été obtenus en 4 heures. Après le retour à température ambiante, le précipité formé est essoré et lavé à l'eau et/ou à l'heptane.

### Protocole SF :

La 2-oxo-1,2-dihydropyridine (Ex. 2-1 pour Ex. 3-1 à 3-4, 3-24, 3-25 ; Ex. 2-2 pour Ex. 3-7 ; Ex. 2-4 pour Ex. 3-12 ; Ex. 2-5 pour Ex. 3-13 ; Ex. 2-6 pour Ex. 3-15 ; Ex. 2-7 pour Ex. 3-16 ; 3-18 ; acide 2-hydroxynicotinique pour Ex. 3-9) est solubilisée dans du toluène (0,06 à 0,4 mol/L) puis, l'oxyde d'argent (1 à 1,2 éq.) et le dérivé halogéné (par exemple, 1 à 1,2 de éq. ; iodure de méthyle pour Ex. 3-1, 3-7, 3-9, 3-12, 3-15, 3-16, 3-19, 5-5; iodure d'éthyle pour Ex. 3-24 ; iodure d'isopropyle pour Ex. 3-25 ; bromure de tertiobutyle pour Ex. 3- 2; 1-iodohexane pour Ex. 3-3 ; iodocyclohexane pour Ex. 3-4) sont successivement ajoutés. Le milieu réactionnel est maintenu sous agitation à reflux à une temperature qui varie entre 50°C et 70°C, et les sels sont écartés par filtration. Pour un rendement d'au moins 40-80 % après purification selon le protocole de purification PA, le temps de réaction estimé varie entre 1 et 48 heures. Des résultats satisfaisants ont été obtenus en 16 heures. La réaction peut être avantageusement menée au reflux de l'acétonitrile pour améliorer la solubilité du milieu réactionnel.

### Protocole SG :

La 2-oxo-1,2-dihydropyridine (Ex. 2-1 pour Ex. 3-5) est solubilisée dans le trichlorure de phosphoryle (10 éq.) en présence d'une quantité catalytique de N,N-diméthylformamide. Le milieu réactionnel est maintenu sous agitation à reflux pendant 16 heures.

### Protocole SH :

L'halopyridine (Ex. 3-5 pour Ex. 3-8, 3-14) est solubilisée dans de l'acétonitrile (0,06 à 0,4 mol/L) et le carbonate de potassium (2 à 3 éq.) est ajouté. Le thiol (par exemple 1,2 à 3 éq. ; thiophénol pour Ex. 3-8 ; éthanethiol pour Ex. 3-14) est additionné goutte à goutte et l'ensemble est agité à reflux. Pour un rendement d'au moins 80 %, le temps de réaction estimé varie entre 18 et 48 heures. Des résultats satisfaisants ont été obtenus en 18 heures.

### Protocole SI :

L'intermédiaire (0,15 mol/L ; Ex. 3-1 pour Ex. 3-11 ; Ex. 3-10 pour Ex. 3-17, Ex. 3-18, Ex. 3-22, Ex. 3-23 ; Ex. 3-20 pour 3-21), le carbonate de potassium (3 éq.) et l'eau (11 éq.) sont solubilisés dans du N,N-diméthylformamide sous atmosphère inerte. L'acétate de palladium (0,1 éq.) est additionné, puis la solution d'acide boronique (acide phénylboronique pour Ex. 3-11 et 3-21 ; acide 4-trifluorométhyllboronique pour Ex. 3-17 ; acide 3-trifluorométhylphénylboronique pour Ex. 3-18 ; acide 4-chlorophénylboronique pour Ex. 3-22 ; acide naphtalèn-2-ylboronique pour Ex. 3-23) dans du N,N-diméthylformamide (1,5 éq., 1 mol/L) est ajoutée goutte à goutte. Le temps de réaction estimé varie entre 16 et 48 heures Le milieu est laissé sous agitation, sous atmosphère inerte à température ambiante.

### Protocole SJ :

L'ester de 2-alkoxy-1,2-dihydropyridine (Ex. 3-1 pour Ex. 4-1 ; Ex. 3-2 pour Ex. 4-2 ; Ex. 3-3 pour Ex. 4-3 ; Ex. 3-4 pour Ex. 4-4; Ex. 3-6 pour Ex. 4-5; Ex. 3-7 pour Ex. 4-6; Ex. 3-8 pour Ex. 4-7; Ex. 3-11 pour Ex. 4-8; Ex. 3-12 pour Ex. 4-9; Ex. 3-13 pour Ex. 4-10; Ex. 3-14 pour Ex. 4-12; Ex. 3- pour Ex. 4- ; Ex. 3-15 pour Ex. 4-13; Ex. 3-16 pour Ex. 4-14; Ex. 3-17 pour Ex. 4-17; Ex. 3-18 pour Ex. 4-18 ; Ex. 3-21 pour Ex. 4-19 ; Ex. 3-22 pour Ex. 4-20 ; Ex. 3-23 pour Ex. 4-21; Ex. 3-24 pour Ex. 4-22; Ex. 3-25 pour Ex. 4-23) est solubilisé dans du tétrahydrofurane (0,05 à 1,1 mol/L) et la solution est refroidie à 0°C. Le tétrahydruroaluminate de lithium (1 à 2 éq.) est ajouté par portions et l'ensemble est maintenu sous agitation à température ambiante. Le milieu réactionnel est traité par de l'eau (2,5 éq.), de la soude à 15% (17 éq.) puis, dilué par de l'eau (7,5 éq.) et maintenu sous agitation. Le temps de réaction estimé varie entre 1 et 24 heures.

### Protocole SK :

La pyridine carboxaldéhyde (Ex. 5-1 pour Ex. 4-11) est solubilisée dans du tétrahydrofurane (0,45 mol/L) et la solution est refroidie à -78°C. Une solution d'éthylmagnésium bromide (3,4 éq., 2M) est ajoutée goutte à goutte. L'ensemble est maintenu sous agitation pendant 18 heures à température ambiante.

### Protocole SL :

L'hydroxyméthylpyridine (Ex. 4-1 pour Ex. 4-15) et la triéthylamine (1,5 éq.) sont solubilisées dans du tétrahydrofurane (1 mol/L) puis, le chlorure de paratoluène sulfonyle (1,5 éq.) est ajouté. L'ensemble est maintenu sous agitation à reflux pendant 16 heures.

### Protocole SM :

L'hydroxyméthylpyridine (Ex. 4-1 pour Ex. 4-16) est solubilisée dans du dichlorométhane (0,2 mol/L) puis, la solution est refroidie à 0°C. Le tribromure de phosphore (1 éq.) est ajouté. L'ensemble est maintenu sous agitation à 0°C. Après 0,2 heures, le milieu réactionnel est versé sur de la glace pillée puis extrait par du dichlorométhane.

### Protocole SN :

L'hydroxyméthylpyridine (Ex. 4-1 pour Ex. 5-1 ; Ex. 4-6 pour Ex. 5-3 ; Ex. 4-8 pour Ex. 5-4 ; Ex. 4-9 pour Ex. 5-6 ; Ex.4-10 pour Ex. 5-7 ; Ex. 4-11 pour Ex. 5-8 ; Ex. 4-12 pour Ex. 5-9 ; Ex. 4-13 pour Ex. 5-10 ; Ex. 4-14 pour Ex. 5-11 ; Ex. 4.17 pour Ex. 5-12 ; Ex. 4-18 pour Ex. 5-13 ; Ex. 4-19 pour Ex. 5-14 ; Ex. 4-20 pour Ex. 5-15 ; Ex. 4-21 pour Ex. 5-16 ; Ex. 4-22 pour Ex. 5-17 ; Ex. 4-23 pour Ex. 5-18) est solubilisée dans du dichlorométhane (0,06 à 0,5 mol/L) puis, le chlorochromate de pyridinium (PCC ; 1,2 à 2 éq.) est ajouté. Le milieu réactionnel est maintenu sous agitation à température ambiante. Le temps de réaction estimé varie entre 2 et 48 heures.

### Protocole SO :

L'hydroxyméthylpyridine (Ex. 4-2 pour Ex. 5-2) est solubilisée dans du dichlorométhane (1,5 mol/L) puis, la solution est refroidie à 0°C. Le réactif de Dess-Martin (1,1 éq.) est ajouté goutte à goutte et l'ensemble est maintenu sous agitation à 0°C.

### Protocole SP :

Le 1,2-dihydropyridine-carbonitrile (Ex. 2-3 pour Ex. 5-5) est dissous dans de l'acide formique (1,1 mol/L) et le Nickel de Raney (50%Wt) est ajouté (1,5 éq.). L'ensemble est agité pendant 2 heures à reflux avant le retour à température ambiante.

### Protocole SQ :

Le phénol (4-benzyloxyphénol pour Ex. 6-1 à 6-5 ; 3-nitrophénol pour Ex. 6-12, 6-13 ; 4-nitrophénol pour Ex. 6-6, 6-11 ; 4-nitro-2,6-diméthylphénol pour Ex. 6-20), le thiophénol (4-aminothiophénol pour Ex. 6-10, 6-14, 6-16 à 6-19), l'aniline (4-hydroxy-10-tertiobutoxycarbonylaniline pour Ex. 6-7, 6-8, 6-9) ou l'acide (acide 2,6 diméthoxynicotiniquel pour Ex. 3-19) est solubilisé dans le solvant approprié (0,2 à 1,2 mol/L) puis le dérivé halogéné ou le tosylate (1,2 à 3 éq. ; bromodifluoroacétate d'éthyle pour Ex. 6-18 ; iodure de méthyle pour Ex. 3-19 ; 2-bromopropanoate d'éthyle pour Ex. 6-3,6-7 ; bromoisobutyrate d'éthyle pour Ex. 6-1, 6-9, 6-10; bromoisobutyrate de tertiobutyle pour Ex. 6-2, 6-5, 6-11, 6-12, 6-16 ; bromoacétate de tertiobutyle pour Ex. 6-13, 6-14; bromoacétate d'éthyle pour Ex. 6-4, 6-8, 6-17, 6-20; bromoacétate de tertiobutyle pour Ex. 6-6 ; 2-bromo-2-phénylacétate d'éthyle pour Ex. 6-19) et le carbonate de potassium (2,5 à 6 éq.) sont ajoutés. Le milieu réactionnel est maintenu sous vive agitation à la température adéquate et, si nécessaire, au reflux de l'acétonitrile, du N,N-diméthylformamide, de l'acétonitrile d'un mélange acétonitrile/N,N-diméthylformamide (6%), ou en présence de tétrabutylammonium bromide (0,3 éq.). Pour les composés selon l'invention, le phénol, le thiophénol, l'aniline, ou l'acide a été préparé dans l'Exemple 6, et le dérivé halogéné ou le tosylate a été préparé dans l'Exemple 4 ou 5. Le temps de réaction estimé varie entre 0.1 et 48 heures et le milieu réactionnel est refroidi à température ambiante.

### Protocole SR :

L'ester de l'étape précédente est solubilisé dans le solvant approprié (0,3 à 1,2 mol/L de méthanol, éthanol, dichlorométhane, ou mélange méthanol/dichlorométhane 1/1 ou 2/1) puis, du palladium sur charbon (10%Wt) est ajouté en quantités catalytiques. L'ensemble est placé sous agitation, sous atmosphère d'hydrogène à la pression adéquate. Le temps de réaction estimé varie entre 4 et 120 heures d'agitation à température ambiante. Le catalyseur est éliminé par filtration.

### Protocole SS :

L'amine protégée (acide 3-(4-aminophényl)propanoïque pour Ex. 6-15) est solubilisée dans de l'éthanol (0,3 à 0,6 mol/L) puis une solution éthanolique d'acide chlorhydrique est ajoutée (2 éq.). Le milieu réactionnel est maintenu sous agitation à température ambiante. Le temps de réaction estimé varie entre 2 et 16 heures,

### Protocole ST :

Le triéthylphosphonacétate (0,5 mol,L) est additionné goutte à goutte à une suspension d'hydrure de sodium (1éq.) dans du tétrahydrofurane placée à 0°C. Après une demi-heure d'agitation à température ambiante, le dérivé carbonylé (1 éq. ; 4-nitrobenzophenone pour Ex. 6-21; 4-nitroacétophénone pour Ex. 6-24; 3-méthoxy-4-nitrobenzaldéhyde pour Ex. 6-23; 2-méthoxy-4-nitrobenzaldéhyde pour Ex. 6-22) est ajouté et le milieu réactionnel est porté à reflux pendant 16 heures.

### Protocole SU :

L'ester de l'étape précédente est solubilisé dans de l'éthanol (0,03 à 1 mol/L) puis une solution de soude 1 N ou 2N (1 à 84 éq., préférentiellement de 2 à 20 éq.) est additionnée. L'ensemble est maintenu sous agitation à température ambiante. Pour un rendement d'au moins 30-85 % après purification selon l'une des alternatives présentées en exemple 2, le temps de réaction estimé varie entre 1 et 96 heures. Des résultats satisfaisants ont été obtenus en 16 heures. Le cas échéant, du tétrahydrofurane peut être avantageusement ajouté pour améliorer la solubilité du milieu réactionnel.

### Protocole SV :

Le phénol (0,1 à 0,9 mol/L), la triphénylphosphine (1,05 éq.) sont solubilisés dans du tétrahydrofurane sous atmosphère inerte. L'azodicarboxylate de diisopropyle (1,05 éq.) et la solution d'alcool dans du tétrahydrofurane (1,05 éq., 0,1 à 0,9 mol/L) sont successivement additionnés goutte à goutte. L'ensemble est maintenu sous agitation à température ambiante. Pour un rendement d'au moins 30-80 %, le temps de réaction estimé varie entre 16 et 72 heures. Des résultats satisfaisants ont été obtenus en 16 heures. Le cas échéant, du dichlorométhane peut être avantageusement utilisé pour améliorer la solubilité du milieu réactionnel ; l'utilisation d'un micro-onde (par exemple 0.1 heure à 50°C) peut améliorer notablement les performances de la réaction.

### Protocole SW :

L'ester de tertiobutyle de l'étape précédente est solubilisé dans le dichlorométhane (0,08 à 0,2 mol/L), l'acide trifluoroacétique (10 à 72 éq., préférentiellement 10 éq.) est additionné. L'agitation est maintenue à température ambiante. La réaction est menée à température ambiante et sous agitation. Le précipité est filtré, repris dans de l'eau (0,2 Umol) et traité par une solution de soude 2N (3 éq.) pendant 0,5 heures. L'ensemble est acidifié par une solution d'acide citrique 1 N, sous vive agitation puis, filtré. Pour un rendement d'au moins 30-80 %, le temps de réaction estimé varie entre 16 et 24 heures. Des résultats satisfaisants ont été obtenus en 16 heures. Le milieu réactionnel peut être traité sous vive agitation par une solution de carbonate de potassium à 10% pendant 0,5 heures (pH=8-9) avant d'être acidifié.

### Protocole SX :

L'aldéhyde et l'aniline (1 à 1,5 éq.) sont solubilisés dans du dichlorométhane (0,1 à 0,4 mo/L), sous atmosphère inerte. Le milieu réactionnel est maintenu sous agitation à température ambiante pendant 1 heure. Le triacétoxyborohydrure de sodium (1,2 à 1,5 éq.) est additionné par portions, puis l'ensemble est maintenu sous vive agitation à température ambiante. Lorsque l'aniline requise est disponible sous sa forme chlorhydrate, elle est au préalable re-larguée par un traitement basique. Pour un rendement d'au moins 40-85 % après purification selon l'une des alternatives présentées en exemple 2, le temps de réaction estimé varie entre 2.5 et 72 heures. Des résultats satisfaisants ont été obtenus en 16 heures. Travailler avec un solvant anhydre et/ou en présence d'un tamis moléculaire 3Å peut améliorer notablement les performances de la réaction.

### Protocole SY :

La cétone et l'aniline (1,2 éq.) sont solubilisés dans du toluène (0,03 mo/L), en présence d'acide paratoluènesulfonique (1 éq.) et de tamis moléculaire 3Å, dans un montage Dean-Stark. Le milieu réactionnel est maintenu sous agitation à 110°C pendant 16 heures. Après refroidissement les insolubles sont filtrés et le filtrat est concentré sous pression réduite. Le résidu d'évaporation est repris dans du dichlorométhane. Le triacétoxyborohydrure de sodium (1,5 éq.) est additionné par portions, puis l'ensemble est maintenu sous vive agitation à température ambiante en présence de tamis moléculaire 3Å. pendant 48 heures. Lorsque l'aniline requise est disponible sous sa forme chlorhydrate, elle est au préalable re-larguée par un traitement basique.

Les bruts réactionnels obtenus peuvent être purifiés suivant l'un ou plusieurs des protocoles généraux de purification PA à PE, A cet effet, des étapes préalables classiques, d'hydrolyse (sans ou avec diluiton, par exemple, par de l'acétate d'éthyle) ou de lavage (neutre, acide ou basique, par exemple au moyen d'eau, d'une solution saturée de chlorure de sodium, d'une solution d'acide citrique 1N, d'une solution saturée de chlorure d'ammonium, d'une solution de carbonate de potassium à 10%, d'une solution d'hydroxyde de sodium 1N) puis, d'extraction du milieu réactionnel à l'aide d'un solvant approprié (éther diéthylique ou dichlorométhane, par exemple), de séchage des phases organiques (par exemple, sur sulfate de magnésium), de concentration (notamment du milieu réactionnel, notamment par évaporation sous pression réduite), et/ou d'élimination des insolubles peuvent être avantageusement entreprises (les sels sont éliminés par filtration, par exemple).

Dans le cas du protocole PA, la chromatographie flash sur gel de silice (40-63µm) a été performé avec des différentes conditions d'elution (phase mobile), comme résumé dans le Tableau 1-1.

**Tableau 1-1**

| **Phase Mobile** | **Exemples** |
|---|---|
| dichlorométhane/méthanol ou (9/1 à 98/2) | Etp. 1 pour Ex. 2-5, 4-1, 4-7, 5-5 ; Cpd 33 ; |
| | Etp. 2 pour Cpd 1, Cpd 8, Cpd 10, Cpd 11, Cpd 15, Cpd 21, Cpd 22, Cpd 24, Cpd 28, Cpd 29, Cpd 32-36, Cpd 38-48, Cpd 50, Cpd 51, Cpd 53, Cpd 54 |
| | Etp. 3 pour 2-7, |
| dichlorométhane/ cyclohexane (8/2) | Etp. 2 pour 5-5 |
| dichlorométhane/acétate d'éthyle ou cyclohexane/acétate d'éthyle (95/5 ou 98/2) | Etp. 1 pour Ex. 3-3, 3-4, 3-12, 5-1, 5-12, 5-13, 5-15, 5-175-6, 5-18, et 6-5 ; Cpd 3, Cpd 6, Cpd 7, Cpd 13-18, Cpd 32, Cpd 34, Cpd 36, Cpd 38, Cpd 42, Cpd 43, Cpd 46-48 |
| | Etp. 2 pour Cpd 15 |
| dichlorométhane/acétate d'éthyle ou cyclohexane /acétate d'éthyle (9/1 ou 85/15) | Etp. 1 pour Ex. 2-3 , 3-2, 3-5, 3-7, 3-8, 3-11, 3-13, 3-14, 3.22, 3-23, 3-24, 4-5, 4-9, 4-11, 4-21, 5-2, 5-4, 5-7, 5-8, 5-9, 5-14, 5-16, 6-1, 6-3, 6-4, 6-12, 6-14, 6-18, 6-22; Cpd 1, Cpd 2, Cpd 4, Cpd 5, Cpd 8, Cpd 9, Cpd 10, Cpd 11, Cpd 12, Cpd 16, Cpd 19-26, Cpd 30, Cpd 31, Cpd 39, Cpd 40, Cpd 41, Cpd 44, Cpd 45, Cpd 49-54 |
| | Etp. 2 pour Ex. 6-3, 6-12 |
| dichlorométhane/ acétate d'éthyle ou cyclohexane /acétate d'éthyle (7/3, 8/2, ou 6/4) | Etp. 1 pour Ex. 3-1, 3-9, 3-15, 3-16, 3-19, 3-25, 4-2, 4-6, 4-10, 4-12, 4-13, 4.14, 4-15, 4-19, 5-3, 5-10, 5-11, 6-10, 6-11, 6-13, 6-16, 6-17, 6-19, 6-21, 6-23, 6-24 ; Cpd 27, Cpd 28, Cpd 29, Cpd 35 ; |
| | Etp. 2 pour Ex. 6-5 6-21, 6-24 |

Dans le cas du protocole PB, une chromatographie sur gel de silice est accomplie par HPLC préparative (lichrospher, Merck; RP18 12µm 100A, colonne : 25*250 mm ; Etp. 2 pour Cpd 5, Cpd 13, Cpd 14, Cpd 17, Cpd 18, Cpd 20, Cpd 23, Cpd 52 ; Etp. 1 pour Cpd 37).

Dans le cas du protocole PC, une précipitation est accomplie dans un mélange de solvants qui sont choisis parmi les solvants usuels de l'homme de métier comme notamment le dichlorométhane, l'heptane, le cyclohexane, le Toluene, par exemple dichlorométhane/heptane 4/6 (Etp. 1 pour Ex. 2-1, 2-2, 2-6 ; Etp. 2 pour Cpd 12, Cpd 16, Cpd 19, Cpd 22, Cpd 27,Cpd 30, Cpd 31, Cpd 49 ; Etp. 3 pour Cpd 46, Cpd 47, Cpd 48, Cpd 51, Cpd 53), dichlorométhane/toluène 4/6 (Etp. 2 pour Ex. 2-5), ou dichlorométhane/cyclohexane 4/6 (Etp. 3 pour Cpd 45, Cpd 54).

Dans le cas du protocole PD, la (re)cristalisation est accomplie dans un solvant choisi parmi les solvants usuels de l'homme de métier comme l'isopropanol (Etp. 2 pour Cpd 9), l'éthanol (Etp. 2 pour Ex. 2-1, Cpd 25), l'Acetone (Etp. 2 pour Ex. 2-2), le méthanol (Etp. 2 pour Ex. 2-6, Cpd 3, Cpd 6, Cpd 7), l'éther de pétrole (Etp. 1 pour Ex. 6-2, 6-13), le dichlorométhane, l'heptane (Etp. 1 pour Ex. 6-11, 6-20), le cyclohexane (Etp. 1 pour Ex. 6-6, 6-7, 6-9), ou des mélange comme par exemple avec dichlorométhane/ heptane (Cpd 15, Cpd 21, Cpd 24, Cpd 28, Cpd 29, Cpd 32, Cpd 42, Cpd 43, Cpd 44)

Dans le cas du protocole PE, la filtration du produit qui précipite est accomplie à l'issu de la réaction. Le temps de réaction estimé varie entre 12 et 24 heures. Après que le milieu réactionnel est refroidi à 0°C, l'hydrolyse ou l'hydrolyse acide du milieu réactionnel (Etp. 3 pour Ex. 2-1, 2.2, 2-5 à 2-7, Etp. 3 pour Ex. 2-3, 2-4; Cpd 2, Cpd 4, Cpd 26 ; Etp. 1 pour Ex. 3-20) ou une séquence d'hydrolyse basique / filtration / trituration du gâteau en milieu aqueux acide (par exemple dans l'éthanol ; Etp. 2 pour Ex. 6-7, 6-8, 6-9; Etp. 1 pour Ex. 6-15) est accomplie.

### Exemple 2 : Synthèse des intermédiaires de type 2-oxo-1,2-dihydropyridine selon l'invention

La synthèse de ces Intermédiaires (Figure 2) nécessite 2 ou 3 étapes comme résumé dans le Tableau 2-1:

**Tableau 2-1**

| **Ex.** | **No. et type d'Etape (Protocole)** | **Détails** |
|---|---|---|
| **2-1** | 1 : Préparation du 3-diméthylamino-1-phénylprop-2-én-1-one (Protocole SA et PC) | Rendement : 92%. Aspect : solide jaune. |
| | | RMN ¹H: 2,93-3,14 (m, 6H); 5,73 (d, 1H, J=12,4Hz); 7,38-7,49 (m, 3H); 7,82 (d, 1H, J=12,4Hz); 7,89 (d, 2H, J=7,9Hz). |
| | 2 : Préparation du 2-carbamoyl-5-diméthylamino-5-phénylpentan-2,4-diènoate de méthyle (Protocole SB et PD) | Rendement : 63% |
| | 3 : Obtention du 2-oxo-6-phényl-1,2-dihydropyridine-3-carboxylate de méthyle (Protocole SC et PE) | Rendement : 66%. Aspect : solide jaune. |
| | | RMN ¹H: 3,77 (s, 3H); 6,79 (d, 1H, J=7,6Hz); 7,51-7,53 (m, 3H); 7,82-7,85 (m, 2H); 8,14 (d, 1H, J=7,6Hz). |
| **2-2** | 1 : Préparation du 3-diméthylamino-1-(4-trifluorométhylphényl)prop-2-én-1-one(Protocole SA et PC) | Rendement : 81%. Aspect : solide jaune. |
| | | RMN ¹H :2,94 (s, 3H); 3,17 (s, 3H); 5,69 (d, 1H, J=12,3Hz); 7,66 (d, 2H, J=8,2Hz); 7,83 (d, 1H, J=12,3Hz); 7,97 (d, 2H, J=8,2Hz). |
| | 2 : Préparation du 2-carbamoyl-5-diméthylamino-5-(4-trifluorométhylphényl)pentan-2,4-dienoate de méthyle (Protocole SB et PD) | Rendement : 37%. Aspect : solide jaune. |
| | 3 : Obtention du 2-oxo-6-(4-(trifluorométhyl)phényl)-1,2-dihydropyridine-3-carboxylate de méthyle (Protocole SC et PE) | Rendement : 38%. Aspect : solide jaune. |
| | | RMN ¹H :4,02 (s, 3H); 7,35 (d, 1H, J=8,3Hz); 7,75 (d, 2H, J=8,3Hz); 8,17 (d, 2H, J=8,3Hz); 8,31 (d, 1H, J=8,3Hz). |
| **2-3** | 1 : Préparation du 1,2-diphénylprop-2-én-1-one (Protocole SA et PE) | Rendement : 68%. Aspect : solide jaune. |
| | | RMN ¹H :2,72 (s, 6H); 7,09-7,46 (m, 11H). |
| | 2 : Obtention du 2-oxo-5,6-diphényl-1,2-dihydropyridine-3-carbonitrile (Protocole SD et PE) | Rendement : 73%. Aspect : solide blanc. |
| | | RMN ¹H: 7,03-7,06 (m, 2H); 7,19-7,40 (m, 8H); 8,23 (s, 1H); 12,76 (s, 1H). |
| **2-4** | 5-bromo-2-oxo-6-phényl-1,2-dihydropyridine-3-carboxylate de méthyle (Protocole SE et PE) | Rendement : 37%. Aspect : solide blanc. |
| | | RMN ¹H: 3,79 (s, 3H); 7,50-7,57 (m, 5H); 8,23 (s, 1H); 12,49 (s(I), 1H). |
| **2.5** | 1 : Préparation 3-diméthylamino-1-furyl-prop-2-én-1-one (Protocole SA et PA) | Rendement : 69%. Aspect : solide jaune. |
| | | RMN ¹H :2,93 (s, 3H); 3,14 (s, 3H); 5,68 (d, 1H, J=12,6Hz); 6,48 (m, 1H); 7,06 (m, 1H); 7,49 (m, 1H); 7,80 (d, 1H, J=12,6Hz). |
| | 2 : Préparation du 2-carbamoyl-5-diméthylamino-5-furyl-pentan-2,4-dienoate de méthyle (Protocole SB et PC) | Rendement : 24%. Aspect : solide jaune. |
| | 3 : Obtention du 6-furyl-2-oxo-1,2-dihydropyridine-3-carboxylate de méthyle (Protocole SC et PE) | Rendement : 47%. Aspect : solide jaune. |
| | | RMN ¹H :3,98 (s, 3H); 6,59-6,60 (m, 1H); 7,15 (m, 1H); 7,40 (m, 1H); 7,59 (d, 1H, J=1,1Hz); 8,26 (d, 1H, J=7,9Hz). |
| **2-6** | 1 : Préparation du 3-diméthylamino-1-parabiphénylprop-2-én-1-one (Protocole SA et PC) | Rendement : 40%. Aspect : solide jaune. |
| | | RMN ¹H :2,97-3,16 (m, 6H); 5,79 (d, 1H, J=12,3Hz); 7,35-7,40 (m, 1H); 7,46-7,49 (m, 2H); 7,63-7,67 (m, 4H); 7,85 (d, 1H, J=12,3Hz); 7,89 (d, 2H, J=8,2Hz). |
| | 2 : Préparation du 2-carbamoyl-5-diméthylamino-5-(parabiphényl) pentan-2,4-dienoate de méthyle (Protocole SB et PD) | Rendement : 43%. Aspect : solide jaune. |
| | 3 :Obtention du 2-oxo-6-parabiphényl-1,2-dihydropyridine-3-carboxylate de méthyle. (Protocole SC et PE) | Rendement : 59%. Aspect : solide jaune. |
| | | RMN ¹H :4,00 (s, 3H); 7,17-7,24 (m, 1H); 7,38-7,42 (m, 1H); 7,46-7,51 (m, 2H); 7,66 (d, 2H, J=7,3Hz); 7,74 (d, 2H, J=8,5Hz); 8,09 (d, 2H, J=8,5Hz); 8,29 (d, 1H, J=7,9Hz). |
| **2-7** | 1 : Préparation du 3-diméthylamino-1-(3-trifluorométhylphényl)prop-2-én-1-one (Protocole SA et PA) | Rendement : 53%. Aspect : huile marron. |
| | | RMN ¹H :2,88 (s, 3H); 3,10 (s, 3H); 5,64 (d, 1H, J=12,3Hz); 7,48 (m, 1H); 7,64 (d, 1H, J=7,9Hz); 7,80 (d, 1H, J=12,3Hz); 8,03 (d, 1H, J=7,6Hz); 8,11 (s, 1H). |
| | 2 : Préparation du 2-carbamoyl-5-diméthylamino-5-(3-trifluorométhylphényl)-pentan-2,4-dienoate de méthyle (Protocole SB et PA) | Rendement : 23%. Aspect : solide jaune. |
| | 3: Obtention du 2-oxo-6-(4-(trifluorométhyl)phényl)-1,2-dihydropyridine-3-carboxylate de méthyle (Protocole SC et PE) | Rendement : 58%. Aspect : solide beige. |
| | | RMN ¹H :4,02 (s, 3H); 7,36 (d, 1H, J=8,2Hz); 7,62 (t, 1H, J=7,9Hz); 7,73 (d, 1H, J=7,9Hz); 8,25 (d, 1H, J=7,9Hz); 8,30-8,32 (m, 2H). |

### Exemple 3 : Synthèse des intermédiaires de type alkoxy, alkylthio, alkylamino, halo-Pyridine selon l'invention

La synthèse des Intermédiaires de Figure 3a est résumé dans le Tableau 3-1.

**Tableau 3-1**

| **Ex.** | **Intermédiaires (Protocoles)** | **Détails** |
|---|---|---|
| **3-1** | 2-méthoxy-6-phényl-pyridine-3-carboxylate de méthyle (Protocole SF et PA) | Rendement : 73%. Aspect : huile incolore. |
| | | RMN ¹H :3,94 (s, 3H); 4,19 (s, 3H); 7,42-7,54 (m, 4H); 8,11 (dd, 2H, J=1,7Hz J=7,9Hz); 8,27 (d, 1H, J=7,9Hz). |
| **3-2** | 2-tertiobutoxy-6-phényl-pyridine-3-carboxylate de méthyle (Protocole SF et PA) | Rendement : 56%. Aspect : solide blanc. |
| | | RMN ¹H :1,74 (s, 9H); 3,91 (s, 3H); 7,38 (d, 1H, J=7,9 Hz); 7,45-7,52 (m, 3H); 8,06 (m, 2H); 8,19 (d, 1H, J=7,9 Hz). |
| **3-3** | 2-hexyloxy-6-phényl-pyridine-3-carboxylate de méthyle (Protocole SF et PA) | Rendement : 80%. Aspect : huile incolore. |
| | | RMN ¹H :0,93 (t, 3H, J=6,7Hz); 1,34-1,42 (m, 4H); 1,50-1,57 (m, 2H); 1,85-1,94 (m, 2H); 3,93 (s, 3H); 4,58 (t, 2H, J=6,7Hz); 7,41 (d, 1H, J=7,9Hz); 7,43-7,53 (m, 3H); 8,09 (dd, 2H, J=1,9Hz, J=8,1 Hz); 8,26 (d, 1H, J=7,9Hz). |
| **3-4** | 2-cyclohexyloxy-6-phényl-pyridine-3-carboxylate de méthyle (Protocole SF et PA) | Rendement : 12%. Aspect : huile incolore. |
| | | RMN ¹H :1,46-2,09 (m, 10H); 3,92 (s, 3H); 5,41-5,46 (m, 1H); 7,38 (d, 1H, J=7,9Hz); 7,45-7,53 (m, 3H); 8,06 (dd, 2H, J=1,9Hz, J=8,2Hz); 8,24 (d, 1H, J=7,9Hz). |
| **3-7** | 2-méthoxy-6-(4-(trifluorométhyl)phényl)-pyridine-3-carboxylate de méthyle (Protocole SF et PA) | Rendement : 67%. Aspect : solide blanc. |
| | | RMN ¹H :3,94 (s, 3H); 4,18 (s, 3H); 7,46 (d, 1H, J=7,6Hz); 7,75 (d, 2H, J=8,2Hz); 8,21 (d, 2H, J=8,2Hz); 8,29 (d, 1H, J=7,6Hz). |
| **3-9** | 2-méthoxy-pyridine-3-carboxylate de méthyle (Protocole SE et PA) | Rendement : 38%. Aspect : huile incolore. |
| | | RMN ¹H :3,90 (s, 3H); 4,04 (s, 3H); 6,95 (dd, 1H, J=4,8Hz, J=7,5Hz); 8,15 (dd, 1H, J=2,0Hz J=7,5Hz); 8,31 (dd, 1H, J=2,0Hz J=4,8Hz). |
| **3-12** | 5-bromo-2-méthoxy-6-phényl-pyridine-3-carboxylate de méthyle (Protocole SF et PA) | Rendement : 48%. Aspect : solide blanc. |
| | | RMN ¹H :3,94 (s, 3H); 4,07 (s, 3H); 7,45-7,50 (m, 3H); 7,78-7,82 (m, 2H); 8,43 (s, 1H). |
| **3-13** | 2-méthoxy-6-furyl-pyridine-3-carboxylate de méthyle (Protocole SF et PA) | Rendement : 70%. Aspect : solide blanc. |
| | | RMN ¹H :3,91 (s, 3H); 4,11 (s, 3H); 6,55-6,57 (m, 1H); 7,15 (d, 1H, J=3,5Hz); 7,33 (d, 1H, J=7,9Hz); 7,56 (m, 1H); 8,23 (d, 1H, J=7,9Hz). |
| **3-15** | 2-méthoxy-6-(parabiphényl)-pyridine-3-carboxylate de méthyle (Protocole SF et PA) | Aspect : solide blanc. |
| | | RMN ¹H :3,94 (s, 3H); 4,20 (s, 3H); 7,34-7,40 (m, 1H); 7,46-7,51 (m, 3H); 7,67 (d, 2H, J=7,6Hz); 7,73 (d, 2H, J=8,2Hz); 8,19 (d, 2H, J=8,2Hz); 8,28 (d, 1H, J=7,6Hz). |
| **3-16** | 2-méthoxy-6-(3-(trifluorométhyl)phényl)-pyridine-3-carboxylate de méthyle (Protocole SF et PA) | Rendement : 30%. Aspect : solide blanc |
| | | RMN ¹H :3,93 (s, 3H); 4,17 (s, 3H); 7,43 (d, 1H, J=7,7Hz); 7,59 (m, 1H); 7,69 (d, 1H, J=7,9Hz); 8,24-8,26 (m, 1H); 8,27 (d, 1H, J=7,7Hz); 8,33 (s, 1H). |
| **3-24** | 2-éthoxy-6-phényl-pyridine-3-carboxylase de méthyle (Protocole SF et PA) | Rendement : 86%. Aspect : solide blanc. |
| | | RMN ¹H :1,51 (t, 3H, J=7,0Hz); 3,92 (s, 3H); 4,65 (q, 2H, J=7,0Hz); 7,40 (d, 1H, J=7,9Hz); 7,43-7,52 (m, 3H); 8,08 (dd, 2H, J=2,1 Hz J=8,2Hz); 8,24 (d, 1H, J=7,9Hz). |
| **3-25** | 2-isopropoxy-6-phényl-pyridine-3-carboxylate de méthyle (Protocole SF et PA) | Rendement : 74%. Aspect :huile incolore. |
| | | RMN ¹H:1,47 (s, 3H); 1,49 (s, 3H); 3,91 (s, 3H); 5,58-5,66 (m, 1H); 7,37 (d, 1H, J=7,9Hz); 7,42-7,52 (m, 3H); 8,07 (dd, 2H, J=2,0Hz, J=8,2Hz); 8,22 (d, 1H, J=7,9Hz). |

L'introduction de groupements en position 5 et, notamment des groupements de type aryle ou alkyle, peut être réalisée par couplage de type Suzuki entre l'acide boronique précurseur convenablement choisi et la 5-halopyridine adéquate. Les intermédiaires 3-11, 3-17, 3-18, 3-21 à 3-23 ont été préparés à partir des 5-bromopyridines 3-10 et 3-20 La synthèse de ces Intermédiaires (Figure 3b) est résumé dans le Tableau 3-2.

**Tableau 3-2**

| **Ex.** | **Intermédiaire (Protocole)** | **Détails** |
|---|---|---|
| **3-10** | 5-bromo-2-méthoxy-pyridine-3-carboxylate de méthyle (Protocole SE et PE) | Rendement : 33%. Aspect : solide gris. |
| | | RMN ¹H :3,81 (s, 3H); 3,91 (s, 3H); 8,24 (d, 1H, J=2,3Hz); 8,50 (d, 1H, J=2,3Hz). |
| **3-11** | 2-méthoxy-5-phényl-pyridine-3-carboxylate de méthyle (Protocole SI et PA) | Rendement : 71%. Aspect : huile incolore. |
| | | RMN ¹H :3,94 (s, 3H); 4,11 (s, 3H); 7,36-7,42 (m, 1H); 7,45-7,50 (m, 2H); 7,55-7,58 (m, 2H); 8,39 (d, 1H, J=2,6Hz); 8,55 (d, 1H, J=2,6Hz). |
| **3-17** | 2-méthoxy-5-(4-(trifluorométhyl)phényl)-pyridine-3-carboxylate de méthyle (Protocole SI et PA) | Rendement : 21%. Aspect : solide blanc. |
| | | RMN ¹H :3,96 (s, 3H); 4,12 (s, 3H); 7,67 (d, 2H, J=8,3Hz); 7,74 (d, 2H, J=8,3Hz); 8,40 (d, 1H, J=2,6Hz); 8,57 (d, 1H, J=2,6Hz). |
| **3-18** | 2-méthoxy-5-(3-(trifluorométhyl)phényl)-pyridine-3-carboxylate de méthyle (Protocole SI et PA) | Rendement : 30%. Aspect : solide blanc. |
| | | RMN ¹H :3,92 (s, 3H); 4,12 (s, 3H); 7,57-7,67 (m, 2H); 7,73-7,75 (m, 1H); 7,80 (s, 1H); 8,39 (d, 1H, J=2,6Hz); 8,56 (d, 1H, J=2,6Hz). |
| **3-19** | 2,6-diméthoxy-pyridine-3-carboxylate de méthyle (Protocole SQ et PA) | Rendement : 69%. Aspect : solide blanc. |
| | | RMN ¹H :3,86 (s, 3H); 3,98 (s, 3H); 4,05 (s, 3H); 6,33 (d, 1H, J=8,2Hz); 8,14 (d, 1H, J=8,2Hz). |
| **3-20** | 5-bromo-2,6-diméthoxy-pyridine-3-carboxylate de méthyle (Protocole SE et PE) | Rendement : 81%. Aspect : solide blanc. |
| | | RMN ¹H: 3,76 (s, 3H); 3,96 (s, 3H); 4,01 (s, 3H); 8,24 (s, 1H). |
| **3-21** | 5-phényl-2,6-diméthoxy-pyridine-3-carboxylase de méthyle (Protocole SI et PA) | Rendement : 76%. Aspect : solide blanc. |
| | | RMN ¹H :3,88 (s, 3H); 4,03 (s, 3H); 4,11 (s, 3H); 7,31-7,36 (m, 1H); 7,39-7,44 (m, 2H); 7,52-7,55 (m, 2H); 8,26 (s, 1H). |
| **3-22** | 5-(4-chlorophényl)-2-méthoxy-pyridine-3-carboxylate de méthyle (Protocole SI et PA) | Rendement : 97%. Aspect : solide blanc. |
| | | RMN ¹H :3,95 (s, 3H); 4,10 (s, 3H); 7,44 (d, 2H, J=8,8Hz); 7,49 (d, 2H, J=8,8Hz); 8,35 (d, 1H, J=2,4Hz); 8,50 (d, 1H, J=2,4Hz). |
| **3-23** | 5-(naphthalèn-2-yl)-2-méthoxy-pyridine-3-carboxylate de méthyle (Protocole SI et PA) | Rendement : 84%. Aspect : solide blanc. |
| | | RMN ¹H :3,97 (s, 3H); 4,13 (s, 3H); 7,49-7,57 (m, 2H); 7,70 (dd, 1H, J=2,0Hz J=8,5Hz); 7,87-7,97 (m, 3H); 8,02 (s, 1H); 8,52 (d, 1H, J=2,5Hz); 8,68 (d, 1H, J=2,5Hz). |

Des intermédiaires substitués en position 2 par des groupements alkylthio, alkylamino sont accessibles au départ des 2-halo-pyridine correspondantes les intermédiaires 3-6, 3-8 et 3-14 ont été préparés à partir de la 2-chloropyridine 3-5 (Figure 3c), comme il est résumé dans le Tableau 3-3.

**Tableau 3-3**

| **Ex.** | **Intermédiaire (protocoles)** | **Détails** |
|---|---|---|
| **3-5** | 2-chloro-6-phényl-pyridine-3-carboxylate de méthyle (Protocole SG et PA) | Rendement : 83%. Aspect : solide blanc. |
| | | RMN ¹H :3,99 (s, 3H); 7,48-7,53 (m, 3H); 7,75 (d, 1H, J=8,1 Hz); 8,05-8,08 (m, 2H); 8,26 (d, 1H, J=8,1 Hz). |
| **3-6** | 2-(piperidin-1-yl)-6-phényl-pyridine-3-carboxylate de méthyle (l'intermédiaire 3-5 est solubilisé dans de la pipéridine (0,1 mol/L) et l'ensemble est maintenu sous agitation à reflux pendant 16 heures ; Protocole PA) | Rendement : 95%. Aspect : huile jaune. |
| | | RMN ¹H :1,60-1,73 (m, 6H); 3,47-3,49 (m, 4H); 3,91 (s, 3H); 7,18 (d, 1H, J=7,9Hz); 7,41-7,49 (m, 3H); 8,05 (d, 1H, J=7,9Hz); 8,06 (dd, 2H, J=1,6Hz, J=8,2Hz). |
| **3-8** | 2-phénylthio-6-phényl-pyridine-3-carboxylate de méthyle ( Protocole SH et PA) | Rendement : quantitatif. Aspect : huile incolore. |
| | | RMN ¹H :4,01 (s, 3H); 7,25-7,35 (m, 3H); 7,46-7,53 (m, 4H); 7,60-7,66 (m, 4H); 8,28 (d, 1H, J=8,2Hz). |
| **3-14** | 2-éthylthio-6-phényl-pyridine-3-carboxylate de méthyle ( Protocole SH et PA) | Rendement : 90%. Aspect : solide beige. |
| | | RMN ¹H :1,47 (t, 3H, J=7,3Hz); 3,34 (q, 2H, J=7,3Hz); 3,95 (s, 3H); 7,47-7,54 (m, 4H); 8,11-8,14 (m, 2H); 8,28 (d, 1H, J=8,2Hz). |

### Exemple 4 : Synthèse des intermédiaires de type 3-hydroxyméthyl-, 3-halométhyl-et 3-arylsultonylméthyl-pyridine selon l'invention

La synthèse des Intermédiaires de Figure 4a est résumé dans le Tableau 4-1.

**Tableau 4-1**

| **Ex.** | **Intermédiaire (Protocole)** | **Détails** |
|---|---|---|
| **4-1** | (2-méthoxy-6-phénylpyridin-3-yl)méthanol (Protocole SJ et PA) | Rendement : 96%. Aspect : solide jaune. |
| | | RMN ¹H :4,12 (s, 3H); 4,71 (s, 2H); 7,36-7,50 (m, 4H); 7,64 (d, 1H, J=7,3Hz); 8,06 (m, 2H). |
| **4-2** | (2-tertiobutoxy-6-phénylpyridin-3-yl)méthanol (Protocole SJ et PA) | Rendement: 91%. Aspect : solide blanc. RMN ¹H :1,73 (s, 9H); 4,64 (s, 2H); 7,33 (d, 1H, J=7,5Hz); 7,37-7,50 (m, 3H); 7,58 (d, 1H, J=7,5Hz); 8,02 (m, 2H). |
| **4-3** | (2-hexyloxy-6-phénylpyridin-3-yl)méthanol (Protocole SJ) | Rendement : quantitatif. Aspect : solide blanc. |
| | | RMN ¹H :0,92 (t, 3H, J=6,7Hz); 1,27-1,53 (m, 6H); 1,80-1,90 (m, 2H); 4,52 (t, 2H, J=6,7Hz); 4,70 (d, 2H, J=6,2Hz); 7,34 (d, 1H, J=7,3Hz); 7,37-7,49 (m, 3H); 7,62 (d, 1H, J=7,6Hz); 8,02-8,05 (m, 2H). |
| **4-4** | (2-cyclohexyloxy-6-phénylpyridin-3-yl)méthanol (Protocole SJ) | Rendement : 88%. Aspect : huile transparente. |
| | | RMN ¹H :1,37-1,69 (m, 6H); 1,79-1,83 (m, 2H); 2,05 (m, 2H); 4,68 (d, 2H, J=6,4Hz); 5,32-5,38 (m, 1H); 7,32 (d, 1H, J=7,3Hz); 7,36-7,49 (m, 3H); 7,60 (d, 1H, J=7,6Hz); 8,01 (m, 2H). |
| **4-5** | (6-phényl-2-(pipéridin-1-yl)pyridin-3-yl)méthanol (Protocole SJ et PA) | Rendement : 46%. Aspect : solide blanc. |
| | | RMN ¹H :1,62-1,83 (m, 6H); 3,18-3,22 (t, 4H, J=5,4Hz); 4,80 (s, 2H); 4,94 (s, 1H); 7,36-7,49 (m, 4H); 7,57 (d, 1H, J=7,9Hz); 8,04 (m, 2H). |
| **4-6** | (2-méthoxy-6-(4-(trifluoro méthyl)phényl)pyridin-3-yl) méthanol (Protocole SJ et PA) | Rendement : 93%. Aspect : solide blanc. |
| | | RMN ¹H :4,12 (s, 3H); 4,72 (s, 2H); 7,41 (d, 1H, J=7,6Hz); 7,68-7,73 (m, 3H); 8,16 (d, 2H, J=8,2Hz). |
| **4-7** | (6-phényl-2-(phénylthio)pyridin-3-yl) méthanol (Protocole SJ et PA) | Rendement : 39%. Aspect : solide blanc. |
| | | RMN ¹H :4,84 (s, 2H); 7,31-7,36 (m, 3H); 7,38-7,46 (m, 3H); 7,55-7,63 (m, 3H); 7,73-7,78 (m, 3H). |
| **4-8** | (2-méthoxy-5-phénylpyridin-3-yl)méthanol (Protocole SJ) | Rendement : quantitatif. Aspect : solide blanc. |
| | | RMN ¹H :4,05 (s, 3H); 4,73 (s, 2H); 7,34-7,39 (m, 1H); 7,43-7,47 (m, 2H); 7,53-7,56 (m, 2H); 7,83 (d, 1H, J=2,3Hz); 8,32 (d, 1H, J=2,3Hz). |
| **4-9** | (5-bromo-2-méthoxy-6-phénylpyridin-3-yl)méthanol (Protocole SJ et PA) | Rendement : 50%. Aspect : solide blanc. |
| | | RMN ¹H :4,00 (s, 3H); 4,68 (s, 2H); 7,42-7,50 (m, 3H); 7,74-7,77 (m, 2H); 7,86 (s, 1H). |
| **4-10** | (2-méthoxy-6-furylpyridin-3-yl)méthanol (Protocole SJ et PA) | Rendement : 78%. Aspect : solide blanc. |
| | | RMN ¹H :2,25 (t, 1H, J=6,3Hz); 4,05 (s, 3H); 4,66 (d, 2H, J=6,3Hz); 6,51-6,53 (m, 1H); 7,01-7,02 (m, 1H); 7,29 (d, 1H, J=7,6Hz); 7,5-7,51 (m, 1H); 7,60 (d, 1H, J=7,6Hz). |
| **4-12** | (2-(éthylthio)-6-phénylpyridin-3-yl)méthanol (Protocole SJ et PA) | Rendement : 75%. Aspect : huile jaune. |
| | | RMN ¹H :1,47 (t, 3H, J=7,3Hz); 3,39 (q, 2H, J=7,3Hz); 4,72 (s, 2H); 7,39-7,51 (m, 4H); 7,67 (d, 1H, J=7,9Hz); 8,07 (d, 2H, J=7,0Hz). |
| **4-13** | (2-méthoxy-6 (parabiphényl) pyridin-3-yl)méthanol (Protocole SJ et PA) | Rendement : 59%. Aspect : solide blanc. RMN ¹H :2,29 (t, 1H, J=6,6Hz); 4,14 (s, 3H); 4,71 (d, 2H, J=6,6Hz); 7,36-7,50 (m, 4H); 7,65-7,72 (m, 5H); 8,14 (d, 2H, J=6,7Hz). |
| **4-14** | (2-méthoxy-6-(3-(trifluorométhyl)phényl)pyridin-3-yl)méthanol (Protocole SJ et PA) | Rendement : 85%. Aspect : solide blanc. |
| | | RMN ¹H :2,26 (t, 1H, J=6,6Hz); 4,12 (s, 3H); 4,72 (d, 2H, J=6,6Hz); 7,41 (d, 1H, J=7,3Hz); 7,56-7,70 (m, 3H); 8,22 (d, 1H, J=7,9Hz); 8,31 (s, 1H). |
| **4-17** | (2-méthoxy-5-(4-(trifluorométhyl)phényl)pyridin-3-yl)méthanol (Protocole SJ) | Rendement : quantitatif. Aspect : solide blanc. |
| | | RMN ¹H :4,02 (s, 3H); 4,72 (d, 2H, J=5,3Hz); 7,60 (d, 2H, J=8,3Hz); 7,67 (d, 2H, J=8,3Hz); 7,86 (d, 1H, J=2,5Hz); 8,30 (d, 1H, J=2,5Hz). |
| **4-18** | (2-méthoxy-5-(3-(trifluorométhyl)phényl)pyridin-3-yl)méthanol (Protocole SJ) | Rendement : 77%. Aspect : solide blanc. |
| | | RMN ¹H :4,05 (s, 3H); 4,74 (s, 2H); 7,54-7,63 (m, 2H); 7,71-7,73 (m, 1H); 7,78 (s, 1H); 7,85 (d, 1H, J=2,5Hz); 8,33 (d, 1H, J=2,5Hz). |
| **4-19** | (2,6-diméthoxy-5-phénylpyridin-3-yl)méthanol (Protocole SJ et PA) | Rendement : 77%. Aspect : solide blanc. |
| | | RMN ¹H :3,98 (s, 3H); 4,04 (s, 3H); 4,65 (s, 2H); 7,30-7,33 (m, 1H); 7,38-7,43 (m, 2H); 7,52-7,57 (m, 3H). |
| **4-20** | (5-(4-chlorophényl)-2-méthoxypyridin-3-yl)méthanol (Protocole SJ) | Rendement: 84%. Aspect : huile incolore. |
| | | RMN ¹H :4,04 (s, 3H); 4,72 (d, 2H, J=6,5Hz); 7,42 (d, 2H, J=8,8Hz); 7,47 (d, 2H, J=8,8Hz); 7,79 (d, 1H, J=2,3Hz); 8,28 (d, 1H, J=2,3Hz). |
| **4-21** | (2-méthoxy-5-(naphthalen-2-yl)pyridin-3-yl)méthanol (Protocole SJ et PA) | Rendement : 75%. Aspect : solide blanc. |
| | | RMN ¹H :4,07 (s, 3H); 4,77 (s, 2H); 7,49-7,53 (m, 2H); 7,69 (dd, 1H, J=1,9Hz, J=8,5Hz); 7,86-7,99 (m, 5H); 8,45 (d, 1H, J=2,5Hz). |
| **4-22** | (2-éthoxy-6-phénylpyridin-3-yl)méthanol (Protocole SJ) | Rendement : 91%. Aspect : solide blanc. |
| | | RMN ¹H :1,47 (t, 3H, J=7,2Hz); 4,59 (q, 2H, J=7,2Hz); 4,69 (d, 2H, J=5,6Hz); 7,34 (d, 1H, J=7,6Hz); 7,37-7,49 (m, 3H); 7,62 (d, 1H, J=7,6Hz); 8,01-8,05 (m, 2H). |
| **4-23** | (2-isopropoxy-6-phénylpyridin-3-yl)méthanol (Protocole SJ) | Rendement : quantitatif. Aspect : huile incolore. RMN ¹H :1,43 (s, 3H); 1,45 (s, 3H); 4,67 (d, 2H, J=6,4Hz); 5,56-5,64 (m, 1H); 7,32 (d, 1H, J=7,5Hz); 7,36-7,49 (m, 3H); 7,60 (d, 1H, J=7,5Hz); 8,00-8,04 (m, 2H). |

La synthèse des intermédiaires des Figures 4b, 4c et 4d est résumé dans le Tableau 4-2

**Tableau 4-2**

| **Ex.** | **Intermédiaire (Protocole)** | **Détails** |
|---|---|---|
| **4-11** | 1-(2-méthoxy-6-phénylpyridin-3-yl)propan-1-ol (Protocole SK et PA) | Rendement : 66%. Aspect : solide beige |
| | | RMN ¹H :0,99 (t, 3H, J=7,3Hz); 1,86 (m, 2H); 4,10 (s, 3H); 4,77 (t, 1H, J=6,4Hz); 7,37-7,50 (m, 4H); 7,65 (d, 1H, J=7,6Hz); 8,06 (d, 2H, J=7,0Hz). |
| **4-15** | (2-méthoxy-6-phénylpyridin-3-yl)méthyl-4-méthylbenzènesulfonate (Protocole SL et PA) | Rendement : 31%. Aspect : huile jaune. |
| | | RMN ¹H :2,51 (s, 3H); 4,13 (s, 3H); 4,67 (s, 2H); 7,37-7,51 (m, 6H); 7,72 (d, 1H, J=7,6Hz); 7,95 (d, 2H, J=8,5Hz); 8,07 (m, 2H). |
| **4-16** | 3-(bromométhyl)-2-méthoxy-6-phénylpyridine (Protocole SM) | Rendement : 91%. Aspect : solide blanc. |
| | | RMN ¹H :4,14 (s, 3H); 4,57 (s, 2H); 7,36 (d, 1H, J=7,6Hz); 7,42-7,53 (m, 3H); 7,68 (d, 1H, J=7,6Hz); 8,06 (m, 2H). |

### Exemple 5 : Synthèse des intermédiaires de type Pyridine 3-carboxaldéhyde et cétone selon l'invention

La synthèse des Intermédiaires de Figures 5a est résumé dans le Tableau 5-1.

**Tableau 5-1**

| **Ex.** | **Intermédiaires (Protocoles)** | **Détails** |
|---|---|---|
| **5-1** | 2-méthoxy-6-phényl-pyridine-3-carboxaldéhyde (Protocole SN et PA) | Rendement : 63%. Aspect : solide blanc. |
| | | RMN ¹H :4,20 (s, 3H); 7,50 (m, 4H); 8,11 (m, 2H); 8,19 (d, 1H, J=7,9Hz); 10,40 (s, 1H). |
| **5-3** | 2-méthoxy-6-(4-(trifluorométhyl)phényl)-pyridine-3-carboxaldéhyde (Protocole SN et PA) | Rendement : 94%. Aspect : solide blanc. |
| | | RMN ¹H :4,16 (s, 3H); 7,48 (d, 1H, J=7,9Hz); 7,72 (d, 2H, J=8,2Hz); 8,16-8,19 (m, 3H); 10,38 (s, 1H). |
| **5-4** | 2-méthoxy-5-phényl-pyridine-3-carboxaldéhyde (Protocole SN et PA) | Rendement : 63%. Aspect : solide blanc. |
| | | RMN ¹H :4,14 (s, 3H); 7,38-7,43 (m, 1H); 7,46-7,51 (m, 2H); 7,55-7,59 (m, 2H); 8,34 (d, 1H, J=2,6Hz); 8,63 (d, 1H, J=2,6Hz); 10,44 (s, 1H). |
| **5-6** | 5-bromo-2-méthoxy-6-phénylpyridine-3-carboxaldéhyde (Protocole SN et PA) | Rendement : 63%. Aspect : solide blanc. |
| | | RMN ¹H :4,10 (s, 3H); 7,48-7,51 (m, 3H); 7,77-7,82 (m, 2H); 8,35 (s, 1H); 10,33 (s, 1H). |
| **5-7** | 2-méthoxy-6-furyl-pyridine-3-carboxaldéhyde (Protocole SN et PA) | Rendement : 86%. Aspect : solide jaune. |
| | | RMN ¹H: 4,07 (s, 3H); 6,73-6,75 (m, 1H); 7,34 (d, 1H, J=3,2Hz); 7,48 (d, 1H, J=7,9Hz); 7,97 (m, 1H); 8,14 (d, 1H, J=7,9Hz); 10,21 (s, 1H). |
| **5-8** | 1-(2-méthoxy-6-phénylpyridin-3-yl)propan-1-one (Protocole SN et PA) | Rendement : 92%. Aspect : solide blanc. |
| | | RMN ¹H :1,21 (t, 3H, J=7,2Hz); 3,09 (q, 2H, J=7,2Hz); 4,17 (s, 3H); 7,45-7,52 (m, 4H); 8,10 (d, 2H, J=7,9Hz); 8,22 (d, 1H, J=7,9Hz). |
| **5-9** | 2-éthylthio-6-phényl-pyridine-3-carboxaldéhyde (Protocole SN et PA) | Rendement : 86%. Aspect : huile jaune. |
| | | RMN ¹H :1,49 (t, 3H, J=7,3Hz); 3,42 (q, 2H, J=7,3Hz); 7,51-7,55 (m, 3H); 7,62 (d, 1H, J=7,9Hz); 8,08 (d, 1H, J=7,9Hz); 8,12-8,15 (m, 2H); 10,27 (s, 1H). |
| **5-10** | 2-méthoxy-6-(parabiphényl)-pyridine-3-carboxaldéhyde (Protocole SN et PA) | Rendement : 79%. Aspect : solide blanc. |
| | | RMN ¹H :4,22 (s, 3H); 7,41-7,43 (m, 1H); 7,49-,756 (m, 3H); 7,66-7,69 (m, 2H); 7,73-7,76 (m, 2H); 8,20-8,22 (m, 3H); 10,41 (s, 1H). |
| **5-11** | 2-méthoxy-6-(3-(trifluorométhyl)phényl)-pyridine-3-carboxaldéhyde (Protocole SN et PA) | Rendement : 68%. Aspect : solide blanc |
| | | RMN ¹H :4,21 (s, 3H); 7,53 (d, 1H, J=7,9Hz); 7,64 (t, 1H, J=7,9Hz); 7,73 (d, 1H, J=7,9Hz); 8,23 (d, 1H, J=7,9Hz); 8,29 (d, 1H, J=7,9Hz); 8,37 (s, 1H); 10,41 (s, 1H). |
| **5-12** | 2-méthoxy-5-(4-(trifluorométhyl)phényl)-pyridine-3-carboxaldéhyde (Protocole SN et PA) | Rendement : 70%. Aspect : solide blanc |
| | | RMN ¹H :4,15 (s, 3H); 7,68 (d, 2H, J=8,2Hz); 7,73 (d, 2H, J=8,2Hz); 8,35 (d, 1H, J=2,7Hz); 8,65 (d, 1H, J=2,7Hz); 10,44 (s, 1H). |
| **5-13** | 2-méthoxy-5-(3-(trifluorométhyl)phényl)-pyridine-3-carboxaldéhyde (Protocole SN et PA) | Rendement : 58%. Aspect : solide blanc. |
| | | RMN ¹H :4,15 (s, 3H); 7,55-7,68 (m, 2H); 7,74 (d, 1H, J=7,3Hz); 7,81 (s, 1H); 8,34 (d, 1H, J=2,6Hz); 8,64 (d, 1H, J=2,6Hz); 10,45 (s, 1H). |
| **5-14** | 2,6-diméthoxy-5-phényl-pyridine-3-carboxaldéhyde (Protocole SN et PA) | Rendement : 93%. Aspect : solide blanc. |
| | | RMN ¹H :4,07 (s, 3H); 4,12 (s, 3H); 7,32-7,45 (m, 3H); 7,50-7,54 (m, 2H); 8,13 (s, 1H); 10,27 (s, 1H). |
| **5-15** | 2-méthoxy-5-(4-chlorophényl)-pyridine-3-carboxaldéhyde (Protocole SN et PA) | Rendement : 58%. Aspect : solide blanc. |
| | | RMN ¹H :4,14 (s, 3H); 7,45 (d, 2H, J=8,8Hz); 7,49 (d, 2H, J=8,8Hz); 8,30 (d, 1H, J=2,6Hz); 8,59 (d, 1H, J=2,6Hz); 10,43 (s, 1H). |
| **5-16** | 2-méthoxy-5-(naphthalen-2-yl)-pyridine-3-carboxaldéhyde (Protocole SN et PA) | Rendement : 75%. Aspect : solide blanc. |
| | | RMN ¹H :4,16 (s, 3H); 7,50-7,57 (m, 2H); 7,70 (dd, 1H, J=1,9Hz J=8,5Hz); 7,87-7,97 (m, 3H); 8,03 (d, 1H, J=1,5Hz); 8,46 (d, 1H, J=2,6Hz); 8,76 (d, 1H, J=2,6Hz); 10,47 (s, 1H). |
| **5-17** | 2-éthoxy-6-phényl-pyridine-3-carboxaldéhyde (Protocole SN et PA) | Rendement : 87%. Aspect : solide blanc. |
| | | RMN ¹H :1,52 (t, 3H, J=7,0Hz); 4,67 (q, 2H, J=7,0Hz); 7,44-7,54 (m, 4H); 8,07-8,11 (m, 2H); 8,18 (d, 1H, J=7,9Hz); 10,42 (s, 1H). |
| **5-18** | 2-isopropoxy-6-phényl-pyridine-3-carboxaldéhyde (Protocole SN et PA) | Rendement : 77%. Aspect : huile incolore. |
| | | RMN ¹H :1,48 (s, 3H); 1,50 (s, 3H); 5,64-5,72 (m, 1H); 7,43-7,53 (m, 4H); 8,07-8,10 (m, 2H); 8,17 (d, 1H, J=7,9Hz); 10,40 (s, 1H). |

La synthèse des Intermédiaires des Figures 5b et 5c est résumé dans le Tableau 5-2.

**Tableau 5-2**

| **Ex.** | **No. et type d'Etape (Protocole)** | **Détails** |
|---|---|---|
| **5-2** | 2-tertiobutoxy-6-phényl-pyridine-3-carboxaldéhyde (Protocole SO et PA) | Rendement : 83%. Aspect : solide blanc. |
| | | RMN ¹H :1,76 (s, 9H); 7,42-7,53 (m, 4H); 8,07 (m, 2H); 8,16 (d, 1H, J=8,2Hz); 10,36 (s, 1H). |
| **5-5** | Etape 1 : Préparation du 5,6-diphényl-2-oxo-pyridine-3-carboxaldéhyde (Protocole SP et PA) | Rendement : 59%. Aspect : solide jaune. |
| | | RMN ¹H :7,07-7,10 (m, 2H); 7,25-7,43 (m, 8H); 8,23 (s, 1H); 10,27 (s, 1H). |
| | Etape 2 : Obtention du 2-méthoxy-5,6-diphényl-pyridine-3-carboxaldéhyde (Protocole SF et PA) | Rendement : 16%. Aspect : solide blanc. |
| | | RMN ¹H :4,18 (s, 3H); 7,14-7,20 (m, 2H); 7,26-7,34 (m, 6H); 7,44-7,47 (m, 2H); 8,16 (s, 1H); 10,44 (s, 1H). |

### Exemple 6 : Synthèse des intermédiaires de type phénol, thiophénol, aniline

La synthèse des Intermédiaires de Figure 6a, 6c, et 6e nécessite 2 étapes et elle est résumé dans le Tableau 6-1.

**Tableau 6-1**

| **Ex.** | **No. et type d'Etape (Protocole)** | **Détails** |
|---|---|---|
| **6-1** | Etape 1 : Préparation du 2-(4-(benzyloxy)phénoxy)-2-méthylpropanoate d'éthyle (Protocole SQ et PA) | Rendement : 70%. Aspect : huile incolor. |
| | | RMN ¹H:1,3 (t, 3H, J=7,3Hz); 1,56 (s, 6H); 4,26 (q, 2H, J=7,3Hz); 5,02 (s, 2H); 6,87 (s, 4H); 7,32-7,36 (m, 5H). |
| | Etape 2 : Obtention du 2-(4-hydroxyphénoxy)-2-méthylpropanoate d'éthyle (Protocole SR) | Rendement : 94%. Aspect : solide rose. |
| | | RMN ¹H :1,29 (t, 3H, J=7,0Hz); 1,53 (s, 6H); 4,26 (q, 2H, J=7,0Hz); 6,70 (d, 2H, J=9,0Hz); 6,80 (d, 2H, J=9,0Hz). |
| **6-2** | Etape 1 : Préparation du 2-(4-(benzyloxy)phénoxy)éthanoate de tertiobutyle (Protocole SQ) | Rendement : quantitatif. Aspect : huile incolore. |
| | | RMN ¹H :1,5 (s, 9H); 4,48 (s, 2H); 5,03 (s, 2H); 6,84-6,94 (m, 4H); 7,33-7,46 (m, 5H). |
| | Etape 2 : Obtention du 2-(4-hydroxyphénoxy)éthanoate de tertiobutyle (Protocole SR et PD) | Rendements: 97%. Aspect : solide blanc. RMN ¹H :1,48 (s, 9H); 4,47 (s, 2H); 6,75-6,83 (m, 4H). |
| **6-3** | Etape 1 : Préparation du 2-(4-(benzyloxy)phénoxy)propanoate d'éthyle (Protocole SQ et PA) | Rendement : 92%. Aspect : huile incolore. |
| | | RMN ¹H :1,28 (t, 3H, J=7,2Hz); 1,63 (d, 3H, J=6,9Hz); 4,24 (q, 2H, J=7,2Hz); 4,68 (q, 1H, J=6,9Hz); 5,03 (s, 2H); 6,85-6,94 (m, 4H); 7,32-7,46 (m, 5H). |
| | Etape 2 : Obtention du 2-(4-hydroxyphénoxy)propanoate d'éthyle (Protocole SR et PA) | Rendement : 86%. Aspect : huile jaune. |
| | | RMN ¹H :1,26 (t, 3H, J=7,0Hz); 1,59 (d, 3H, J=6,7Hz); 4,23 (q, 2H, J=7,3Hz); 4,67 (q, 1H, J=6,7Hz); 6,71-6,78 (m, 4H). |
| **6-4** | Etape 1 : Préparation du 2-(4-(benzyloxy)phénoxy)éthanoate d'éthyle (Protocole SQ et PA) | Rendement : 80%. Aspect : solide blanc. |
| | | RMN ¹H :1,32 (t, 3H, J=7,0Hz); 4,28 (q, 2H, J=7,0Hz); 4,59 (s, 2H); 5,03 (s, 2H); 6,86-6,95 (m, 4H); 7,28-7,50 (m, 5H). |
| | Etape 2 : Obtention du 2-(4-hydroxyphénoxy) éthanoate d'éthyle (Protocole SR) | Rendement : 95%. Aspect : solide blanc. |
| | | RMN ¹H :1,31 (t, 3H, J=7,2Hz); 4,28 (q, 2H, J=7,2Hz); 4,57 (s, 2H); 5,03 (s, 1H); 6,74-6,83 (m, 4H). |
| **6-5** | Etape 1 : Préparation du 2-(4-(benzyloxy)phénoxy)2-méthylpropanoate de tertiobutyle (Protocole SQ et PA) | Rendement : 71%. Aspect : solide blanc. |
| | | RMN ¹H :1,47 (s, 9H); 1,53 (s, 6H); 5,02 (s, 2H); 6,87 (s, 4H); 7,33-7,46 (m, 5H). |
| | Etape 2 : Obtention du 2-(4-hydroxyphénoxy)-2-méthylpropanoate de tertiobutyle (Protocole SR et PA) | Rendement : 64%. Aspect : solide blanc. |
| | | RMN ¹H :1,48 (s, 9H); 1,52 (s, 6H); 3,80 (s, 1H); 6,69-6,73 (m, 2H); 6,78-6,83 (m, 2H). |
| **6-6** | Etape 1 : Préparation du 2-(4-nitrophénoxy)éthanoate de tertiobutyle (Protocole SQ et PD) | Rendement : 60%. Aspect : solide ocre. |
| | | RMN ¹H :1,5 (s, 9H); 4,63 (s, 2H); 6,96 (d, 2H, J=9,3Hz); 8,22 (d, 2H, J=9,3Hz). |
| | Etape 2 : Obtention du 2-(4-aminophénoxy)éthanoate de tertiobutyle (Protocole SR) | Rendement : quantitatif. Aspect : solide rose/rouge RMN ¹H :1,49 (s, 9H); 3,35 (s (large), 2H); 4,44 (s, 2H); 6,57 (d, 2H, J=8,8Hz); 6,76 (d, 2H, J=8,8Hz). |
| **6-7** | Etape 1 : Préparation du 2-(4-(tertiobutylcarbonylamino)phénox y)propanoate d'éthyle (Protocole SQ et PD) | Rendement : 80%. Aspect : solide blanc. |
| | | RMN ¹H : 1,26 (t, 3H, J=7,3Hz); 1,52 (s, 9H); 1,62 (d, 3H, J=6,8Hz); 4,21 (q, 2H, J=7,3Hz); 4,70 (q, 1H, J=6,8Hz); 6,37 (s (large), 1H); 6,83 (d, 2H, J=9,0Hz); 7,26 (d, 2H, J=9,0Hz). |
| | Etape 2 : Obtention du chlorhydrate de 2-(4-aminophénoxy)propanoate d'éthyle (Protocole SS et PE) | Rendement : 99%. Aspect : solide blanc. |
| | | RMN ¹H : 1,17 (t, 3H, J=7,2Hz); 1,15 (d, 3H, J=6,7Hz); 4,13 (q, 2H, J=7,2Hz); 4,99 (q, 1H, J=6,7Hz); 6,99 (d, 2H, J=8,8Hz); 7,31 (d, 2H, J=8,8Hz); 10,28 (s (large), 3H). |
| **6-8** | Etape 1 : Préparation du 2-(4-(tertiobutylcarbonylamino)phénox y)éthanoate d'éthyle (Protocole SQ) | Rendement : quantitatif. Aspect : solide blanc. |
| | | RMN ¹H :1,29 (t, 3H, J=7,0Hz); 1,51 (s, 9H); 4,27 (q, 2H, J=7,0Hz); 4,59 (s, 2H); 6,44 (s (large), 1H); 6,85 (d, 2H, J=8,8Hz); 7,28 (d, 2H, J=8,8Hz). |
| | Etape 2 : Obtention du chlorhydrate de 2-(4-aminophénoxy)éthanoate d'éthyle (Protocole SS et PE) | Rendement : 86%. Aspect : solide rose |
| | | RMN ¹H : 1,20 (t, 3H, J=7,0Hz); 4,16 (q, 2H, J=7,0Hz); 4,81 (s, 2H); 7,03 (d, 2H, J=8,8Hz); 7,33 (d, 2H, J=8,8Hz); 10,26 (s (large), 3H). |
| **6-9** | Etape 1 : Préparation du 2-(4-(tertiobutylcarbonylamino)phénox y)-2-méthyl-propanoate d'éthyle (Protocole SQ et PD) | Rendement : 95%. Aspect : huile incolore. |
| | | RMN ¹H :1,28 (t, 3H, J=7,0Hz); 1,52 (s, 9H); 1,56 (s, 6H); 4,23 (q, 2H, J=7,0Hz); 6,39 (s (large), 1H); 6,83 (d, 2H, J=9,0Hz); 7,24 (d, 2H, J=9,0Hz). |
| | Etape 2 : Obtention du chlorhydrate de 2-(4-aminophénoxy)-2-méthylpropanoate d'éthyle (Protocole SS et PE) | Rendement : 89%. Aspect : solide blanc. |
| | | RMN ¹H : 1,17 (t, 3H, J=7,0Hz); 1,53 (s, 6H); 4,15 (q, 2H, J=7,0Hz); 6,88 (d, 2H, J=8,8Hz); 7,29 (d, 2H, J=8,8Hz); 10,24 (s (large), 3H). |
| **6-11** | Etape 1 : Préparation du 2-(4-nitrophénoxy)-2-méthylpropanoate de tertiobutyle (Protocole SQ et PA) | Rendement : 65%. Aspect : solide jaune |
| | | RMN ¹H :1,43 (s, 9H); 1,65 (s, 6H); 6,86 (d, 2H, J=7,3Hz); 8,22 (d, 2H, J=7,3Hz). |
| | Etape 2 : Obtention du 2-(4-aminophénoxy)-2-méthylpropanoate de tertiobutyle (Protocole SR et PD) | Rendement : 91%. Aspect : solide marron. |
| | | RMN ¹H :1,46 (s, 9H); 1,5 (s, 6H); 4,33 (s (large), 2H); 6,69 (d, 2H, J=8,8Hz); 6,78 (d, 2H, J=8,8Hz). |
| **6-12** | Etape 1 : Préparation du 2-(3-nitrophénoxy)-2-méthylpropanoate de tertiobutyle (Protocole SQ et PA) | Rendement : 11%. Aspect : solide vert. |
| | | RMN ¹H :1,47 (s, 9H); 1,62 (s, 6H); 7,17-7,20 (m, 1H); 7,40 (t, 1H, J=8,2Hz); 7,68 (t, 1H, J=2,3Hz); 7,83-7,86 (m, 1H). |
| | Etape 2 : Obtention du 2-(3-aminophénoxy)-2-méthylpropanoate de tertiobutyle (Protocole SR et PD) | Rendement : 37%. Aspect : solide vert. |
| | | RMN ¹H :1,46 (s, 9H); 1,5 (s, 6H); 4,33 (s (large), 2H); 6,69 (d, 2H, J=8,8Hz); 6,78 (d, 2H, J=8,8Hz). |
| **6-13** | Etape 1 : Préparation du 2-(3-nitrophénoxy)éthanoate de tertiobutyle (Protocole SQ et PC) | Rendement : 96%. Aspect : solide rouge-orangé. |
| | | RMN ¹H :1,51 (s, 9H); 4,62 (s, 2H); 7,27 (dd, 1H, J=2,OHz, J=8,2Hz); 7,46 (t, 1H, J=8,2Hz); 7,71 (t, 1H, J=2,0Hz); 7,88 (d, 1H, J=8,2Hz). |
| | Etape 2 : Obtention du 2-(3-aminophénoxy)éthanoate de tertiobutyle (Protocole SR et PA) | Rendement : 72%. Aspect : huile rose/rouge. |
| | | RMN ¹H :1,49 (s, 9H); 4,48 (s, 2H); 5,35 (s (large), 2H); 6,48 (m, 3H); 7,12 (d, 2H, J=7,9Hz). |
| **6-20** | Etape 1 : Préparation du 2-(4-nitro-2,6-diméthylphénoxy)éthanoate d'éthyle (Protocole SQ et PD) | Rendement : 99%. Aspect : solide jaune. |
| | | RMN ¹H :1,34 (t, 3H, J=7,0Hz); 2,40 (s, 6H); 4,31 (q, 2H, J=7,0Hz) 4,47 (s, 2H); 7,93 (s, 2H). |
| | Etape 2 : Obtention du 2-(4-amino-2,6-diméthylphénoxy)éthanoate d'éthyle (Protocole SR) | Rendement : quantitatif. Aspect : huile incolore. |
| | | RMN ¹H :1,33 (t, 3H, J=7,0Hz); 2,22 (s, 6H); 4,30 (q, 2H, J=7,0Hz); 4,33 (s, 2H); 6,34 (s, 2H). |
| **6-21** | Etape 1 : Préparation du 3-(4-nitrophényl)-3-phénylacrylate d'éthyle (Protocole ST et PD) | Rendement : 57%. Aspect : huile jaune. |
| | | RMN ¹H :1,17 (t, 3H, J=7,3Hz); 4,08 (q, 2H, J=7,3Hz); 6,49 (s, 1H); 7,33-7,43 (m, 2H); 7,54 (t, 2H, J=7,9Hz); 7,64-7,69 (m, 1H); 7,95 (d, 2H, J=8,8Hz); 8,27 (d, 2H, J=8,8Hz). |
| | Etape 2 : Obtention du 3-(4-aminophényl)-3-phénylpropanoate d'éthyle (Protocole SR) | Rendement : 53%. Aspect : huile incolore. |
| | | RMN ¹H :1,12 (t, 3H, J=7,0Hz); 2,99 (d, 2H, J=8,2Hz); 7,03 (q, 2H, J=7,3Hz); 4,45 (t, 1H, J=7,9Hz); 6,61 (d, 2H, J=8,5Hz); 7,02 (d, 2H, J=8,5Hz); 7,14-7,30 (m, 5H). |
| **6-22** | Etape 1 : Préparation du 3-(2-méthoxy-4-nitrophényl)acrylate d'éthyle (Protocole ST et PA) | Rendement : 20%. Aspect : solide jaune. |
| | | RMN ¹H :1,36 (t, 3H, J=7,2Hz); 4,01 (s, 3H); 4,30 (q, 2H, J=7,2Hz); 6,64 (d, 1H, J=16,2Hz); 7,63 (d, 1H, J=8,5Hz); 7,78 (d, 1H, J=2,0Hz); 7,85 (dd, 1H, J=2,0Hz J=8,5Hz); 7,95 (d, 1H, J=16,2Hz). |
| | Etape 2 : Obtention du 3-(4-amino-2-méthoxyphényl)propanoate d'éthyle (Protocole SR) | Rendement : 64%. Aspect : huile incolore. |
| | | RMN ¹H :1,25 (t, 3H, J=7,0Hz); 2,54 (t, 2H, J=7,3Hz); 2,83 (t, 2H, J=7,3Hz); 3,78 (s, 3H); 4,12 (q, 2H, J=7,0Hz); 6,20-6,24 (m, 2H); 6,92 (d, 1H, J=8,8Hz). |
| **6-23** | Etape 1 : Préparation du 3-(3-méthoxy-4-nitrophényl)acrylate d'éthyle (Protocole ST et PA) | Rendement : 43%. Aspect : solide jaune. |
| | | RMN ¹H :1,36 (t, 3H, J=7,3Hz); 4,00 (s, 3H); 4,30 (q, 2H, J=7,3Hz); 6,52 (d, 1H, J=16,1Hz); 7,18-7,21 (m, 2H); 7,65 (d, 1H, J=16,1Hz); 7,88 (d, 1H, J=8,8Hz). |
| | Etape 2 : Obtention du 3-(4-amino-3-méthoxyphényl)propanoate d'éthyle (Protocole SR) | Rendement : 99%. Aspect : huile incolore. |
| | | RMN ¹H :1,25 (t, 3H, J=7,0Hz); 2,58 (m, 2H); 2,87 (m, 2H); 3,84 (s, 3H); 4,13 (q, 2H, J=7,0Hz); 6,66-6,64 (m, 3H). |
| **6-24** | Etape 1 : Préparation du 3-(4-nitrophenyl)but-2-ènoate d'éthyle (Protocole SQ et PD) | Rendement : 84%. Aspect : solide jaune. |
| | | RMN ¹H :1,34 (s, 3H, J=7,2Hz); 2,59 (d, 3H, J=1,5Hz); 4,24 (q, 2H, J=7,2Hz); 6,19 (d, 1H, J=1,5Hz); 7,62 (d, 2H, J=8,9Hz); 8,23 (d, 2H, J=8,9Hz). |
| | Etape 2: Obtention du 3-(4-aminophényl)butanoate d'éthyle (Protocole SR) | Rendement : 59%. Aspect : huile incolore |
| | | RMN ¹H :1,20 (t, 3H, J=7,0Hz); 1,26 (d, 3H, J=7,0Hz); 2,44-2,60 (m, 2H); 3,12-3,22 (m, 1H); 3,57 (m, 2H); 4,08 (q, 2H, J=7,0Hz); 6,64 (d, 2H, J=8,3Hz); 7,02 (d, 2H, J=8,3Hz). |

La synthèse des Intermédiaires de Figures 6b et 6d nécessite une seule étape et elle est résumé dans le Tableau 6-2.

**Tableau 6-2**

| **Ex.** | **Intermédiaires (Protocole)** | **Détails** |
|---|---|---|
| **6-10** | 2-(4-aminophénylthio)-2-méthylpropanoate d'éthyle (Protocole SQ et PA) | Rendement : 87%. Aspect : huile incolore. RMN ¹H : 1,13 (t, 3H, J=7,1Hz); 1,33 (s, 6H); 4,00 (q, 2H, J=7,1Hz); 5,47 (s, 2H); 6,51 (d, 2H, J=8,5Hz); 7,04 (d, 2H, J=8,5Hz). |
| **6-14** | 2-(4-aminophénylthio)acétate de tertiobutyle (Protocole SQ et PA) | Rendement : 28%. Aspect : huile jaune. |
| | | RMN ¹H :1,40 (s, 9H); 3,37 (s, 2H); 3,77 (s(large), 2H); 6,59 (d, 2H, J=8,5Hz); 7,28 (d, 2H, J=8,5Hz). |
| **6-15** | chlorhydrate de 3-(4-aminophényl)propanoate d'éthyle (Protocole SS et PE) | Rendement : 86%. Aspect : solide blanc. |
| | | RMN ¹H : 1,12 (t, 3H, J=7,3Hz); 2,60 (t, 2H, J=7,3Hz); 2,85 (t, 2H, J=7,3Hz); 4,00 (q, 2H, J=7,3Hz); 7,27 (d, 2H, J=8,2Hz); 7,33 (d, 2H, J=8,2Hz). |
| **6-16** | 2-(4-aminophénylthio)-2-méthylpropanoate de tertiobutyle (Protocole SQ et PA) | Rendement : 79%. Aspect : solide blanc. |
| | | RMN ¹H :1,39 (s, 6H); 1,43 (s, 9H); 3,81 (s(large), 2H); 6,60 (d, 2H, J=8,5Hz); 7,28 (d, 2H, J=8,5Hz). |
| **6-17** | 2-((4 amino)phénylthio) éthanoate d'éthyle (Protocole SQ et PA) | Rendement: 41%. Aspect: huile incolore. RMN ¹H :1,21 (t, 3H, J=7,2Hz); 3,45 (s, 2H); 3,75 (s, 2H); 4,13 (q, 2H, J=7,2Hz); 6,61 (d, 2H, J=8,5Hz); 7,29 (d, 2H, J=8,5Hz) |
| **6-18** | 2-(4-aminophénylthio)-2,2-difluoroacétate d'éthyle (Protocole SQ et PA) | Rendement : 29%. Aspect : huile incolore |
| | | RMN ¹H :1,29 (t, 3H, J=7,0Hz); 4,26 (q, 2H, J=7,0Hz); 6,66 (d, 2H, J=8,8Hz); 7,37 (d, 2H, J=8,5Hz). |
| **6-19** | 2-(4-aminophénylthio)-2-phényléthanoate d'éthyle (Protocole SQ et PA) | Rendement : 37%. Aspect : huile jaune. |
| | | RMN ¹H :1,18 (t, 3H, J=7,3Hz); 3,75 (s (large), 2H); 4,06-4,18 (m, 2H); 4,73 (s, 1H); 6,54 (d, 2H, J=8,8Hz); 7,21 (d, 2H, J=8,5Hz); 7,27-7,34 (m, 3H); 7,41-7,44 (m, 2H). |

Pour Exemple 6-25 (5-(1-(4-hydroxyphényl)but-2-ynyl)-2,2-diméthyl-1,3-dioxane-4,6-dione), la synthèse de ce composé nécessite 2 étapes. Dans la première (préparation du 5-(4-hydroxybenzylidene)-2,2-dimethyl-1,3-dioxane-4,6-dione), une solution de 4-hydroxybenzaldéhyde dans de l'eau (1 mol/L) est portée à 75°C. L'acide de meldrum (1,05 éq.) est additionné par portions puis, le milieu réactionnel est laissé sous agitation à 75°C pendant 2 heures. Le milieu réactionnel est refroidi et agité pendant 2 heures à 0°C. Le précipité est essoré puis, lavé par de l'eau glacée et de l'heptane (rendement : 90%; Aspect : solide jaune ; RMN ¹H :1,80 (s, 6H); 6,94 (d, 2H, J=8,8Hz); 8,19 (d, 2H, J=8,8Hz); 8,39 (s, 1H)). Dans la deuxième (obtention du 5-(1-(4-hydroxyphényl)but-2-ynyl)-2,2-diméthyl-1,3-dioxane-4,6-dione), une solution de l'intermédiaire précédent dans du tétrahydrofurane (0,5 mol/L) est additionnée sous atmosphère inerte, goutte à goutte (0,25 heures), sur une solution de bromure de 1-propylmagnésium dans du tétrahydrofurane (0,5 mol/L, 2 éq.). Après 0,25 heures d'agitation à température ambiante, le milieu réactionnel est dilué par une solution aqueuse de chlorure d'ammonium (0,6 N, 3 éq.), extrait par du cyclohexane, acidifié (pH=2) par du bisulfate de sodium . Le résidu d'évaporation est utilisé sans autre forme de purification (rendement: 98%; Aspect: solide jaune; RMN ¹H: 1,64 (s, 3H); 1,82 (s, 3H); 1,83 (s, 3H); 4,46 (d, 1H, J=2,6Hz); 4,73 (d, 1H, J=2,6Hz); 6,76 (d, 2H, J=8,5Hz); 7,39 (d, 2H, J=8,5Hz); 8,24 (s, 1H)).

### Exemple 7 : Synthèse des composés selon l'invention

La synthèse des composés selon l'invention de Figures 7a nécessite 2 ou 3 étapes et elle est résumée dans le Tableau 7-1.

**Tableau 7-1**

| **Cpd** | **No. et type d'Etape (Ex. ; Protocole)** | **Détails** |
|---|---|---|
| **4** | 1 : Préparation du 2-(4-((2-méthoxy-6-phénylpyridin-3-yl)méthylamino)-phénoxy)éthanoate de tertiobutyle (Ex. 5-1 et Ex. 6-6 avec Protocole SX et PA) | Rendement : quantitatif. Aspect : huile jaune |
| | | RMN ¹H :1,49 (s, 9H); 4,11 (s, 3H); 4,30 (s, 2H); 4,43 (s, 2H); 6,61 (d, 2H, J=8,8Hz); 6,78 (d, 2H, J=8,8Hz); 7,31 (d, 1H, J=7,6Hz); 7,34-7,49 (m, 3H); 7,61 (d, 1H, J=7,6Hz); 8,02 (m, 2H). |
| | 2: Obtention du acide 2-(4-(((2-méthoxy-6-phénylpyridin-3yl)méthyl)amino)phénoxy) éthanoïque (Protocole SW et PE) | Rendement: 81%. Aspect: solide blanc |
| | | RMN ¹H: 4,03 (s, 3H); 4,18 (s, 2H); 4,47 (s, 2H); 6,50 (d, 2H, J=8,8Hz); 6,68 (d, 2H, J=8,8Hz); 7,55-7,35 (m, 4H); 7,64 (d, 1H, J=7,6Hz); 8,06 (m, 2H). |
| **8** | 1 : Préparation du 2-(4-((2-tertiobutoxy-6-phénylpyridin-3-yl)méthylamino)-phénoxy)éthanoate d'éthyle (Ex. 5-2 et Ex. 6-8 avec Protocole SX et PA) | Rendement : 51%. Aspect : solide blanc |
| | | RMN ¹H :1,29 (t, 3H, J=7,0Hz); 1,71 (s, 9H); 4,23-4,29 (m, 4H); 4,53 (s, 2H); 6,64 (d, 2H, J=9,1Hz); 6,80 (d, 2H, J=9,1 Hz); 7,27 (d, 1H, J=7,6Hz); 7,35-7,47 (m, 3H); 7,57 (d, 1H, J=7,6Hz); 8,0 (d, 2H, J=7,3Hz). |
| | 2 : Obtention du acide 2-(4-((2-tertio-butyloxy-6-phénylpyridin-3-yl)méthyl)amino)phénoxy) éthanoïque (Procole SU et PA) | Rendement : 63%. Aspect : solide blanc. |
| | | RMN ¹H: 1,67 (s, 9H); 4,12 (s, 2H); 4,43 (s, 2H); 6,48 (d, 2H, J=9,1 Hz); 6,67 (d, 2H, J=9,1Hz); 7,35-7,49 (m, 4H); 7,60 (d, 1H, J=7,9Hz); 7,99 (d, 2H, J=7,3Hz). |
| **9** | 1 : Préparation du 2-(4-((2-tertiobutoxy-6-phénylpyridin-3-yl)méthylamino)-phénoxy)-2-méthyl-propanoate d'éthyle (Ex. 5-2 et Ex. 6-9 avec Protocole SX et PA) | Rendement : 79%. Aspect : huile orange. |
| | | RMN ¹H :1,28 (t, 3H, J=7,3Hz); 1,52 (s, 6H); 1,71 (s, 9H); 4,19-4,26 (m, 4H); 6,55 (d, 2H, J=9,0Hz); 6,77 (d, 2H, J=9,0Hz); 7,27 (d, 1H, J=7,6Hz); 7,35-7,48 (m, 3H); 7,56 (d, 1H, J=7,6Hz); 7,99 (d, 2H, J=7,7Hz). |
| | 2 : Obtention du acide 2-(4-(((2-tertio-butyloxy-6-phénylpyridin-3-yl)méthyl)amino)phénoxy)-2-méthyl-propanoïque (Protocole SU et PD) | Rendement : 46%. Aspect : solide jaune. RMN ¹H: 1,36 (s, 6H); 1,66 (s, 9H); 4,11 (s, 2H); 6,46 (d, 2H, J=8,8Hz); 6,67 (d, 2H, J=8,8Hz); 7,35-7,49 (m, 4H); 7,61 (d, 1H, J=7,6Hz); 8,0 (d, 2H, J=7,0Hz). |
| **10** | 1 : Préparation du 2-(4-((2-tertiobutoxy-6-phénylpyridin-3-yl)méthylamino)-phénoxy)propanoate d'éthyle (Ex. 5-2 et Ex. 6-7 avec Protocole SX et PA) | Rendement : 52%. Aspect : huile jaune. |
| | | RMN ¹H :1,24 (t, 3H, J=7,3Hz); 1,57 (d, 3H, J=6,7Hz); 1,71 (s, 9H); 4,17-4,23 (m, 4H); 4,61 (q, 1H, J=6,7Hz); 6,60 (d, 2H, J=9,0Hz); 6,77 (d, 2H, J=9,0Hz); 7,26 (d, 1H, J=7,6Hz); 7,37-7,47 (m, 3H); 7,56 (d, 1H, J=7,6Hz); 7,99 (d, 2H, J=7,0Hz). |
| | 2 : Obtention du acide 2-(4-(((2-tertio-butyloxy-6-phénylpyridin-3-yl)méthyl)amino)phénoxy)-propanoïque (Protocole SU et PA) | Rendement : 18%. Aspect : solide blanc. RMN ¹H : 1,4 (d, 3H, J=6,7Hz); 1,66 (s, 9Hz); 4,11 (s, 2H); 4,50-4,57 (m, 1H); 6,48 (d, 2H, J=8,8Hz); 6,64 (d, 2H, J=8,8Hz); 7,35-7,40 (m, 1H); 7,44-7,49 (m, 3H); 7,6 (d, 1H, J=7,6Hz); 7,99 (d, 2H, J=7,3Hz). |
| **11** | 1 : Préparation du 2-(4-((2-méthoxy-6-phénylpyridin-3-yl)méthylamino)-phénylthio)-2-méthyl-propanoate d'éthyle (Ex. 5-1 et Ex. 6-10 avec Protocole SX et PA) | Rendement: 87%. Aspect : huile orangée RMN ¹H :1,22 (t, 3H, J=7,3Hz); 1,45 (s, 6H); 4,04-4,16 (m, 5H); 4,35 (s, 2H); 6,56 (d, 2H, J=8,5Hz); 7,26 (d, 2H, J=8,5Hz); 7,31 (d, 1H, J=7,6Hz); 7,37-7,51 (m, 3H); 7,59 (d, 1H, J=7,6Hz); 8,19 (d, 2H, J=8,5Hz). |
| | 2 : Obtention du acide 2-(4-(((2-méthoxy-6-phénylpyridin-3-yl) méthyl)amino)phénylthio)-2-méthyl-propanoïque (Protocole SU et PA) | Rendement : 57%. Aspect : solide beige |
| | | RMN ¹H : 1,29 (s, 6H); 4,04 (s, 3H); 4,24 (d, 2H, J=5,8Hz); 6,54 (m, 3H); 7,14 (d, 2H, J=8,5Hz); 7,37-7,54 (m, 4H); 7,64 (d, 1H, J=7,6Hz); 8,08 (d, 2H, J=7,0Hz), 12,34 (s, 1H). |
| **12** | 1 : Préparation du 2-(4-((2-méthoxy-6-phénylpyridin-3-yl)méthylamino)phénoxy)-2-méthyl-propanoate de tertiobutyle (Ex. 5-1 et Ex. 6-11 avec Protocole SX et PA) | Rendement : 71%. Aspect : huile jaune. |
| | | RMN ¹H :1,44 (s, 9H); 1,49 (s, 6H); 4,09 (s, 3H); 4,32 (s, 2H); 6,61-6,72 (m, 2H); 6,79 (d, 2H, J=9,1 Hz); 7,30 (d, 1H, J=7,6Hz); 7,36-7,48 (m, 3H); 7,62-7,65 (m, 1H); 8,03 (d, 2H, J=8,2Hz). |
| | 2 : Obtention du acide 2-(4-(((2-méthoxy-6-phénylpyridin-3-yl)méthyl)amino)phénoxy)-2-méthyl-propanoïque (Protocole SW et PC) | Rendement : 70%. Aspect : solide blanc |
| | | RMN ¹H : 1,36 (s, 6H); 4,03 (s, 3H); 4,17 (s, 2H); 5,89 (s(large), 1H); 6,47 (d, 2H, J=9,1Hz); 6,67 (d, 2H, J=9,1Hz); 7,37-7,54 (m, 4H); 7,66 (d, 1H, J=7,6Hz); 8,07 (d, 2H, J=7,0Hz). |
| **16** | 1 : Préparation du 2-(4-((2-méthoxy-6-phénylpyridin-3-yl)méthylamino)-phénylthio)éthanoate de tertiobutyle (Ex. 5-1 et Ex. 6-14 avec Protocole SX et PA) | Rendement : 99%. Aspect : huile jaune. |
| | | RMN ¹H :1,40 (s, 9H); 3,38 (s, 2H); 4,12 (s, 3H); 4,34 (s, 2H); 6,58 (d, 2H, J=8,8Hz); 7,29-7,31 (m, 3H); 7,37-7,48 (m, 3H); 7,58 (d, 1H, J=7,6Hz); 8,04 (d, 2H, J=8,5Hz). |
| | 2 : Obtention du acide 2-(4-(((2-méthoxy-6-phénylpyridin-3-yl)méthyl)amino)phénylthio)-éthanoïque (Protocole SW et PC) | Rendement : 64%. Aspect : solide blanc |
| | | RMN ¹H :3,49 (s, 2H); 4,11 (s, 3H); 4,34 (s, 2H); 6,58 (d, 2H, J=8,0Hz); 7,30-7,48 (m, 6H); 7,58 (d, 1H, J=7,1 Hz); 8,03 (d, 2H, J=8,0Hz). |
| **19** | 1 : Préparation du 3-(4-((2-méthoxy-6-phénylpyridin-3-yl)méthylamino)-phényl)propanoate d'éthyle (Ex. 5-1 et Ex. 6-15 avec Protocole SX et PA) | Rendement : 53%. Aspect : huile jaune. |
| | | RMN ¹H :1,24 (t, 3H, J=7,3Hz); 2,56 (t, 2H, J=8,2Hz); 2,84 (t, 2H, J=8,2Hz); 4,09-4,16 (m, 5H); 4,33 (s, 2H); 6,64 (d, 2H, J=8,2Hz); 7,03 (d, 2H, J=8,2Hz); 7,30 (d, 1H, J=7,6Hz); 7,36-7,48 (m, 3H); 7,63 (d, 1H, J=7,6Hz); 8,04 (d, 2H, J=7,3Hz). |
| | 2 : Obtention du acide 3-(4-(((2-méthoxy-6-phénylpyridin-3-yl)méthyl)amino)phényl) propanoïque (Protocole SU et PC) | Rendement : 73%. Aspect : solide jaune. |
| | | RMN ¹H :2,62 (t, 2H, J=7,6Hz); 2,85 (t, 2H, J=7,6Hz); 4,10 (s, 3H); 4,34 (s, 2H); 6,66 (d, 2H, J=8,2Hz); 7,03 (d, 2H, J=8,2Hz); 7,30 (d, 1H, J=7,6Hz); 7,36-7,48 (m, 3H); 7,63 (d, 1H, J=7,6Hz); 8,04 (m, 2H). |
| **22** | 1 : Préparation du 2-(4-((2-méthoxy-6-(4-(trifluorométhyl)phényl)pyridin-3-yl)méthylamino)phénylthio)-2-méthylpropanoate de tertiobutyle (Ex. 5-3 et Ex. 6-16 avec Protocole SX et PA) | Rendement : 92%; Aspect : huile jaune; |
| | | RMN ¹H :1,39 (s, 6H); 1,43 (s, 9H); 4,12 (s, 3H); 4,37 (s, 2H); 6,56 (d, 2H, J=8,5Hz); 7,30 (d, 2H, J=8,5Hz); 7,34 (d, 1H, J=7,6Hz); 7,62 (d, 1H, J=7,6Hz); 7,71 (d, 2H, J=8,2Hz); 8,50 (d, 2H, J=8,2Hz). |
| | 2 : Obtention du acide 2-(4-(((2-méthoxy-6-(4-(trifluorométhyl) phényl)pyridin-3-yl)méthyl)amino) - phénylthio)-2-méthylpropanoïque (Protocole SW et PB) | Rendement : 55%; Aspect : solide jaune. |
| | | RMN ¹H :1,47 (s, 6H); 4,11 (s, ·3H); 4,36 (s, 2H); 6,57 (d, 2H, J=8,8Hz); 7,3-7,35 (m, 3H); 7,61 (d, 1H, J=7,6Hz); 7,69 (d, 2H, J=8,0Hz); 8,12 (d, 2H, J=8,0Hz |
| **23** | 1 : Préparation du 2-(4-((2-méthoxy-6-(4-(trifluorométhyl)phényl)pyridin-3-yl)méthylamino)phénylthio)éthanoate de tertiobutyle (Ex. 5-3 et Ex. 6-14 avec Protocole SX et PA) | Rendement : 96%. Aspect : huile jaune orange. |
| | | RMN ¹H :1,41 (s, 9H); 3,37 (s, 2H); 4,12 (s, 3H); 4,42 (s, 2H); 6,57 (d, 2H, J=8,6Hz); 7,30-7,35 (m, 3H); 7,62 (d, 1H, J=7,6Hz); 7,71 (d, 2H, J=8,5Hz); 8,14 (d, 2H, J=7,9Hz). |
| | 2 : Obtention du acide 2-(4-(((2-méthoxy-6-(4-(trifluorométhyl) phényl)pyridin-3-yl)méthyl)amino) - phénylthio)éthanoïque (Protocole SW et PB) | Rendement : 21%. Aspect : solide jaune. |
| | | RMN ¹H :3,50 (s, 2H); 4,12 (s, 3H); 4,36 (s, 2H); 6,58 (d, 2H, J=8,8Hz); 7,32-7,36 (m, 3H); 7,61 (d, 1H, J=7,6Hz); 7,70 (d, 2H, J=8,2Hz); 8,14 (d, 2H, J=8,2Hz). |
| **26** | 1 : Préparation du 2-(4-((2-méthoxy-5-phénylpyridin-3-yl)méthylamino)-phénylthio)éthanoate d'éthyle (Ex. 5-4 et Ex. 6-17 avec Protocole SX et PA) | Rendement: 73%. Aspect : huile jaune |
| | | RMN ¹H :1,19 (t, 3H, J=7,3Hz); 3,48 (s, 2H); 4,03 (s, 3H); 4,12 (q, 2H, J=7,3Hz); 4,37 (s, 2H); 6,77 (d, 2H, J=7,9Hz); 7,29-7,50 (m, 8H); 7,84 (d, 1H, J=2,0Hz); 8,30 (d, 1H, J=2,0Hz). |
| | 2 : Obtention du acide 2-(4-(((2-méthoxy-5-phénylpyridin-3-yl)méthyl)amino)phénylthio) éthanoïque (Protocole SU et PE) | Rendement : 66%. Aspect : solide blanc. |
| | | RMN ¹H : 3,44 (s, 2H); 3,97 (s, 3H); 4,24 (d, 2H, J=5,0Hz); 6,39 (s (large), 1H); 6,56 (d, 2H, J=8,6Hz); 7,17 (d, 2H, J=8,6Hz); 7,31-7,37 (m, 1H); 7,41-7,46 (m, 2H); 7,54 (d, 2H, J=7,3Hz); 7,86 (d, 1H, J=2,3Hz); 8,36 (d, 1H, J=2,3Hz); 12,44 (s, 1H). |
| **27** | 1 : Préparation du 2-(4-((2-méthoxy-6-phénylpyridin-3-yl)méthylamino)-phénylthio)-2-2-difluoroéthanoate d'éthyle (Ex. 5-1 et Ex. 6-18 avec Protocole SX et PA) | Rendement : 88%. Aspect : huile jaune. |
| | | RMN ¹H :1,26 (t, 3H, J=7,3Hz); 4,11 (s, 3H); 4,24 (q, 2H, J=7,3Hz); 4,37 (s, 2H); 6,65 (d, 2H, J=8,8Hz); 7,32 (d, 1H, J=7,6Hz); 7,38-7,49 (m, 5H); 7,58 (d, 1H, J=7,3Hz); 8,04 (d, 2H, J=7.0Hz). |
| | 2 : Obtention du acide 2-(4-(((2-méthoxy-6-phénylpyridin-3-yl) méthyl)amino)phénylthio)-2,2-difluoro - éthanoïque (Protocole SU et PC) | Rendement : 36%. Aspect : solide blanc. |
| | | RMN ¹H : 4,04 (s, 3H); 4,27 (s, 2H); 6,62 (d, 2H, J=8,5Hz); 7,26 (d, 2H, J=8,5Hz); 7,37-7,54 (m, 4H); 7,62 (d, 1H, J=7,6Hz); 8,07 (d, 2H, J=7,3Hz). |
| **28** | 1 : Préparation du 2-(4-((2-méthoxy-5-6-diphénylpyridin-3-yl)méthylamino)-phénylthio) éthanoate d'éthyle (Ex. 5-5 et Ex. 6-17 avec Protocole SX et PA) | Rendement : 73%. Aspect : huile incolore. RMN ¹H :1,19 (t, 3H, J=7,0Hz); 3,46 (s, 2H); 4,08 (s, 3H); 4,12 (q, 2H, J=7,0Hz); 4,38 (s, 2H); 6,66 (d, 2H, J=8,5Hz); 7,09-7,12 (m, 2H); 7,18-7,25 (m, 6H); 7,34 (d, 2H, J=8,8Hz); 7,37-7,40 (m, 2H); 7,59 (s, 1H). |
| | 2 : Obtention du acide 2-(4-(((2-méthoxy-5,6-diphénylpyridin-3-yl)méthyl)amino)phénylthio)-éthanoïque (Protocole SU et PA) 3 : Protocole PC | Rendement : 6,56%. Aspect : solide blanc. |
| | | RMN ¹H : 3,38 (s, 2H); 4,00 (s, 3H); 4,26 (d, 2H, J=5,6Hz); 6,31 (m, 1H); 6,55 (d, 2H, J=8,5Hz); 7,05 (dd, 2H, J=2,3Hz J=7,9Hz); 7,13 (d, 2H, J=8,5Hz); 7,23-7,32 (m, 8H); 7,59 (s, 1H). |
| **29** | 1 : Préparation du 2-(4-((2-méthoxy-5-6-diphénylpyridin-3-yl) méthyl amino)-phénylthio)éthanoate d'éthyle (Ex. 5-6 et Ex. 6-17 avec Protocole SX et PA) | Rendement : 58%. Aspect : huile incolore. |
| | | RMN ¹H :1,23 (t, 3H, J=7,1Hz); 3,50 (s, 2H); 4,00 (s, 3H); 4,15 (q, 2H, J=7,1Hz); 4,34 (s, 2H); 6,72 (m, 2H); 7,31-7,46 (m, 5H); 7,74-7,87 (m, 3H). |
| | 2 : Obtention du acide 2-(4-(((2-méthoxy-5-bromo-6-phénylpyridin-3-yl)méthyl)amino) phénylthio)-éthanoïque (Protocole SU et PA) 3 : Protocole PC | Rendement : 34%. Aspect : solide blanc. RMN ¹H : 3,46 (s, 2H); 3,93 (s, 3H); 4,22 (d, 2H, J=5,8Hz); 6,45 (t, 1H, J=5,8Hz); 6,56 (d, 2H, J=8,6Hz); 7,19 (d, 2H, J=8,6Hz); 7,42-7,50 (m, 3H); 7,67 (m, 2H); 7,80 (s, 1H); 12,51 (s, 1H). |
| **30** | 1 : Préparation du 2-(4-((2-méthoxy-6-furylpyridin-3-yl)méthylamino)-phénylthio)éthanoate d'éthyle (Ex. 5-è et Ex. 6-17 avec Protocole SX et PA) | Rendement : 85%. Aspect : huile jaune. RMN ¹H :1,21 (t, 3H, J=7,0Hz); 3,49 (s, 2H); 4,02 (s, 3H); 4,15 (q, 2H, J=7,0Hz); 4,32 (s, 2H); 6,50-6,53 (m, 1H); 6,76 (d, 2H, J=8,6Hz); 7,00 (d, 1H, J=2,9Hz); 7,20 (d, 1H, J=7,6Hz); 7,31 (d, 2H, J=8,6Hz); 7,49-7,54 (m, 1H); 7,60 (d, 1H, J=7,6Hz). |
| | 2 : Obtention du acide 2-(4-(((2-méthoxy-6-furylpyridin-3-yl)méthyl)amino)phénylthio)-éthanoïque (Protocole SU et PC) | Rendement : 57%. Aspect : solide beige. RMN ¹H : 3,44 (s, 2H); 3,98 (s, 3H); 4,19 (d, 2H, J=5,6Hz); 6,39 (t, 1H, J=5,6Hz); 6,51 (d, 2H, J=8,8Hz); 6,61-6,63 (m, 1H); 7,02 (d, 1H, J=3,2Hz); 7,16 (d, 2H, J=8,8Hz); 7,28 (d, 1H, J=7,6Hz); 7,59 (d, 1H, J=7,6Hz); 7,78 (m, 1H). |
| **31** | 1 : Préparation du 3-(4-((2-méthoxy-6-furylpyridin-3-yl)méthylamino)-phényl)propanoate d'éthyle (Ex. 5-6 et Ex. 6-15 avec Protocole SX et PA) | Rendement : 68%. Aspect : huile jaune. |
| | | RMN ¹H : 1,13 (t, 3H, J=7,1Hz); 2,46 (t, 2H, J=7,3Hz); 2,66 (t, 2H, J=7,3Hz); 3,99 (s, 3H); 4,00 (q, 2H, J=7,1Hz); 4,17 (d, 2H, J=6,0Hz); 6,03 (t, 1H, J=6,0Hz) 6,46 (d, 2H, J=8,3Hz); 6,61-6,63 (m, 1H); 6,90 (d, 2H, J=8,3Hz); 7,02 (d, 1H, J=3,2Hz); 7,26 (d, 1H, J=7,6Hz); 7,59 (d, 1H, J=7,6Hz); 7,79 (s, 1H). |
| | 2 : Obtention du acide 3-(4-(((2-méthoxy-6-furylpyridin-3-yl)méthyl)amino)phényl)propanoïque (Protocole SU et PC) | Rendement : 24%. Aspect : solide beige. RMN ¹H : 2,41 (t, 2H, J=7,6Hz); 2,64 (t, 2H, J=7,6Hz); 3,99 (s, 3H); 4,17 (s, 2H); 6,03 (s (large), 1H); 6,47 (d, 2H, J=8,2Hz); 6,63 (m, 1H); 6,91 (d, 2H, J=8,2Hz); 7,03 (d, 1H, J=3,2Hz); 7,26 (d, 1H, J=7,6Hz); 7,60 (d, 1H, J=7,6Hz); 7,79 (s, 1H). |
| **32** | 1 : Préparation du 2-(4-((2-méthoxy-6-phénylpyridin-3-yl)méthylamino)-phénylthio)-2-phényléthanoate d'éthyle (Ex. 5-1 et Ex. 6-19 avec Protocole SX et PA) | Rendement : 75%. Aspect : huile jaune. |
| | | RMN ¹H :1,16 (t, 3H, J=7,0Hz); 4,05-4,20 (m, 5H); 4,33 (s, 2H); 4,69 (s, 1H); 6,53 (d, 2H, J=8,6Hz); 7,22 (d, 2H, J=8,6Hz); 7,28-7,33 (m, 4H); 7,39-7,49 (m, 5H); 7,56 (d, 1H, J=7,6Hz); 8,02-8,05 (m, 2H). |
| | 2 : Obtention du acide 2-(4-(((2-méthoxy-6-phénylpyridin-3-yl) méthyl)amino)phénylthio)-2-phényl-éthanoïque (Protocole SU et PA) | Rendement : 58%. Aspect : solide blanc. |
| | | RMN 1H : 4,03 (s, 3H); 4,21 (d, 2H, J=5,6Hz); 4,71 (s, 1H); 6,43-6,48 (m, 3H); 7,07 (d, 2H, J=8,8Hz); 7,25-7,59 (m, 10H); 8,07 (d, 2H, J=7,0Hz). |
| | 3 : Protocole PC | |
| **35** | 1 : Préparation du 2-(4-((2-méthoxy-6-phénylpyridin-3-yl)méthylamino)-2,6-diméthyl-phénoxy)éthanoate d'éthyle (Ex. 5-1 et Ex. 6-20 avec Protocole SX et PA) | Rendement : 96%. Aspect : huile jaune. |
| | | RMN 1H (300MHz, CDCl3, d en ppm) : 1,33 (t, 3H, J=7,0Hz); 2,23 (s, 6H); 4,11 (s, 3H); 4,26-4,34 (m, 6H); 6,31 (s, 2H); 7,32 (d, 1H, J=7,6Hz); 7,38-7,48 (m, 3H); 7,61 (d, 1H, J=7,6Hz); 8,03-8,07 (d, 2H, J=7,0Hz). |
| | 2: Obtention du acide 2-(4-(((2-méthoxy-6-phénylpyridin-3-yl) méthyl)amino)-2,6-diméthyl-phénoxy) éthanoïque (Protocole SU et PA) | Rendement : 6%. Aspect : solide blanc. |
| | | RMN ¹H : 2,08 (s, 6H); 4,03 (s, 3H); 4,15 (m, 4H); 6,21 (s, 2H); 7,36-7,54 (m, 4H); 7,63 (d, 1H, J=7,6Hz); 8,06-8,09 (m, 2H). |
| **36** | 1 : Préparation du 3-(4-((2-méthoxy-6-(4-(trifluorométhyl)phényl)pyridin-3-yl)méthylamino)phényl)propanoate d'éthyle (Ex. 5-3 et Ex. 6-15 avec Protocole SX et PA) | Rendement : 43%. Aspect : huile orange. |
| | | RMN ¹H :1,24 (t, 3H, J=7,1 Hz); 2,56 (t, 2H, J=7,5Hz); 2,85 (t, 2H, J=7,5Hz); 4,09 (s, 3H); 4,12 (q, 2H, J=7,1Hz); 4,37 (s, 2H); 6,73-6,94 (m, 2H); 7,05 (d, 2H, J=8,1Hz); 7,32 (d, 1H, J=7,5Hz); 7,66-7,72 (m, 3H); 8,13 (d, 2H, J=8,0Hz). |
| | 2: Obtention du acide 3-(4-(((2-méthoxy-6-(4-(trifluorométhyl)phényl)pyridin-3-yl)méthyl)amino) -phényl)-propanoïque (Protocole SU et PA) | Rendement : 58%. Aspect : solide blanc. |
| | | RMN ¹H : 2,39 (t, 2H, J=7,3Hz); 2,63 (t, 2H, J=7,3Hz); 4,06 (s, 3H); 4,22 (d, 2H, J=5,9Hz); 6,05 (t, 1H, J=5,9Hz); 6,48 (d, 2H, J=8,3Hz); 6,91 (d, 2H, J=8,3Hz); 7,62-7,69 (m, 2H); 7,83 (d, 2H, J=8,4Hz); 8,29 (d, 2H, J=8,4Hz). |
| **38** | 1 : Préparation du 3-(4-((2-(éthyfthio)-6-phénylpyridin-3-yl)méthylamino)-phényl)propanoate d'éthyle (Ex. 5-9 et Ex. 6-15 avec Protocole SX et PA) | Rendement : 84%. Aspect : solide jaune. RMN ¹H :1,24 (t, 3H, J=7,3Hz); 1,50 (t, 3H, J=7,3Hz); 2,56 (t, 2H, J=7,6Hz); 2,85 (t, 2H, J=7,6Hz); 3,41 (q, 2H, J=7,3Hz); 4,13 (q, 2H, J=7,3Hz); 4,32 (s, 2H); 6,57 (d, 2H, J=8,5Hz); 7,02 (d, 2H, J=8,5Hz); 7,40-7,50 (m, 4H); 7,60 (d, 1H, J=7,9Hz); 8,04-8,07 (m, 2H). |
| | 2 : Obtention du acide 3-(4-(((2-(éthylthio)-6-phénylpyridin-3-yl)méthyl)amino)phényl)-propanoïque (Protocole SU et PA) | Rendement : 40%. Aspect : solide beige. |
| | | RMN ¹H : 1,40 (t, 3H, J=7,3Hz); 2,41 (t, 2H, J=7,3Hz); 2,64 (t, 2H, J=7,3Hz); 3,30-3,38 (m, 2H); 4,16 (d, 2H, J=5,9Hz); 6,15 (t, 1H, J=5,9Hz); 6,44 (d, 2H, J=8,5Hz); 6,92 (d, 2H, J=8,5Hz); 7,39-7,51 (m, 3H); 7,59-7,68 (m, 2H); 8,09 (d, 2H, J=7,0Hz); 12,00 (s, 1H). |
| **39** | 1 : Préparation du 3-(4-((2-méthoxy-6-(parabiphényl)pyridin-3-yl)méthyl-amino)phényl)-propanoate d'éthyle (Ex. 5-10 et Ex. 6-15 avec Protocole SX et PA) | Rendement : 84%. Aspect : huile jaune. |
| | | RMN ¹H :1,25 (t, 3H, J=7,0Hz); 2,57 (t, 2H, J=7,3Hz); 2,86 (t, 2H, J=7,3Hz); 4,10-4,17 (m, 5H); 4,34 (s, 2H); 6,61 (d, 2H, J=8,5Hz); 7,03 (d, 2H, J=8,5Hz); 7,34-7,41 (m, 2H); 7,48 (m, 2H); 7,62-7,72 (m, 5H); 8,13 (d, 2H, J=8,5Hz). |
| | 2 : Obtention du acide 3-(4-(((2-méthoxy-6-(parabiphényl)pyridin-3-yl)méthyl)amino)phényl)-propanoïque (Protocole SU et PA) | Rendement : 60%. Aspect : solide beige. |
| | | RMN ¹H : 2,41 (t, 2H, J=7,3Hz); 2,64 (t, 2H, J=7,3Hz); 4,06 (s, 3H); 4,21 (d, 2H, J=5,6Hz); 6,03 (t, 1H, J=5,6Hz); 6,49 (d, 2H, J=8,5Hz); 6,91 (d, 2H, J=8,5Hz); 7,36-7,41 (m, 1H); 7,49 (t, 2H, J=7,0Hz); 7,58 (d, 1H, J=7,6Hz); 7,64 (d, 1H, J=7,6Hz); 7,72-7,79 (m, 4H); 8,17 (d, 2H, J=8,5Hz). |
| **40** | 1 : Préparation du 3-(4-((2-méthoxy-6-(3-(trifluorométhyl)phényl)pyridin-3-yl)méthylamino)phényl)propanoate d'éthyle (Ex. 5-11 et Ex. 6-15 avec Protocole SX et PA) | Rendement : 86%. Aspect : huile jaune . |
| | | RMN ¹H :1,24 (t, 3H, J=7,3Hz); 2,56 (t, 2H, J=7,3Hz); 2,85 (t, 2H, J=7,3Hz); 4,09-4,17 (m, 5H); 4,35 (s, 2H); 6,59 (d, 2H, J=8,5Hz); 7,02 (d, 2H, J=8,5Hz); 7,33 (d, 1H, J=7,6Hz); 7,54-7,66 (m, 3H); 8,20 (d, 1H, J=7,6Hz); 8,30 (s, 1H). |
| | 2 : Obtention du acide 3-(4-(((2-méthoxy-6-(3-(trifluorométhyl)phényl)pyridin-3-yl)méthyl)amino)-phényl)propanoïque (Protocole SU et PA) | Rendement : 53%. Aspect : solide beige. |
| | | RMN ¹H :2,63 (t, 2H, J=7,6Hz); 2,86 (t, 2H, J=7,6Hz); 4,12 (s, 3H); 4,34 (s, 2H); 6,59 (d, 2H, J=8,5Hz); 7,03 (d, 2H, J=8,5Hz); 7,34 (d, 1H, J=7,6Hz); 7,54-7,66 (m, 3H); 8,20 (d, 1H, J=7,6Hz); 8,30 (s, 1H). |
| **41** | | Rendement : 72%. Aspect : huile incolore. |
| | 1 : Préparation du 3-(4-((2-méthoxy-5-phénylpyridin-3-yl)méthylamino)-phényl)propanoate d'éthyle (Ex. 5-4 et Ex. 6-15 avec Protocole SX et PA) | RMN ¹H (300MHz, CDCl₃, d en ppm) : 1,22 (t, 3H, J=7,3Hz); 2,56 (t, 2H, J=7,3Hz); 2,84 (t, 2H, J=7,3Hz); 4,05 (s, 3H); 4,11 (q, 2H, J=7,3Hz); 4,34 (s, 2H); 6,60 (d, 2H, J=8,5Hz); 7,02 (d, 2H, J=8,5Hz); 7,31-7,36 (m, 1H); 7,39-7,50 (m, 4H); 7,80 (d, 1H, J=2,4Hz); 8,29 (d, 1H, J=2,4Hz). |
| | 2: Obtention du acide 3-(4-(((2-méthoxy-5-phénylpyridin-3-yl)méthyl)amino)phényl)-propanoïque (Protocole SU et PA) | Rendement : 57%. Aspect : solide blanc |
| | | RMN ¹H : 2,39 (t, 2H, J=7,9Hz); 2,63 (t, 2H, J=7,3Hz); 3,97 (s, 3H); 4,22 (d, 2H, J=5,0Hz); 6,00 (m, 1H); 6,51 (d, 2H, J=8,5Hz); 6,91 (d, 2H, J=8,5Hz); 7,33 (m, 1H); 7,43 (m, 2H); 7,54 (d, 2H, J=7,3Hz); 7,86 (d, 1H, J=2,3Hz); 8,34 (d, 1H, J=2,3Hz). |
| **42** | 1 : Préparation du 3-(4-((2-méthoxy-6-phénylpyridin-3-yl)méthylamino)-phényl)-3-phénylpropanoate d'éthyle (Ex. 5-1 et Ex. 6-14 avec Protocole SX et PA) | Rendement : 75%. Aspect : huile incolore. |
| | | RMN ¹H :1,11 (t, 3H, J=7,0Hz); 3,00 (d, 2H, J=8,1Hz); 4,03 (q, 2H, J=7,0Hz); 4,10 (s, 3H); 4,31 (s, 2H); 4,45 (t, 1H, J=8,1Hz); 6,57 (d, 2H, J=8,6Hz); 7,05 (d, 2H, J=8,6Hz); 7,15-7,32 (m, 6H); 7,36-7,49 (m, 4H); 7,60 (d, 1H, J=7,6Hz); 8,04 (d, 1H, J=7.0Hz). |
| | 2 : Obtention du acide 3-(4-((2(-méthoxy-6-phénylpyridin-3-yl)méthyl)amino)phényl)-3-phényl-propanoïque (Protocole SU et PA) 3 : Protocole PC | Rendement : 36%. Aspect : solide blanc. |
| | | RMN ¹H: 2,88 (dd, 2H, J=7,9Hz J=9,4Hz); 4,03 (s, 3H); 4,17-4,25 (m, 3H); 6,05 (m, 1H); 6,46 (d, 2H, J=8,5Hz); 6,98 (d, 2H, J=8,5Hz); 7,08-7,16 (m, 1H); 7,22-7,26 (m, 4H); 7,36-7,50 (m, 4H); 7,61 (d, 1H, J=7,6Hz); 8,05 (d, 2H, J=7,OHz). |
| **43** | 1 : Préparation du 3-(2-méthoxy-4-((2-méthoxy-6-phénylpyridin-3-yl)méthyl-amino)phényl)propanoate d'éthyle (Ex. 5-1 et Ex. 6-22 avec Protocole SX et PA) | Rendement : 57%. Aspect : huile jaune. |
| | | RMN ¹H :1,24 (t, 3H, J=7,3Hz); 2,55 (m, 2H); 2,83 (m, 2H); 3,76 (s, 3H); 4,08-4,15 (m, 5H); 4,33 (s, 2H); 6,17-6,20 (m, 2H); 6,94 (d, 1H, J=7,6Hz); 7,32 (d, 1H, J=7,3Hz); 7,36-7,49 (m, 3H); 7,63 (d, 1H, J=7,6Hz); 8,03-8,06 (m, 2H). |
| | 2 : Obtention du acide 3-(2-méthoxy-4-(((2-méthoxy-6-phénylpyridin-3-yl)méthyl)amino)-phényl)-propanoïque (Protocole SU et PA) 3 : Protocole PC | Rendement : 44%. Aspect : solide jaunâtre. |
| | | RMN ¹H: 2,33 (m, 2H); 2,60 (m, 2H); 3,67 (s, 3H); 4,04 (s, 3H); 4,21 (d, 2H, J=5,0Hz); 6,00 (dd, 1H, J=1,9Hz J=8,1Hz); 6,05 (m, 1H); 6,26 (d, 1H, J=1,9Hz); 6,78 (d, 1H, J=8,1Hz); 7,37-7,54 (m, 4H); 7,66 (d, 1H, J=7,6Hz); 8,07 (d, 2H, J=7,3Hz); 11,97 (s(I), 1H). |
| **44** | 1 : Préparation du 3-(3-méthoxy-4-((2-méthoxy-6-phénylpyridin-3-yl)méthyl-amino)phényl)propanoate d'éthyle (Ex. 5-1 et Ex. 6-23 avec Protocole SX et PA) | Rendement : 51%. Aspect : huile jaune. |
| | | RMN ¹H :1,25 (t, 3H, J=7,1Hz); 2,58 (m, 2H); 2,87 (m, 2H); 3,88 (s, 3H); 4,11 (s, 3H); 4,12 (q, 2H, J=7,1 Hz); 4,35 (s, 2H); 6,48 (d, 1H, J=8,2Hz); 6,64-6,67 (m, 2H); 7,30 (d, 1H, J=7,6Hz); 7,35-7,48 (m, 3H); 7,60 (d, 1H, J=7,3Hz); 8,02-8,05 (m, 2H). |
| | 2 : Obtention du acide 3-(3-méthoxy-4-(((2-méthoxy-6-phénylpyridin-3-yl)méthyl)amino)-phényl)-propanoïque (Protocole SU et PA) 3 : Protocole PC | Rendement : 46%. Aspect : solide blanc. |
| | | RMN ¹H: 2,44 (t, 2H, J=7,9Hz); 2,67 (t, 2H, J=7,9Hz); 3,80 (s, 3H); 4,04 (s, 3H); 4,26 (m, 2H); 5,36 (m, 1H); 6,26 (d, 1H, J=8,1Hz); 6,53 (dd, 1H, J=1,4Hz J=8,1 Hz); 6,71 (d, 1H, J=1,4Hz); 7,36-7,50 (m, 4H); 7,56 (d, 1H, J=7,6Hz); 8,06 (d, 2H, J=7,7Hz); 12,01 (s, 1H). |
| **45** | 1 : Préparation du 3-(4-((2-méthoxy-6-phénylpyridin-3-yl)méthylamino)-phényl)butanoate d'éthyle (Ex. 5-1 et Ex. 6-24 avec Protocole SX et PA) | Rendement : 56%. Aspect : huile jaune ; |
| | | RMN ¹H :1,19 (t, 3H, J=7,2Hz); 1,26 (d, 3H, J=7,0Hz); 2,43-2,60 (m, 2H); 3,14-3,22 (m, 1H); 4,08 (q, 2H, J=7,2Hz); 4,11 (s, 3H); 4,33 (s, 2H); 6,60 (d, 2H, J=8,5Hz); 7,03 (d, 2H, J=8,5Hz); 7,31 (d, 1H, J=7,3Hz); 7,36-7,49 (m, 3H); 7,62 (d, 1H, J=7,6Hz); 8,04 (d, 2H, J=8,5Hz). |
| | 2 : Obtention du acide 3-(4-(((2-méthoxy-6-phénylpyridin-3-yl)méthyl)amino)phényl)butanoïque (Protocole SU et PA) 3 : Protocole PC | Rendement : 52%; Aspect : solide blanc. |
| | | RMN ¹H: 1,12 (d, 3H, J=6,7Hz); 2,37 (m, 2H); 2,93-3,00 (m, 1H); 4,04 (s, 3H); 4,20 (d, 2H, J=4,4Hz); 6,00 (m, 1H); 6,48 (d, 2H, J=8,2Hz); 6,93 (d, 2H, J=8,2Hz); 7,37-7,53 (m, 4H); 7,63 (d, 1H, J=7,6Hz); 8,06 (d, 2H, J=7,3Hz); 11,93 (s, 1H). |
| **46** | 1 : Préparation du 3-(4-((2-méthoxy-5-(4-(trifluorométhyl)phényl)pyridin-3-yl)méthylamino)phényl)propanoate d'éthyle (Ex. 5-12 et Ex. 6-15 avec Protocole SX et PA) | Rendement : 74%. Aspect : huile jaune. RMN ¹H :1,22 (t, 3H, J=7,0Hz); 2,55 (m, 2H); 2,84 (m, 2H); 4,06 (s, 3H); 4,11 (q, 2H, J=7,0Hz); 4,36 (s, 2H); 6,62 (d, 2H, J=8,5Hz); 7,02 (d, 2H, J=8,5Hz); 7,58 (d, 2H, J=8,2Hz); 7,67 (d, 2H, J=8,2Hz); 7,81 (d, 1H, J=2,3Hz); 8,30 (d, 1H, J=2,3Hz). |
| | 2 : Obtention du acide 3-(4-(((2-méthoxy-5-(4-(trifluorométhyl) phényl)pyridin-3-yl)méthyl) amino) phényl)propanoïque (Protocole SU et PA) 3 : Protocole PC | Rendement : 44%. Aspect : solide blanc. |
| | | RMN ¹H: 2,39 (t, 2H, J=7,6Hz); 2,63 (t, 2H, J=7,6Hz); 3,99 (s, 3H); 4,23 (d, 2H, J=5,7Hz); 5,99 (t, 1H, J=5,7Hz); 6,51 (d, 2H, J=8,4Hz); 6,91 (d, 2H, J=8,4Hz); 7,75-7,82 (m, 4H); 7,95 (d, 1H, J=2,5Hz); 8,44 (d, 1H, J=2,5Hz); 12,00 (s, 1H). |
| **47** | 1 : Préparation du 3-(4-((2-méthoxy-5-(3-(trifluorométhyl)phényl)pyridin-3-yl)méthylamino)-phényl)propanoate d'éthyle (Ex. 5-13 et Ex. 6-15 avec Protocole SX et PA) | Rendement : 65%. Aspect : huile jaune. |
| | | RMN ¹H: 1,22 (t, 3H, J=7,0Hz); 2,55 (m, 2H); 2,84 (m, 2H); 4,06 (s, 3H); 4,11 (q, 2H, J=7,0Hz); 4,36 (s, 2H); 6,60 (d, 2H, J=8,5Hz); 7,02 (d, 2H, J=8,5Hz); 7,51-7,66 (m, 3H); 7,70 (s, 1H); 7,79 (d, 1H, J=2,5Hz); 8,29 (d, 1H, J=2,5Hz). |
| | 2 : Obtention du acide 3-(4-(((2-méthoxy-5-(3-(trifluorométhyl) phényl) pyridin-3-yl)méthyl)amino) -phényl) propanoïque (Protocole SU et PA) 3 : Protocole PC | Rendement : 52%. Aspect : solide blanc. |
| | | RMN ¹H : 2,39 (m, 2H); 2,63 (m, 2H); 3,98 (s, 3H); 4,23 (d, 2H, J=5,7Hz); 5,98 (t, 1H, J=5,7Hz); 6,52 (d, 2H, J=8,3Hz); 6,91 (d, 2H, J=8,3Hz); 7,67-7,69 (m, 2H); 7,85-7,89 (m, 2H); 7,96 (d, 1H, J=2,3Hz); 8,44 (d, 1H, J=2,3Hz); 11,98 (s, 1H). |
| **48** | 1 : Préparation du 3-(4-((2,6-diméthoxy-5-phénylpyridin-3-yl)méthylamino)-phényl)propanoate d'éthyle (Ex. 5-14 et Ex. 6-15 avec Protocole SX et PA) | Rendement : 48%. Aspect : huile incolore. |
| | | RMN ¹H :1,23 (t, 3H, J=7,0Hz); 2,56 (t, 2H, J=7,3Hz); 2,84 (t, 2H, J=7,3Hz); 3,97 (s, 3H); 4,00 (s, 3H); 4,12 (q, 2H, J=7,0Hz); 4,25 (s, 2H); 6,61 (d, 2H, J=8,5Hz); 7,01 (d, 2H, J=8,5Hz); 7,28-7,31 (m, 1H); 7,36-7,41 (m, 2H); 7,50 (m, 2H); 7,58 (s, 1H). |
| | 2 : Obtention du acide 3-(4-(((2,6-diméthoxy-5-phénylpyridin-3-yl)méthyl)amino)phényl)-propanoïque (Protocole SU et PA) 3 : Protocole PC | Rendement : 46%. Aspect : solide beige. RMN ¹H : 2,39 (t, 2H, J=7,6Hz); 2,62 (t, 2H, J=7,6Hz); 3,90 (s, 3H); 3,99 (s, 3H); 4,13 (d, 2H, J=5,7Hz); 5,87 (t, 1H, J=5,7Hz); 6,49 (d, 2H, J=8,2Hz); 6,89 (d, 2H, J=8,2Hz); 7,24-7,29 (m, 1H); 7,34-7,44 (m, 4H); 7,62 (s, 1H); 12,05 (s, 1H). |
| **49** | 1 : Préparation du 3-(4-((5-(4-chlorophényl)-2-méthoxypyridin-3-yl)méthyl-amino)phényl)propanoate d'éthyle (Ex. 5-15 et Ex. 6-15 avec Protocole SX et PA) | Rendement : 68%. Aspect : huile incolore. |
| | | RMN ¹H :1,22 (t, 3H, J=7,2Hz); 2,55 (m, 2H); 2,84 (m, 2H); 4,05 (s, 3H); 4,11 (q, 2H, J=7,2Hz); 4,34 (s, 2H); 6,58 (d, 2H, J=8,5Hz); 7,01 (d, 2H, J=8,5Hz); 7,39 (m, 4H); 7,75 (d, 1H, J=2,5Hz); 8,25 (d, 1H, J=2,5Hz). |
| | 2 : Obtention du acide 3-(4-(((5-(4-chlorophényl)-2-méthoxypyridin-3-yl)méthyl)amino)phényl)-propanoïque (Protocole SU et PC) | Rendement : 32%. Aspect : solide blanc. |
| | | RMN ¹H :2,62 (m, 2H); 2,85 (m, 2H); 4,05 (s, 3H); 4,33 (s, 2H); 6,59 (d, 2H, J=8,4Hz); 7,02 (d, 2H, J=8,4Hz); 7,38 (m, 4H); 7,75 (d, 1H, J=2,4Hz); 8,25 (d, 1H, J=2,4Hz). |
| **50** | 1 : Préparation du 3-(4-((2-méthoxy-5-(naphthalen-2-yl)pyridin-3-yl)méthyl-amino)phényl)propanoate d'éthyle (Ex. 5-16 et Ex. 6-15 avec Protocole SX et PA) | Rendement : 80%. Aspect : huile incolore. |
| | | RMN ¹H :1,20 (t, 3H, J=7,2Hz); 2,56 (m, 2H); 2,84 (m, 2H); 4,08 (s, 3H); 4,10 (q, 2H, J=7,2Hz); 4,38 (s, 2H); 6,64 (d, 2H, J=8,4Hz); 7,03 (d, 2H, J=8,4Hz); 7,45-7,54 (m, 2H); 7,62 (dd, 1H, J=1,8Hz J=8,5Hz); 7,84-7,93 (m, 5H); 8,41 (d, 1H, J=2,4Hz). |
| | 2 : Obtention du acide 3-(4-(((2-méthoxy-5-(naphthalèn-2-yl)pyridin-3-yl)méthyl) amino)phényl)-propanoïque (Protocole SU et PC) | Rendement : 53%. Aspect : solide blanc. RMN ¹H: 2,40 (m, 2H); 2,63 (m, 2H); 4,00 (s, 3H); 4,25 (m, 2H); 6,02 (m, 1H); 6,53 (d, 2H, J=8,5Hz); 6,92 (d, 2H, J=8,5Hz); 7,47-7,55 (m, 2H); 7,72 (dd, 1H, J=1,5Hz J=8,5Hz); 7,90-8,09 (m, 5H); 8,50 (d, 1H, J=2,3Hz); 11,99 (s, 1H). |
| **51** | 1 : Préparation du 3-(4-((2-éthoxy-6-phénylpyridin-3-yl) méthylamino)-phényl)propanoate d'éthyle (Ex. 5-17 et Ex. 6-15 avec Protocole SX et PA) | Rendement 49%. Aspect : huile incolore. |
| | | RMN ¹H: 1,24 (t, 3H, J=7,1Hz); 1,47 (t, 3H, J=7,0Hz); 2,56 (m, 2H); 2,84 (m, 2H); 4,12 (q, 2H, J=7,1Hz); 4,33 (s, 2H); 4,58 (q, 2H, J=7,0Hz); 6,60 (d, 2H, J=8,5Hz); 7,02 (d, 2H, J=8,5Hz); 7,27-7,30 (m, 1H); 7,35-7,48 (m, 3H); 7,60 (d, 1H, J=7,6Hz); 8,00-8,03 (m, 2H). |
| | 2 : Obtention du acide 3-(4-(((2-éthoxy-6-phénylpyridin-3-yl)méthyl)amino)phényl)propanoïq ue (Protocole SU et PA) | Rendement : 77%. Aspect : solide blanc. RMN ¹H: 1,41 (t, 3H, J=7,0Hz); 2,41 (m, 2H); 2,64 (m, 2H); 4,20 (s, 2H); 4,52 (q, 2H, J=7,0Hz); 5,99 (s, 1H); 6,48 (d, 2H, J=8,2Hz); 6,91 (d, 2H, J=8,2Hz); 7,36-7,50 (m, 4H); 7,62 (d, 1H, J=7,6Hz); 8,04 (d, 2H, J=7,3Hz); 12,01 (s, 1H). |
| | 3 : Protocole PC | |
| **54** | 1 : Préparation du 3-(4-((2-isopropoxy-6-phénylpyridin-3-yl)méthylamino)-phényl) propanoate d'éthyle (Ex. 5-18 et Ex. 6-15 avec Protocole SX et PA) | Rendement : 65% . Aspect : huile incolore . |
| | | RMN ¹H :1,24 (t, 3H, J=7,1Hz); 1,44 (s, 3H); 1,46 (s, 3H); 2,57 (m, 2H); 2,84 (m, 2H); 4,13 (q, 2H, J=7,1Hz); 4,31 (s, 2H); 5,55-5,63 (m, 1H); 6,59 (d, 2H, J=8,5Hz); 7,02 (d, 2H, J=8,5Hz); 7,27 (d, 1H, J=7,6Hz); 7,35-7,47 (m, 3H); 7,59 (d, 1H, J=7,6Hz); 8,01 (m, 2H). |
| | 2 : Obtention du acide 3-(4-(((2-isopropyloxy-6-phénylpyridin-3-yl)méthyl)amino)phényl)-propanoïque (Protocole SU et PA) | Rendement : 75%. Aspect : solide blanc. RMN ¹H: 1,39 (s, 3H); 1,41 (s, 3H); 2,41 (m, 2H); 2,64 (m, 2H); 4,17 (s, 2H); 5,44-5,52 (m, 1H); 5,97 (m, 1H); 6,48 (d, 2H, J=8,4Hz); 6,92 (d, 2H, J=8,4Hz); 7,35-7,47 (m, 4H); 7,62 (d, 1H, J=7,6Hz); 8,02 (d, 2H, J=7,0Hz); 12,01 (s, 1H). |
| | 3 : Protocole PC | |

La synthèse des composés selon l'invention de Figures 7b et 7c nécessite 2 étapes et elle est résumé dans le Tableau 7-2.

**Tableau 7-2**

| **Cpd** | **No. et type d'Etape (Ex. ; Protocole)** | **Détails** |
|---|---|---|
| **13** | 1 : Préparation du 2-(3-((2-méthoxy-6-phénylpyridin-3-yl)méthylamino)-phénoxy)-2-méthyl-propanoate de tertiobutyle (Ex. 5-1 et Ex. 6-12 avec Protocole SX et PA) | Rendement : 49%. Aspect : huile jaune. |
| | | RMN ¹H :1,45 (s, 9H); 1,55 (s, 6H); 4,11 (s, 3H); 4,18 (s, 1H); 4,31 (s, 2H); 6,20-6,31 (m, 3H); 7,01 (m, 1H); 7,31 (d, 1H, J=7,6Hz); 7,37-7,49 (m, 3H); 7,61 (d, 1H, J=7,6Hz); 8,05 (d, 2H, J=8,8Hz). |
| | 2: Obtention du acide 2-(3-(((2-méthoxy-6-phénylpyridin-3-yl)méthyl)amino)phénoxy)-2-méthyl-propanoïque (Protocole SU et PB) | Rendement : 33%. Aspect : solide blanc. |
| | | RMN ¹H : 1,42 (s, 6H); 4,17 (d, 2H, J=5,5Hz); 5,98-6,02 (m, 2H); 6,16-6,20 (m, 2H); 6,90 (t, 1H, J=7,9Hz); 7,37-7,53 (m, 4H); 7,62 (d, 1H, J=7,6Hz); 8,07 (d, 2H, J=7,3Hz). |
| **14** | 1 : Préparation du 2-(3-((2-méthoxy-6-phénylpyridin-3-yl)méthylamino)-phénoxy)éthanoate de tertiobutyle (Ex. 5-1 et Ex. 6-13 avec Protocole SX et PA) | Rendement : 80%. Aspect : huile jaune. |
| | | RMN ¹H :1,49 (s, 9H); 4,12 (s, 3H); 4,33 (s, 2H); 4,47 (s, 2H); 6,22-6,33 (m, 3H); 7,08 (t, 1H, J=7,6Hz); 7,31 (d, 1H, J=7,6Hz); 7,37-7,49 (m, 3H); 7,61 (d, 1H, J=7,6Hz); 8,05 (d, 2H, J=7.0Hz). |
| | 2: Obtention du acide 2-(3-(((2-méthoxy-6-phénylpyridin-3-yl)méthyl)amino)phénoxy) éthanoïque (Protocole SU et PB) | Rendement : 60%. Aspect : solide blanc. |
| | | RMN ¹H :4,12 (s, 3H); 4,34 (s, 2H); 4,61 (s, 2H); 6,24-6,37 (m, 3H); 7,09 (m, 1H); 7,31 (d, 1H, J=7,6Hz); 7,36-7,48 (m, 3H); 7,60 (d, 1H, J=7,6Hz); 8,04 (d, 2H, J=6,7Hz). |
| **34** | 1 : Préparation du 3-(4-(1-(2-méthoxy-6-phénylpyridin-3-yl)propylamino)-phényl)propanoate d'éthyle (Ex. 5-8 et Ex. 6-15 avec Protocole SY et PA) | Rendement : 14%. Aspect : huile jaune |
| | 2 : Obtention du acide 3-(4-(1-((2-méthoxy-6-phénylpyridin-3-yl)propyl)amino)phényl)-propanoïque (Protocole SU et PB) | Rendement : 58%. Aspect : solide blanc. |
| | | RMN ¹H : 0,94 (t, 3H, J=7,3Hz); 1,65-1,78 (m, 2H); 2,35 (t, 2H, J=7,3Hz); 2,58 (t, 2H, J=7,3Hz); 4,06 (s, 3H); 4,46 (m, 1H); 5,96 (d, 1H, J=7,9Hz); 6,39 (d, 2H, J=8,5Hz); 6,83 (d, 2H, J=8,5Hz); 7,36-7,51 (m, 4H); 7,66 (d, 1H, J=7,9Hz); 8,06 (d, 2H, J=7.0Hz). |

La synthèse des composés selon l'invention de Figures 7d nécessite 2 ou 3 étapes et elle est résumée dans le Tableau 7-3.

**Tableau 7-3**

| **Cpd** | **No. et type d'Etape (Ex. ; Protocole)** | **Détails** |
|---|---|---|
| **2** | 1 : Préparation du 2-(4-((2-méthoxy-6-phénylpyridin-3-yl)méthoxy)phénoxy)-éthanoate de tertiobutyle (Ex. 4-1 et Ex. 6-2 avec Protocole SV et PA) | Rendement : 54%. Aspect : solide blanc. |
| | | RMN ¹H :1,47 (s, 9H); 4,11 (s, 3H); 4,59 (s, 2H); 5,02 (s, 2H); 6,88 (d, 2H, J=9,1Hz); 6,95 (d, 2H, J=9,1Hz); 7,34-7,49 (m, 4H); 7,76 (d, 1H, J=7,6Hz); 8,02 (m, 2H). |
| | 2 : Obtention du acide 2-(4-((2-méthoxy-6-phénylpyridin-3-yl)méthoxy)phénoxy)éthanoïque (Protocole SW et PE) | Rendement : 83%. Aspect : solide blanc. |
| | | RMN ¹H: 4,03 (s, 3H); 4,60 (s, 2H); 5,03 (s, 2H); 6,84 (d, 2H, J=9,1Hz); 6,96 (d, 2H, J=9,1Hz); 7,40-7,55 (m, 3H); 7,58 (d, 1H, J=7,6Hz); 7,84 (d, 1H, J=7,6Hz); 8,10 (d, 2H, J=7,0Hz); 12,83 (s (large), 1H). |
| **6** | 1 : Préparation du 2-(4-((2-tertiobutoxy-6-phénylpyridin-3-yl)méthoxy)phénoxy)-2-méthyl-propanoate d'éthyle (Ex. 4-2 et Ex. 6-1 avec Protocole SV et PA) | Rendement : 33%. Aspect : huile jaune. |
| | | RMN ¹H :1,29 (t, 3H, J=7,2Hz); 1,57 (s, 6H); 1,71 (s, 9H); 4,26 (q, 2H, J=7,2Hz); 5,02 (s, 2H); 6,85-6,92 (m, 4H); 7,34-7,50 (m, 4H); 7,76 (d, 1H, J=7,6Hz); 8,03 (d, 2H, J=7,3Hz). |
| | 2 : Obtention du acide 2-(4-((2-tertio-butyloxy-6-phénylpyridin-3-yl)méthoxy)phénoxy)-2-méthyl-propanoïque (Protocole SU et PD) | Rendement : 67%. Aspect : solide blanc. |
| | | RMN ¹H: 1,44 (s, 6H); 1,63 (s, 9H); 4,97 (s, 2H); 6,83 (d, 2H, J=9,1Hz); 6,93 (d, 2H, J=9,1Hz); 7,42-7,57 (m, 4H); 7,80 (d, 1H, J=7,6Hz); 8,03 (d, 2H, J=7,3Hz); 12,94 (s, 1H). |
| **7** | 1 : Préparation du 2-(4-((2-tertiobutoxy-6-phénylpyridin-3-yl)méthoxy)phénoxy)éthanoate d'éthyle (Ex. 4-2 et Ex. 6-4 avec Protocole SV et PA) | Rendement : 80%. Aspect : solide blanc. |
| | | RMN ¹H :1,32 (t, 3H, J=7,2Hz); 1,71 (s, 9H); 4,29 (q, 2H, J=7,2Hz); 4,59 (s, 2H); 5,02 (s, 2H); 6,88 (d, 2H, J=9,2Hz); 6,95 (d, 2H, J=9,2Hz); 7,34-7,49 (m, 4H); 7,76 (d, 1H, J=7,6Hz); 8,02 (d, 2H, J=7,3Hz). |
| | 2 : Obtention du acide 2-(4-(((2-tertio-butyloxy-6-phénylpyridin-3-yl)méthoxy)phénoxy) éthanoïque (Protocole SU et PD) | Rendement : 33%. Aspect : solide blanc. |
| | | RMN ¹H: 1,64 (s, 9H); 4,59 (s, 2H); 4,97 (s, 2H); 6,85 (d, 2H, J=9,1Hz); 6,94 (d, 2H, J=9,1Hz); 7,39-7,56 (m, 4H); 7,79 (d, 1H, J=7,6Hz); 8,03 (d, 2H, J=7,3Hz); 12,94 (s, 1H). |
| **15** | 1 : Préparation du 2-(4-((2-hexyloxy-6-phénylpyridin-3-yl)méthoxy)-phénoxy)éthanoate de tertiobutyle (Ex. 4-3 et Ex. 6-2 avec Protocole SV et PA) | Rendement : 39%. Aspect : huile jaune. |
| | | RMN ¹H :0,91 (t, 3H, J=7,0Hz); 1,26-1,55 (m, 6H); 1,49 (s, 9H); 1,84 (m, 2H); 4,47 (s, 2H); 4,51 (t, 2H, J=6,5Hz); 5,06 (s, 2H); 6,85 (d, 2H, J=9,4Hz); 6,94 (d, 2H, J=9,4Hz); 7,34-7,48 (m, 4H); 7,77 (d, 1H, J=7,6Hz); 8,03 (d, 2H, J=7.0Hz). |
| | 2 : Obtention du acide 2-(4-((2-hexyloxy-6-phénylpyridin-3-yl)méthoxy)phénoxy)éthanoïque (Protocole SW et PA) 3 : Protocole PC | Rendement : 31%. Aspect : solide blanc. |
| | | RMN ¹H :0,91 (t, 3H, J=6,7Hz); 1,34-1,49 (m, 6H); 1,79-1,88 (m, 2H); 4,51 (t, 2H, J=6,7Hz); 4,63 (s, 2H); 5,07 (s, 2H); 6,89 (d, 2H, J=9,2Hz); 6,95 (d, 2H, J=9,2Hz); 7,34-7,48 (m, 4H); 7,76 (d, 1H, J=7,6Hz); 8,03 (d, 2H, J=7,2Hz). |
| **17** | 1 : Préparation du 2-(4-((2-hexyloxy-6-phénylpyridin-3-yl)méthoxy)-phénoxy)-2-méthyl-propanoate de tertiobutyle (Ex. 4-3 et Ex. 6-5 avec Protocole SV et PA) | Rendement : 37% ; Aspect : huile jaune. RMN ¹H :0,91 (t, 3H, J=7,0Hz); 1,30-1,46 (m, 6H); 1,46 (s, 9H); 1,52 (s, 6H); 1,83 (m, 2H); 4,51 (t, 2H, J=6,4Hz); 5,06 (s, 2H); 6,87 (m, 4H); 7,35-7,49 (m, 4H); 7,78 (d, 1H, J=7,6Hz); 8,03 (d, 2H, J=7,9Hz). |
| | 2: Obtention du acide 2-(4-((2-hexyloxy-6-phénylpyridin-3-yl)méthoxy)phénoxy)-2-méthyl-propanoïque (Protocole SW et PB) | Rendement : 26% ; Aspect : solide blanc. |
| | | RMN ¹H :0,91 (t, 3H, J=7,0Hz); 1,26-1,56 (m, 12H); 1,79-1,89 (m, 2H); 4,52 (t, 2H, J=6,7Hz); 5,08 (s, 2H); 6,94 (m, 4H); 7,35-7,48 (m, 4H); 7,78 (d, 1H, J=7,6Hz); 8,04 (d, 2H, J=8,5Hz). |
| **18** | 1 : Préparation du 2-(4-((2-cyclohexyloxy-6-phénylpyridin-3-yl)méthoxy)-phénoxy)éthanoate de tertiobutyle (Ex. 4-4 et Ex. 6-2 avec Protocole SV et PA) | Rendement : 39%. Aspect : huile jaune . RMN ¹H :1,50 (s, 9H); 1,50-1,72 (m, 6H); 1,80-1,83 (m, 2H); 2,03-2,04 (m, 2H); 4,48 (s, 2H); 5,07 (s, 2H); 5,33 (m, 1H); 6,88 (d, 2H, J=9,2Hz); 6,95 (d, 2H, J=9,2Hz); 7,34 (d, 1H, J=7,6Hz); 7,36-7,49 (d, 3H); 7,78 (d, 1H, J=7,6Hz); 8,02 (d, 2H, J=8,5Hz). |
| | 2 : Obtention du acide 2-(4-((2-cyclohexyloxy-6-phénylpyridin-3-yl)méthoxy)phénoxy)éthanoïque (Protocole SW et PB) | Rendement : 16% . Aspect : solide blanc. RMN ¹H :1,26-2,03 (m, 10H); 4,64 (s, 2H); 5,07 (s, 2H); 5,32-5,36 (m, 1H); 6,88-6,99 (m, 4H); 7,32-7,48 (m, 4H); 7,76 (d, 1H, J=7,6Hz); 8,01 (d, 2H, J=7.0Hz). |
| **20** | 1 : Préparation du 2-(4-((6-phényl-2-(pipéridin-1-yl)pyridin-3-yl)méthoxy)-phénoxy)éthanoate d'éthyle (Ex. 4-5 et Ex. 6-4 avec Protocole SV et PA) | Rendement : 60%. Aspect : solide blanc. |
| | | RMN ¹H :0,84 (m, 3H); 1,34-1,76 (m, 6H); 3,27 (m, 4H); 4,27 (q, 2H, J=7,0Hz); 4,57 (s, 2H); 5,07 (s, 2H); 6,85-6,94 (m, 4H); 7,39-7,48 (m, 4H); 7,82-7,86 (m, 1H); 8,05 (d, 2H, J=6,7Hz). |
| | 2: Obtention du acide 2-(4-((6-phényl-2-(pipéridin-1-yl)pyridin-3-yl)méthoxy)phénoxy)-éthanoïque (Protocole SU et PB) | Rendement : 21%. Aspect : solide blanc. RMN ¹H : 1,59-1,67 (m, 6H); 3,17 (m, 4H); 4,49 (s, 2H); 5,00 (s, 2H); 6,82 (d, 2H, J=9,2Hz); 6,92 (d, 2H, J=9,2Hz); 7,37-7,49 (m, 3H); 7,57 (d, 1H, J=7,7Hz); 7,82 (d, 1H, J=7,7Hz); 8,07 (d, 2H, J=7.0Hz). |
| **21** | 1 : Préparation du 2-(4-((2-méthoxy-6-(4-(trifluorométhyl)phényl)pyridin-3-yl)méthoxy)-phénoxy)-2-méthyl propanoate de tertiobutyle (Ex. 4-6 et Ex. 6-5 avec Protocole SV et PA) | Rendement : 47%; Aspect : solide blanc. |
| | | RMN ¹H :1,47 (s, 9H); 1,53 (s, 6H); 4,09 (s, 3H); 5,07 (s, 2H); 6,85-6,92 (m, 4H); 7,43 (d, 1H, J=7,6Hz); 7,72 (d, 2H, J=8,2Hz); 7,84 (d, 1H, J=7,6Hz); 8,17 (d, 2H, J=8,2Hz). |
| | 2 : Obtention du acide 2-(4-((2-méthoxy-6-(4-(trifluorométhyl) phényl) pyridin-3-yl)méthoxy)-phénoxy)-2-méthylpropanoïque (Protocole SW et PA) | Rendement : 34%. Aspect : solide blanc. RMN ¹H :1,56 (s, 6H); 4,12 (s, 3H); 5,19 (s, 2H); 6,95 (s, 4H); 7,43 (d, 1H, J=7,6Hz); 7,72 (d, 2H, J=8,2Hz); 7,84 (d, 1H, J=7,6Hz); 8,17 (d, 2H, J=8,2Hz). |
| | 3 : Protocole PC | |
| **24** | 1 : Préparation du 2-(4-((2-méthoxy-6-(4-(trifluorométhyl)phényl)pyridin-3-yl)méthoxy)phénoxy)éthanoate de tertiobutyle (Ex. 4-6 et Ex. 6-2 avec Protocole SV et PA) | Rendement : 53%. Aspect : solide blanc. |
| | | RMN ¹H :1,50 (s, 9H); 4,11 (s, 3H); 4,48 (s, 2H); 5,07 (s, 2H); 6,58-6,96 (m, 4H); 7,42 (d, 1H, J=7,6Hz); 7,71 (d, 2H, J=8,2Hz); 7,83 (d, 1H, J=7,6Hz); 8,16 (d, 2H, J=8,2Hz). |
| | 2: Obtention du acide 2-(4-((2-méthoxy-6-(4-(trifluorométhyl)phényl)pyridin-3-yl)méthoxy)-phénoxy)éthanoïque (Protocole SW et PA) | Rendement : 12%. Aspect : solide blanc. |
| | | RMN ¹H :4,12 (s, 3H); 4,64 (s, 2H); 5,09 (s, 2H); 6,92-6,96 (m, 4H); 7,42 (d, 1H, J=7,6Hz); 7,71 (d, 2H, J=8,2Hz); 7,82 (d, 1H, J=7,6Hz); 8,17 (d, 2H, J=8,2Hz). |
| | 3 : Protocole PC | |
| **25** | 1 : Préparation du 2-(4-((2-phénylthio-6-(phényl)pyridin-3-yl)méthoxy)-phénoxy)éthanoate d'éthyle (Ex. 4-7 et Ex. 6-4 avec Protocole SV et PA) | Rendement : 27%. Aspect : solide blanc. |
| | | RMN ¹H :1,31 (t, 3H, J=7,2Hz); 4,28 (q, 2H, J=7,2Hz); 4,59 (s, 2H); 5,14 (s, 2H); 6,89 (d, 2H, J=9,4Hz); 6,96 (d, 2H, J=9,4Hz); 7,32-7,35 (m, 3H); 7,40-7,45 (m, 3H); 7,56 (d, 1H, J=7,9Hz); 7,60-7,63 (m, 2H); 7,75-7,81 (m, 3H). |
| | 2: Obtention du acide 2-(4-((2-phénylthio-6-(phényl)pyridin-3-yl)méthoxy)phénoxy)éthanoïque (Protocole SU et PD) | Rendement : 71%; Aspect : solide blanc |
| | | RMN ¹H :4,65 (s, 2H); 5,15 (s, 2H); 6,91 (d, 2H, J=9,2Hz); 6,98 (d, 2H, J=9,2Hz); 7,33-7,35 (m, 3H); 7,40-7,45 (m, 3H); 7,56 (d, 1H, J=8,2Hz); 7,59-7,63 (m, 2H); 7,74-7,81 (m, 3H). |
| **52** | 1 : Préparation du 3-(4-((2-méthoxy-5-phénylpyridin-3-yl)méthoxy)phényl)-hex-4-ynoate d'éthyle (Ex. 4-8 et Ex. 6-25 avec Protocole SV et PA) | Rendement : 18% ; Aspect : huile jaune. |
| | | RMN ¹H :1,22 (t, 3H, J=7,3Hz); 1,84 (d, 3H, J=2,3Hz); 2,66 (dd, 1H, J=7,0Hz, J=14,9Hz); 2,76 (dd, 1H, J=8,2Hz, J=14,9Hz); 4,05-4,18 (m, 6H); 5,10 (s, 2H); 6,96 (d, 2H, J=8,8Hz); 7,30-7,39 (m, 3H); 7,42-7,47 (m, 2H); 7,53-7,56 (m, 2H); 7,98 (d, 1H, J=2,3Hz); 7,34 (d, 1H, J=2,3Hz). |
| | 2: Obtention du acide 3-(4-((2-méthoxy-5-phénylpyridin-3-yl)méthoxy) ph ényl)hex-4-ynoïque (Protocole SU et PB) | Rendement : 26%. Aspect : solide blanc. |
| | | RMN ¹H (300MHz, DMSO d₆, en ppm) : 1,77 (s, 3H); 2,59 (d, 2H, J=7,6Hz); 3,96 (m, 4H); 5,07 (s, 2H); 6,99 (d, 2H, J=8,5Hz); 7,29 (d, 2H, J=8,5Hz); 7,34-7,39 (m, 1H); 7,47 (m, 2H); 7,65 (d, 2H, J=7,6Hz); 8,08 (d, 1H, J=1,8Hz); 8,47 (d, 1H, J=1,8Hz); 12,24 (s, 1H). |
| **53** | 1 : Préparation du 3-(4-((2-méthoxy-6-phénylpyridin-3-yl)méthoxy)phényl)-hex-4-ynoate d'éthyle (Ex. 4-1 et Ex. 6-25 avec Protocole SV et PA) | Rendement : 36% . Aspect : huile incolore. |
| | | RMN ¹H :1,23 (t, 3H, J=7,0Hz); 1,84 (d, 3H, J=2,3Hz); 2,66 (dd, 1H, J=7,0Hz J=14,9Hz); 2,76 (dd, 1H, J=8,2Hz, J=14,9Hz); 4,07-4,18 (m, 6H); 5,10 (s, 2H); 6,95 (d, 2H, J=8,5Hz); 7,31 (d, 2H, J=8,5Hz); 7,37-7,50 (m, 4H); 7,78 (d, 1H, J=7,6Hz); 8,04-8,08 (m, 2H). |
| | 2: Obtention du acide 3-(4-((2-méthoxy-6-phénylpyridin-3-yl)méthoxy)phényl)hex-4-ynoïque (Protocole SU et PA) 3 : Protocole PC | Rendement : 42%. Aspect : solide blanc. |
| | | RMN ¹H: 2,39 (d, 3H, J=2,3Hz); 3,21 (d, 2H, J=7,6Hz); 4,54-4,59 (m, 1H); 4,65 (s, 3H); 5,68 (s, 2H); 7,58 (d, 2H, J=8,8Hz); 7,90 (d, 2H, J=8,8Hz); 8,01-8,13 (m, 3H); 8,21 (d, 1H, J=7,6Hz); 8,46 (d, 1H, J=7,6Hz); 8,71-8,74 (m, 2H); 12,88 (s, 1H). |

La synthèse des composés selon l'invention de Figures 7e et 7f nécessite 1 ou 2 étapes et elle est résumé dans le Tableau 7-4.

**Tableau 7-4**

| **Cpd** | **No. et type d'Etape (Ex. ; Protocole)** | **Détails** |
|---|---|---|
| **1** | 1 : Préparation du 2-(4-((2-méthoxy-6-phénylpyridin-3-yl)méthoxy)phénoxy)-2-méthyl-propanoate d'éthyle (Ex. 4-15 et Ex. 6-1 avec Protocole SQ et PA) | Rendement : 47%. Aspect : huile incolore. |
| | | RMN ¹H :1,30 (t, 3H, J=7,0Hz); 1,57 (s, 6H); 4,12 (s, 3H); 4,26 (q, 2H, J=7,0Hz); 5,07 (s, 2H); 6,85-6,92 (m, 4H); 7,38-7,50 (m, 4H); 7,80 (d, 1H, J=7,6Hz); 8,07 (d, 2H, J=7,3Hz). |
| | 2: Obtention du acide 2-(4-((2-méthoxy-6-phénylpyridin-3-yl)méthoxy)phénoxy)-2-méthyl-propanoïque (Protocole SU et PA) | Rendement : 20%. Aspect : solide blanc. |
| | | RMN ¹H :1,57 (s, 6H); 4,12 (s, 3H); 5,09 (s, 2H); 6,94 (s, 4H); 7,38-7,50 (m, 4H); 7,79 (d, 1H, J=7,6Hz); 8,06 (d, 2H, J=7,3Hz). |
| **3** | 1 : Préparation du 2-(4-((2-méthoxy-6-phénylpyridin-3-yl)méthoxy)phénoxy)-propanoate d'éthyle (Ex. 4-15 et Ex. 6-3 avec Protocole SQ et PA) | Rendement : quantitatif. Aspect : solide blanc. |
| | | RMN ¹H :1,27 (t, 3H, J=7,0Hz); 1,62 (m, 3H); 4,11 (s, 3H); 4,23 (q, 2H, J=7,0Hz); 4,64-4,71 (m, 1H); 5,06 (s, 2H); 6,85 (d, 2H, J=9,3Hz); 6,93 (d, 2H, J=9,3Hz); 7,38-7,50 (m, 4H); 7,79 (d, 1H, J=7,6Hz); 8,06 (d, 2H, J=7,3Hz). |
| | 2: Obtention du acide 2-(4-((2-méthoxy-6-phénylpyridin-3-yl)méthoxy)phénoxy)propanoïque (Protocole SU et PD) | Rendement : 29% . Aspect : solide blanc. |
| | | RMN ¹H: 1,35 (d, 3H, J=6,4Hz); 4,02 (s, 3H); 4,24 (m, 1H); 4,99 (s, 2H); 6,73 (d, 2H, J=8,8Hz); 6,87 (d, 2H, J=8,8Hz); 7,40-7,51 (m, 3H); 7,59 (d, 1H, J=7,6Hz); 7,82 (d, 1H, J=7,6Hz); 8,10 (d, 2H, J=7,6Hz). |
| **5** | 1 : Préparation du 2-(4-((2-méthoxy-6-phénylpyridin-3-yl)méthylamino)-phénoxy)propanoate d'éthyle, (Ex. 4-16 et Ex. 6-7 avec Protocole SQ et PA) | Rendement : 20%. Aspect : huile jaune. |
| | | RMN ¹H :1,27 (t, 3H, J=7,1Hz); 1,59 (d, 3H, J=6,8Hz); 4,11 (s, 3H); 4,22 (q, 2H, J=7,1Hz); 4,30 (s, 2H); 4,62 (q, 1H, J=6,8Hz); 6,61 (d, 2H, J=9,0Hz); 6,79 (d, 2H, J=9,0Hz); 7,31 (d, 1H, J=7,6Hz); 7,37-7,49 (m, 3H); 7,61 (d, 1H, J=7,6Hz); 8,05 (d, 2H, J=7,3Hz). |
| | 2: Obtention du acide 2-(4-(((2-méthoxy-6-phénylpyridin-3yl)méthyl)amino)phénoxy) propanoïque (Protocole SU et PB) | Rendement : 52%. Aspect : solide jaune. |
| | | RMN ¹H: 1,40 (d, 3H, J=6,7Hz); 3,36 (m, 1H); 4,03 (s, 3H); 4,17 (s, 2H); 4,53 (q, 1H, J=6,7Hz); 6,48 (d, 2H, J=8,9Hz); 6,65 (d, 2H, J=8,9Hz); 7,37-7,53 (m, 4H); 7,63 (d, 1H, J=7,6Hz); 8,07 (d, 2H, J=7,3Hz). |
| **33** | 1 : Préparation du 3-(4-(((2-méthoxy-6-phénylpyridin-3-yl)méthyl)(méthyl)-amino)phényl)propanoate d'éthyle) (Cpd 19 avec Protocole SQ et PA) | Rendement : 39%. Aspect : huile jaune. |
| | | RMN ¹H :1,24 (t, 3H, J=7,3Hz); 2,58 (t, 2H, J=7,3Hz); 2,87 (t, 2H, J=7,3Hz); 3,08 (s, 3H); 4,09-4,18 (m, 5H); 4,49 (s, 2H); 6,71 (m, 1H); 7,08 (d, 2H, J=8,5Hz); 7,29-7,53 (m, 6H); 8,03 (d, 2H, J=7.0Hz). |
| | 2: Obtention du acide 3-(4-(((2-méthoxy-6-phénylpyridin-3-yl)méthyl)(méthyl)amino)phényl)-propanoïque (Protocole SU et PA) | Rendement : 37%. Aspect : solide blanc |
| | | RMN ¹H : 2,43 (t, 2H, J=7,3Hz); 2,67 (t, 2H, J=7,3Hz); 3,02 (s, 3H); 4,04 (s, 3H); 4,46 (s, 2H); 6,59 (d, 2H, J=8,6Hz); 8,05 (d, 2H, J=8,6Hz); 7,32-7,49 (m, 5H); 8,06 (d, 2H, J=7,0Hz). |
| **37** | Obtention du acide 3-(4-((2-méthoxy-6-phénylpyridin-3-yl)méthylthio)phényl)propanoïque (Ex. 4-16 et d'acide 3-(4-mercaptophényl)propanoïque avec Protocole SQ et PB) | Rendement : 9%. Aspect : solide blanc. |
| | | RMN ¹H : 2,41 (t, 2H, J=7,6Hz); 2,64 (t, 2H, J=7,6Hz); 4,03 (s, 3H); 4,21 (s, 2H); 6,50 (d, 2H, J=8,3Hz); 6,92 (d, 2H, J=8,3Hz); 7,36-7,53 (m, 4H); 7,63 (d, 1H, J=7,6Hz); 8,07 (d, 2H, J=7.0Hz). |

### Exemple 8 : Propriétés activatrices de PPAR des composés selon l'invention

### Principe

L'activation des PPARs est évaluée *in vitro* sur une lignée de fibroblastes de rein de singe (COS-7) par la mesure de l'activité transcriptionnelle de chimères constituées du domaine de liaison à l'ADN du facteur de transcription Gal4 de la levure et du domaine de liaison au ligand des différents PPAR d'origine humaine (hPPAR). Les composés sont testés à des doses comprises entre 0.01 et 100 µM sur les chimères Gal4-PPARα, γ, δ et l'EC₅₀ est déterminée.

### Protocole

### a) Culture des cellules

Les cellules COS-7 proviennent de l'ATCC et sont cultivées dans du milieu DMEM supplémenté de 10% (vol/vol) de sérum de veau foetal, 1% de pénicilline/streptomycine (Biochrom, AG), 1% d'acides aminés (Gibco) et 1% de pyruvate de sodium (Gibco). Les cellules sont incubées à 37°C dans une atmosphère humide contenant 5% de CO₂.

### b) Description des plasmides utilisés en transfection

Les plasmides Gal4(RE)_TkpGL3, pGal4-hPPARα, pGal4-hPPARγ, pGal4-hPPARδ et pGal4-φ ont été décrits dans la littérature (Raspe E *et al.,* 1999). Les constructions pGal4-hPPARα, pGal4-hPPARγ et pGal4-hPPARδ ont été obtenues par clonage dans le vecteur pGal4-φ de fragments d'ADN amplifiés par PCR correspondants aux domaines DEF (éléments structuraux du promoteur des PPARs : D= hinge, EF= domaine de fixation du ligand et site de fixation de l'AF2)des récepteurs nucléaires PPARα, PPARγ et PPARδ humains.

### c) Transfection

Les cellules COS-7 adhérentes sont transfectées avec 40 µg d'ADN par flask de 225 cm², avec un ratio pGal4-hPPAR / Gal4(RE)_TkpGL3 de 1/10, en présence de 10% de sérum de veau foetal. Les cellules sont ensuite détachées et ensemencées en absence de sérum dans des plaques de 384 puits (2x10⁴ cellules/puits) puis incubées pendant 4 heures à 37°C. Les composés sont ensuite dilués dans une plaque 96 puits puis transférés dans la plaque 384 puits. L'activation avec les composés à tester s'effectue pendant 24 heures supplémentaires à 37°C en présence de 1% de sérum synthétique Ultroser™ (Biosepra). Ces 2 dernières étapes sont automatisées à l'aide d'une station Genesis Freedom 200™ (Tecan). A l'issue de l'expérience, les cellules sont lysées et l'activité luciférase est déterminée à l'aide du Steady-Lite™ HTS (Perkin Elmer) selon les recommandations du fournisseur.

### Résultats et Conclusion

Les inventeurs ont ainsi mis en évidence une augmentation significative et dose-dépendante de l'activité luciférase dans les cellules transfectées avec les plasmides pGal4-hPPAR et traitées avec les composés selon l'invention. Les données expérimentales sont résumées dans le Tableau 8-1 ci après, qui présente, pour chaque composé, les valeurs d'activation maximale (TOP %) et les EC₅₀ mesurées pour chaque isoforme de PPAR. Les valeurs d'activation maximales sont exprimées en pourcentages par rapport aux activations maximales obtenues avec des agonistes de référence : l'acide fénofibrique pour PPARα, la rosiglitazone pour PPARγ et l'acide 2-méthyl-4-((4-méthyl-2-(4-trifluorométhylphényl)-1,3-thiazol-5-yl)-méthylsulfanyl)phénoxyacétique (encore nommé GW501516) pour PPARδ.

**Tableau 8-1**

| **Composé n°** | **PPARα** | | **PPARγ** | | **PPARδ** | |
|---|---|---|---|---|---|---|
| | **TOP %** | **EC50 µM** | **TOP %** | **EC50 µM** | **TOP %** | **EC50 µM** |
| 2 | 48,29 | 0,143 | 39,15 | 12,4 | 108,56 | 2,00 |
| 3 | 68,71 | 0,432 | 43,64 | 3,24 | 113,83 | 1,20 |
| 4 | 66,66 | 1,47 | 70,47 | 17,7 | 114,90 | 8,67 |
| 5 | 67,52 | 0,389 | 57,27 | 3,72 | 70,97 | 6,17 |
| 6 | 30,27 | 5,43 | 17,28 | 11,8 | 70,72 | 0,489 |
| 7 | 28,85 | 39,6 | 20,65 | 46,8 | 96,49 | 0,550 |
| 8 | 21,94 | 7,92 | 13,36 | 12,2 | 72,39 | 2,11 |
| 9 | 33,72 | 4,50 | 23,87 | 6,09 | 74,16 | 0,464 |
| 10 | 53,23 | 6,06 | 25,65 | 10,5 | 96,74 | 0,754 |
| 11 | 86,19 | 0,022 | 54,51 | 2,45 | 85,67 | 3,00 |
| 12 | 78,06 | 0,195 | 44,92 | 2,64 | 84,16 | 2,36 |
| 13 | 61,87 | 1,43 | 24,15 | 6,93 | 96,12 | 3,22 |
| 14 | 58,41 | 33,3 | 53,67 | 24,3 | 102,80 | 16,2 |
| 15 | 78,15 | 3,24 | 46,58 | 12,3 | 96,55 | 1,52 |
| 16 | 78,03 | 0,510 | 70,03 | 5,18 | 85,15 | 3,37 |
| 17 | 66,84 | 0,009 | 75,61 | 0,583 | 99,60 | 0,992 |
| 18 | 35,30 | 10,7 | 26,08 | 14,2 | 97,12 | 3,97 |
| 19 | 67,98 | 2,37 | 86,95 | 1,29 | 95,12 | 3,13 |
| 20 | 34,19 | 14,3 | 42,98 | 30,2 | 87,68 | 5,26 |
| 21 | 70,17 | ND | 51,48 | 1,43 | 99,96 | 0,276 |
| 22 | 75,94 | ND | 88,88 | 1,05 | 102,03 | 1,15 |
| 23 | 65,27 | 0,044 | 75,58 | 2,84 | 85,44 | 0,501 |
| 24 | 76,78 | 0,371 | 65,74 | 4,49 | 94,08 | 0,784 |
| 25 | 53,90 | 6,96 | 38,34 | 8,29 | 71,03 | 6,38 |
| 26 | 66,97 | 20,9 | 208,15 | 44,1 | 137,99 | 31,1 |
| 27 | 99,78 | 3,64 | 98,68 | 10,6 | 72,87 | 41,1 |
| 28 | 55,37 | 1,74 | 45,03 | 11,3 | 49,14 | 25,2 |
| 29 | 59,30 | 3,85 | 57,71 | 8,43 | 85,61 | 10,11 |
| 30 | 38,70 | 12,1 | 7,28 | ND | 1,13 | ND |
| 31 | 49,52 | 2,49 | 62,35 | 3,36 | 86,72 | 16,3 |
| 32 | 70,40 | 1,48 | 66,62 | 2,51 | 3,59 | 22,8 |
| 33 | 49,93 | 4,55 | 64,95 | 12,4 | 74,46 | 10,5 |
| 34 | 67,58 | 1,42 | 80,40 | 2,33 | 107,79 | 0,526 |
| 35 | 26,15 | 0,647 | 38,80 | 6,18 | 13,16 | 1,54 |
| 36 | 77,98 | 0,304 | 123,27 | 1,52 | 93,88 | 0,595 |
| 37 | 65,47 | 9,95 | 98,51 | 9,17 | 94,71 | 12,7 |
| 38 | 36,24 | 10,8 | 45,37 | 7,60 | 64,54 | 6,78 |
| 39 | 52,19 | 0,175 | 100,29 | 1,94 | 95,28 | 1,39 |
| 40 | 66,70 | 0,165 | 87,25 | 0,955 | 107,35 | 0,080 |
| 41 | 35,90 | 7,36 | 72,14 | 27,2 | 107,40 | 17,0 |
| 42 | 16,53 | 5,66 | 65,78 | 4,36 | 86,55 | 3,04 |
| 43 | 44,93 | 1,44 | 85,61 | 0,402 | 77,90 | 1,51 |
| 44 | 55,48 | 0,929 | 72,21 | 2,64 | 80,64 | 2,38 |
| 45 | 43,20 | 6,43 | 79,38 | 2,39 | 97,34 | 3,36 |
| 46 | 60,00 | 0,622 | 69,37 | 1,94 | 107,16 | 0,028 |
| 47 | 58,64 | 1,90 | 84,98 | 1,08 | 79,17 | 1,91 |
| 48 | 35,92 | 26,4 | 58,17 | 9,57 | 68,11 | 16,7 |
| 49 | 51,13 | 3,62 | 73,20 | 4,04 | 122,96 | 0,408 |
| 50 | 46,76 | 0,771 | 63,71 | 1,92 | 103,28 | 0,77 |
| 51 | 50,50 | 1,29 | 70,94 | 2,23 | 93,12 | 1,93 |
| 52 | 34,31 | 13,7 | 54,80 | 2,76 | 16,07 | 19,8 |
| 53 | 48,64 | 5,14 | 77,95 | 1,01 | 9,86 | 10,3 |
| 54 | 34,51 | 7,97 | 42,17 | 4,85 | 92,14 | 3,20 |

Les activités mesurées diffèrent selon le composé testé et on observe aussi parmi les composés selon l'invention plus ou moins de sélectivité vis-à-vis des différentes isoformes de hPPAR :
- Certains composés selon l'invention sont sélectifs pour à un sous-type de PPAR. C'est le cas par exemple pour Cpd 11 qui montre une grande sélectivité pour hPPARα ; ce dernier active hPPARγ et hPPARδ avec des EC₅₀ plus de 100 fois supérieures à celle mesurée sur hPPARα. De la même manière, Cpd 7 apparait sélectif vis-à-vis de hPPARδ.
- D'autres composés selon l'invention sont simultanément activateurs de deux ou trois sous-types de PPAR. C'est le cas notamment pour Cpd19 et Cpd 1 qui active hPPARα, hPPARγ, et hPPARδ avec des EC₅₀ comparables. Le composé 1 de l'invention a lui aussi été testé pour ses propriétés agonistes PPAR. Comme le Cpd 19, il présente des sélectivités comparables pour les 3 isoformes PPAR. Les mesures obtenues pour le composé 1 ayant été effectuées selon une méthode différente et avec un matériel différent, par rapport aux 53 autres composés exemplifiés, les résultats correspondants ne sont pas consignés dans le Tableau 8-1 précédent.

Les résultats obtenus montrent que, de manière générale, les composés selon l'invention lient et activent les récepteurs hPPARα, hPPARγ, et/ou hPPARδ de manière significative.

### EXEMPLE 9 : Caractère anti-diabétique des composés selon l'invention

### Principe

L'objet de cette étude est d'évaluer *in vivo* le caractère anti-diabétique des composés selon l'invention chez la souris db/db (Berger J et al., 1996). L'effet anti-diabétique des composés est évaluée par mesure de la glycémie et de l'insulinémie après 8 jours de traitement. Chez des animaux diabétiques (comme chez l'homme), l'administration de glucose a pour conséquence une augmentation significative du taux plasmatique d'insuline. Cette hyper-insulinémie induite provoque une baisse de la glycémie qui est retardée chez les animaux insulino-résistants, comme par exemple chez la souris db/db. L'action corrective des composés sur l'insulino-résistance doit notamment se traduire par une amélioration de la tolérance au glucose.

### Protocole

### a) Traitement des animaux

Des souris de sexe mâle db/db (CERJ - Le Genest St Isle-France) sont utilisées pour réaliser cette expérience. Après une acclimatation d'une semaine, les souris ont été pesées et rassemblées par groupe de 8 animaux sélectionnés de telle sorte que la distribution de leurs poids corporel et de leur glycémie à jeun déterminés une première fois avant l'expérience soient uniformes. Le composé testé a été suspendu dans la carboxyméthylcellulose (Sigma C4888) et administré par gavage intra-gastrique, à raison d'une fois par jour pendant 9 jours à la dose choisie. Les animaux ont eu un accès libre à l'eau et à la nourriture (régime standard) et ont été hébergés dans des cages ventilées sous un rythme lumière/obscurité de 12 heures/12 heures à une température constante de 20 ± 3°C. La prise de nourriture et la prise de poids ont été enregistrées tout au long de l'expérience. Après 8 jours d'administration du composé, un prélèvement sanguin a été effectué par ponction dans le sinus rétro-orbitaire des animaux sous anesthésie volatile à l'isoflurane afin de mesurer les concentrations plasmatiques de glucose et d'insuline.

Après 9 jours, les animaux ont été privés de nourriture 16 heures avant la réalisation du test de tolérance au glucose. Ce dernier consiste en une administration unique de glucose après la mise à jeun (administrée par voie orale à la dose de 1g/kg). Des prélèvements sanguins ont ensuite été effectués au fil du temps pour étudier l'évolution de la glycémie plasmatique.

### b) Mesure de l'insulinémie plasmatique

Le dosage de l'insuline murine est effectué par la méthode Elisa (Insulin Elisa Kit- Crystal Chem. USA). Un anticorps anti-insuline de souris est fixé sur une microplaque. Le sérum à doser pour l'insuline est ensuite déposé sur cette plaque. Un anticorps anti-insuline de cobaye va reconnaître le complexe insuline/anticorps monoclonal anti-insuline de souris et s'y fixer. Enfin un anticorps anti-cobaye marqué à la péroxydase est ajouté se fixant à l'anticorps anti-insuline de cobaye. La réaction colorimétrique est réalisée par addition du substrat de l'enzyme OPD (Ortho Phényle Diamine). L'intensité de la coloration est proportionnelle à la quantité d'insuline présente dans l'échantillon.

### c) Mesure de la glycémie plasmatique

Le dosage du glucose a été effectué par dosages enzymatiques (bioMérieux-Lyon-France) selon les recommandations du fournisseur.

### Résultats et Conclusion

Cette étude vise à évaluer *in vivo* le caractère anti-diabétique d'un composé selon l'invention (Cpd 24) par la mesure de la glycémie et de l'insulinémie après 8 jours de traitement par voie orale avec le composé selon l'invention De manière inattendue, les données expérimentales obtenues montrent que Cpd 24, administrés à 30mpk pendant 8 jours, permette d'améliorer globalement les profils glycémique et insulinémique des animaux diabétiques. Ces résultats se traduisent également par une diminution de l'index HOMA, calculé à partir de ces paramètres plasmatiques, ce qui est le reflet d'une amélioration de la sensibilité à l'insuline (Figures 8a, 8b et 8c).

Chez des animaux normaux et diabétiques (comme chez l'homme), l'administration de glucose a pour conséquence une augmentation significative du taux plasmatique d'insuline. Cette hyper-insulinémie induite provoque une baisse de la glycémie qui est retardée chez les animaux insulino-résistants. Le test de tolérance au glucose montre également une nette diminution de l'insulino-résistance chez les animaux traités pendant 9 jours avec Cpd 24 (Figures 8d et 8e).

Ls composés selon l'invention possèdent des propriétés anti-diabétiques en baissant les taux de glucose et d'insuline plasmatiques. Les composés selon l'invention permettent aussi d'améliorer la sensibilité à l'insuline. Ces résultats *in vivo* témoignent du potentiel thérapeutique des composés selon l'invention vis-à-vis de pathologies majeures telle que le diabète de type 2.

### EXEMPLE 10 : Propriétés hypolipémiantes et stimulatrices de la synthèse de HDL-cholestérol des composés selon l'invention

### Principe

Les propriétés hypolipémiantes des composés selon l'invention ont été évaluées *in vivo* par dosage des lipides plasmatiques et par analyse de l'expression génique de gènes cibles des PPARs dans le foie après traitement par voie orale de une souris dyslipidémique par les composés selon l'invention.

Le modèle murin utilisé est la souris de type ApoE2/E2, souris transgénique pour l'isoforme E2 de l'apolipoprotéine E humaine (Sullivan PM *et al.,* 1998). Chez l'homme, cette apolipoprotéine, constituant des lipoprotéines de faible et très faible densité (LDL-VLDL), est présente sous trois isoformes E2, E3 et E4. La forme E2 présente une mutation sur un acide aminé en position 158, ce qui affaiblit considérablement l'affinité de cette protéine pour le récepteur aux LDL. La clairance des VLDL est de ce fait quasi nulle. Il se produit alors une accumulation des lipoprotéines de faible densité et une hyperlipidémie mixte dite de type III (cholestérol et triglycérides élevés).

PPARα régule l'expression de gènes impliqués dans le transport des lipides (apolipoprotéines telles que Apo AI, Apo AII et Apo CIII, transporteurs membranaires tels que FAT) ou le catabolisme des lipides (ACO, CPT-I ou CPT-II, enzymes de la β-oxydation des acides gras). Un traitement par les activateurs de PPARα se traduit donc, chez l'homme comme chez le rongeur, par une diminution des taux circulants de triglycérides. La mesure des lipides plasmatiques après traitement par les composés selon l'invention est donc un indicateur du caractère agoniste de PPARα et donc de l'action hypolipémiante des composés selon l'invention.

Les propriétés agonistes de PPARα préalablement mesurées *in vitro* doivent se traduire au niveau hépatique par modification du niveau d'expression des gènes cibles directement sous le contrôle du récepteur PPARα : les gènes qui ont été étudiés dans ces expériences sont ceux codant pour l'ACO (une enzyme clé dans le mécanisme de la β-oxydation des acides gras), PDK-4 (une enzyme du métabolisme glucidique) et l'Apo CIII (apolipoprotéine impliquée dans le métabolisme lipidique). Cette modification d'expression génique après traitement par les composés selon l'invention est donc aussi un indicateur du leur caractère hypolipémiant.

### Protocole

### a) Traitement des animaux

Des souris transgéniques Apo E2/E2 ont été maintenues sous un cycle lumière/obscurité de 12 heures/12 heures à une température constante de 20 ± 3°C. Après une acclimatation d'une semaine, les souris ont été pesées et rassemblées par groupes de 6 animaux sélectionnés de telle sorte que la distribution de leur poids corporel et de leur taux de lipides plasmatiques déterminés une première fois avant l'expérience soient uniformes. Les composés testés ont été suspendus dans la carboxyméthylcellulose (Sigma C4888) et administrés à la dose choisie par gavage intra-gastrique, à raison d'une fois par jour pendant 7 jours à la dose choisie. Les animaux ont eu un accès libre à l'eau et à la nourriture. A l'issue de l'expérience, les animaux ont été anesthésiés après un jeûne de 4 heures, un prélèvement sanguin a été effectué sur anticoagulant (EDTA) puis les souris ont été pesées et euthanasiées. Le plasma a été séparé par centrifugation à 3000 tours/minutes pendant 20 minutes, les échantillons ont été conservés à + 4°C pour les dosages biochimiques. Des échantillons de foie ont été prélevés et congelés immédiatement dans de l'azote liquide puis conservés à -80°C pour les analyses ultérieures.

### b) Mesure des lipides plasmatiques

Les concentrations plasmatiques de lipides (cholestérol total, HDL-cholestérol, et acides gras libres) ont été mesurées par dosages enzymatiques (bioMérieux-Lyon-France) selon les recommandations du fournisseur.

### c) Analyse d'expression génique par RT-PCR quantitative

L'ARN total a été extrait à partir de fragments de foie en utilisant le kit NucleoSpin^{®} 96 RNA (Macherey Nagel, Hoerdt, France) selon les instructions du fabriquantitatif 1 µg d'ARN total (quantifié en utilisant le Ribogreen RNA quantification kit (Molecular Probes)) a ensuite été reverse transcrit en ADN complémentaire par une réaction d'1 heure à 37°C dans un volume total de 20 µl contenant du tampon 1X (Sigma), 1,5 mM de DTT, 0,18 mM de dNTPs (Promega), 200 ng de pdN6 (Amersham), 30U d'inhibiteur de RNase (Sigma) et 1 µl de MMLV-RT (Sigma).

Les expériences de PCR quantitative ont été effectuées en utilisant le MyiQ Single-Color Real-Time PCR Detection System (Biorad, Marnes-la-Coquette, France) et ont été réalisées en utilisant le kit iQ SYBR Green Supermix selon les recommandations du fournisseur, en plaques 96 puits, sur 5 µl de réactions de reverse transcription diluées, avec une température d'hybridation de 55°C. Des paires d'amorces spécifiques des gènes étudiés ont été utilisées :
- PDK4 : amorce sens : 5'- TACTCCACTGCTCCAACACCTG-3' (SEQ ID NO : 1) et amorce antisens 5'- GTTCTTCGGTTCCCTGCTTG-3' (SEQ ID NO : 2)
- ACO : amorce sens : 5'- GAAGCCAGCGTTACGAGGTG-3' (SEQ ID NO : 3) et amorce antisens 5'-TGGAGTTCTTGGGACGGGTG-3' (SEQ ID NO : 4)
- ApoCIII : amorce sens : 5'- CTCTTGGCTCTCCTGGCATC-3' (SEQ ID NO : 5) et amorce antisens 5'- GCATCCTGGACCGTCTTGGA-3' (SEQ ID NO : 6)

La quantité de fluorescence émise est directement proportionnelle à la quantité d'ADN complémentaire présent au début de la réaction et amplifiée au cours de la PCR. Pour chaque cible étudiée, une gamme est réalisée par des dilutions successives d'un pool constitué de quelques µl de différentes réactions de reverse transcription. Les niveaux d'expression relatifs de chaque cible sont ainsi déterminés en utilisant les courbes d'efficacité obtenues avec les points de gamme.

Les niveaux d'expression des gènes d'intérêt ont ensuite été normalisés par rapport au niveau d'expression du gène de référence 36B4 (dont les amorces spécifiques sont: amorce sens: 5'-CATGCTCAACATCTCCCCCTTCTCC-3' (SEQ ID NO : 11) et amorce antisens : 5'-GGGAAGGTGTAATCCGTCTCCACAG -3' (SEQ ID NO : 12).

Le facteur d'induction, c'est-à-dire le rapport entre le signal relatif (induit par le composé selon l'invention) et la moyenne des valeurs relatives du groupe contrôle, a ensuite été calculé pour chaque échantillon. Plus ce facteur est élevé, plus le composé a un caractère activateur d'expression génique. Le résultat final est représenté comme moyenne des valeurs d'induction dans chaque groupe expérimental.

### Résultats et Conclusion

### a) Mesure des lipides plasmatiques

Les taux de cholestérol total ont été diminués suite à 7 jours de traitement avec les composés 2 et 4 (administrés à 10 et 100 mpk ; Figure 9a) et avec le composé 19 administré à 100 mpk (Figure 10a). Egalement, le traitement avec Cpd 2 ou avec Cpd 4 a entraîné une augmentation du taux plasmatique du HDL-cholestérol de manière dose dépendante (Figure 9b). Le traitement avec Cpd 19, à 100 mpk, a aussi entraîné une diminution du taux plasmatique des acides gras libres (Figure 10b).

### b) Analyse de l'expression génique par RT-PCR quantitative

Cpd 2 et Cpd 4 induisent une surexpression hépatique des gènes codant pour ACO et PDK-4, et une inhibition significative de l'expression hépatique du gène codant pour l'ApoCIII ((Figures 9c, 9d, et 9c).

Les composés selon l'invention ont des propriétés hypolipémiantes en baissant les taux de cholestérol et d'acides gras libres plasmatiques. Les composés selon l'invention ont aussi la propriété d'augmenter la fraction bénéfique de HDL-cholestérol. De plus, les composés selon l'invention sont des régulateurs de l'expression de gènes codant pour des enzymes fortement impliquées dans le métabolisme des lipides et des glucides. Ces résultats, obtenus *in vivo*, témoignent du potentiel thérapeutique des composés selon l'invention vis-à-vis de pathologies majeures telles que les dyslipidémies.

### Exemple 11 : Propriétés activatrices de PPARδ des composés selon l'invention par mesure de l'expression de gènes cibles de PPARδ dans des myocytes

### Principe

Les propriétés agonistes de PPARδ préalablement mesurées in vitro ont été évaluées par la mesure, dans des myocytes murins, de l'expression de gènes impliqués dans le métabolisme lipidique, glucidique et la dépense énergétique (PDK4, CPT1b, UCP2). La régulation de l'expression de ces gènes, dans ce type cellulaire, est directement la conséquence de l'activation par les composés selon l'invention de PPARδ. Plus l'expression des gènes est augmentée, plus le composé selon l'invention est stimulateur du métabolisme dans les cellules musculaires (Dressel U et al., 2003).

### Protocole

### a) Différentiation des cellules C2C12 en myocytes

Les cellules murines C2C12 (provenance ECACC) sont cultivées dans du milieu DMEM (Gibco ; 41965-039) additionné de 1% L-Glutamine (Gibco ; 25030), de 1% pénicilline/streptomycine (VWR; BWSTL0022/100) et 10% sérum de veau foetal décomplémenté (SVF. Gibco ; 10270-106).

Les cellules sont ensemencées dans des plaques de 24 puits à la densité de 50 x10³ cellules/puits. A confluence, le milieu est remplacé par un milieu de différenciation (milieu de culture de base additionné de 2% de sérum de cheval (Gibco ; 26050-088)) puis la culture est poursuivie à 37°C et 5% de CO₂ pendant 7 jours afin de permettre la différenciation des myoblastes en myocytes.

### b) Traitement

Après 6 jours de différentiation, les cellules sont placées dans un milieu de déprivation (milieu de culture de base sans sérum) pendant 6 heures. Les cellules sont ensuite traitées avec les composés selon l'invention dans le milieu de déprivation. Les composés selon l'invention ont été testés à des doses de à 0.1, 1, 10µM et 0.2, 2 et 20 µM respectivement. Les composés selon l'invention ont été dissous dans du diméthyl sulfoxide (DMSO, Sigma ; D5879). Les cellules sont traitées pendant 24 heures à 37°C, 5% CO2. Les effets relatifs aux composés testés ont été comparés à l'effet du DMSO seul à la concentration de 0, 1 %.

### c) Extraction des ARN, transcription inverse et PCR quantitative.

Ces procédés ont été accomplis sur les cellules essentialement comme décris dans l'Example 10 (point c) des Protocoles).

Les suivants paires d'amorces spécifiques des gènes étudiés ont été utilisées :
- PDK4 : amorce sens : 5'-TACTCCACTGCTCCAACACCTG-3' (SEQ ID NO : 1) et amorce antisens 5'-GTTCTTCGGTTCCCTGCTTG-3' (SEQ ID NO : 2)
- CPT1b: amorce sens : 5'-GGACTGAGACTGTGCGTTCCTG-3' (SEQ ID NO : 7) et amorce antisens 5'-AGTGCTTGGCGGATGTGGTT-3' (SEQ ID NO : 8)
- UCP2 : amorce sens : 5'-GTCGGAGATACCAGAGCACTGTCG-3' (SEQ ID NO : 9) et amorce antisens 5'-CACATCAACAGGGGAGGCGA-3' (SEQ ID NO : 10)

### Résultats et Conclusion

Dans des myocytes murins *in vitro,* que les composés de l'invention comme Cpd 7 et Cpd 24 possèdent des effets stimulateurs significatfs sur l'expression de gènes impliqués dans le métabolisme glucidique, lipidique et dans la thermorégulation, et connus pour être régulés par les agonistes de PPARδ, comme PDK4, CPT1b et UCP2 (Figures 11a, 11b et 11c).

Les données expérimentales présentées montrent que les composés de l'invention ont une action métabolique dans les myocytes murins par l'activation de PPARδ.

### Exemple 12 : Propriétés activatrices de PPARγ des composés selon l'invention

### Principe

L'effet fonctionnel de l'activation de PPARγ est évalué *in vitro* sur une lignée de cellules pré-adipocytaires 3T3-L1 (Thompson GM et al., 2004) par la mesure de la concentration de triglycérides intracellulaires et de la concentration d'adiponectine secrétée dans le milieu de culture après 9 jours de traitement avec les composés selon l'invention. Cet effet, similaire a celui des composés agonistes de PPARγ comme les thiazolidinediones (Kallen CB and Lazar MA, 1996), qui a permis de démontrer in vitro des propriétés fonctionnelles des ces composés.

### Protocole

Les cellules 3T3-L1 (*Mus musculus* embryo fibroblasts, ATCC ; CL-173) sont cultivées dans du milieu DMEM (4,5 g/L glucose, Gibco 41965) supplémenté avec 10% de Serum de Veau Foetal (Gibco ; 10270), 1% de L-glutamine (Gibco ; 25030), 100 unités/mL de pénicilline + 100 µg/mL streptomycine (VWR ; BWSTL0022/100) jusqu'à confluence (37°C, 5% CO2). A confluence, les cellules ont été alors rincées au PBS (Phosphate Buffered Saline solution) et le milieu de culture remplacé par du DMEM 4,5 g/L glucose (10% SVF, 1% L-Glutamine, 100 unités/mL pénicilline and 100 µg/mL streptomycine, 0,25 mM) contenant un cocktail d'initiation de la différenciation adipocytaire (IBMX (3-Isobutyl-1-methylxanthine Sigma ; I7018), 0,1µM dexamethasone (Sigma ; D1756) et 0,4 µM d'insuline (Sigma ; I2643). Les cellules ont été également traitées avec les composés selon l'invention (1µL de composé solubilisé / mL de milieu, 200µL par puits).

Après 2 jours d'incubation, les cellules ont été lavées avec du PBS et le milieu de différenciation a été remplacé par du DMEM 4,5g/L de glucose supplémenté de 10% SVF, 1% L-Glutamine, 100 unités/mL pénicilline et 100 µg/mL streptomycine, 0,4µM insuline, et les composés selon l'invention dans le but d'achever la différenciation cellulaire et d'obtenir des adipocytes. Le milieu a été changé tous les 2 jours jusqu'à totale différenciation. Après 9 jours de culture, le traitement a été arrêté par retrait du milieu de culture qui a été stocké pour le dosage d'adiponectine sécrétée. Les cellules ont été ensuite lavées 2 fois au PBS et placées dans l'isopropanol pour la perméabilisation membranaire. Le contenu en triglycérides intracellulaires a été immédiatement évalué.

La quantification des triglycérides intracellulaires a été réalisée avec le kit «Triglyceride Enzymatique PAP1000 » (bioMérieux ; 61238) selon les recommandations du fournisseur. L'adiponectine sécrétée a été mesurée dans le milieu de culture via le kit « Mouse Adiponectin / Acrp30 Elisa Kit» (R&D Systems; DY1119) selon les recommendations du fournisseur.

### Résultats et Conclusion

Les données expérimentales montrent que Cpd 19, Cpd 24 et Cpd 36 stimulent *in vitro* de façon dose dépendante l'accumulation de triglycérides dans les adipocytes, et la sécrétion d'adiponectine. Ces résultats montrent donc une capacité activatrice de PPARγ *in vitro* des composés selon l'invention, dont la stimulation de l'adipogénèse dans le modèle cellulaire de pré-adipocytes murins 3T3-L1 est le reflet.

### BIBLIOGRAPHIE

Berger J et al., Endocrinology. 1996, 137(10), 4189-95.
Blaschke F et al., Arterioscler Thromb Vasc Biol. 2006, 26, 28-40.
Burger A, Prog Drug Res. 1991, 37, 287-371
Dressel U et al., Mol Endocr..2003, 17(12):2477-2493.
Gervois P et al., Nat Clin Pract Endocrinol Metab, 2007, 3 (2) 145-156.
Gilde AJ et al., J Am Coli Cardiol. 2006, 48(9), A24-A32.
Gross B et al., Drug Disc Today: Therapeutic Strategies, 2005, 2 (3), 237-243.
Hansen MK et Connolly TM, Curr Opin Invest Drugs. 2008, 9(3), 247-255.
Hourton D et al. Biochem J 2001? 354(Pt1), 225-232.
Kallen CB and Lazar MA, Proc Natl Acad Sci U S A. 1996, 93(12),5793-6.
Krause B, Curr Opin Invest Drugs. 2008, 9(9), 945-9.
Lefebvre P et al., J Clin Invest, 2006, 116 (3), 571-580
Lehrke M and Lazar MA, Cell, 2005, 123 (6), 993-999.
Lima LM and Barreiro EJ, Curr Med Chem. 2005, 12(1), 23-49.
Peraza M et al., Toxicol. Sci., 2006, 90(2), 269-295.
Raspe E J Lipid Res, 1999, 40 (11), 2099-110.
Sullivan PM et al., J Clin Invest. 1998, 102(1), 130-5.
Thompson GM et al., Anal Biochem. 2004, 330(1), 21-8.

## Revendications

1. Composé de Formule Générale: 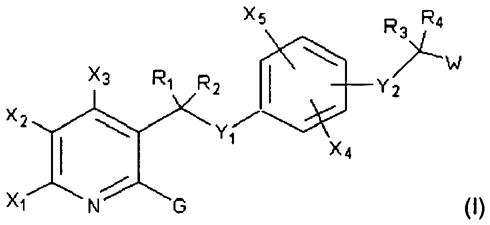 dans laquelle,
G représente :
- un radical -ORₐ, -SRₐ, ou
- un radical -NRₐR_{b},
Rₐ étant choisi parmi un radical alkyle de 1 à 6 atomes de carbone ou alkényle de 2 à 6 atomes de carbone, un cycle de 3 à 14 atomes, un radical phényle, un radical phénylalkyle de partie alkyle ayant 1 à 3 atomes de carbone ;
R_{b} étant choisi parmi un atome d'hydrogène, un radical alkyle de 1 à 6 atomes de carbone ou alkényle de 2 à 6 atomes de carbone, un cycle de 3 à 14 atomes, un radical phényle, ou un radical phénylalkyle de partie alkyle ayant 1 à 3 atomes de carbone ;
Rₐ et R_{b} peuvent former, ensemble et avec l'atome d'azote auquel ils sont liés, un hétérocycle de 3 à 8 atomes.
R₁ et R₂, identiques ou différents, représentent un atome d'hydrogène ou un radical alkyle de 1 à 6 atomes de carbone ou alkényle de 2 à 6 atomes de carbone;
R₁ et R₂ pouvant former, ensemble et avec l'atome de carbone auquel ils sont liés, un carbocycle de 3 à 6 atomes de carbone ;
Y₁ représente :
- un atome d'oxygène ou de soufre, ou
- un groupe -NR-, dans lequel R a la même définition que R_{b};
Y₂ représente :
- un atome d'oxygène ou de soufre, ou
- un radical -CR₅R₆-; avec R₅ et R₆, identiques ou différents, choisis parmi un atome d'hydrogène ou d'halogène, un radical alkyle de 1 à 6 atomes de carbone ou un radical alkényle ou alkynyle de 2 à 6 atomes de carbone, un cycle de 3 à 6 atomes, un radical phényle, un radical phénylalkyle de partie alkyle ayant 1 à 3 atomes de carbone;
X₁, X₂, X₃ représentent indépendamment un atome d'hydrogène ou d'halogène, un radical alkyle de 1 à 6 atomes de carbone ou alkényle de 2 à 6 atomes de carbone, un groupement -OR'ₐ, -SR'ₐ, -NR'ₐR'_{b}, un cycle de 5 à 14 atomes, ou un radical phénylalkyle de partie alkyle ayant 1 à 3 atomes de carbone,
avec au moins un des groupements X₁, X₂ et X₃ différent d'un atome d'hydrogène et d'un atome d'halogène ;
R'ₐ et R'_{b}, identiques ou différents, ayant les mêmes définitions que Rₐ et R_{b};
X₄ et X₅ représentent indépendamment un atome d'hydrogène ou d'halogène, un radical alkyle de 1 à 6 atomes de carbone ou alkényle de 2 à 6 atomes de carbone, un groupement -OR"ₐ, -SR"ₐ ou -NR"ₐR"_{b}, un cycle de 3 à 14 atomes, un radical phényle, ou un radical phénylalkyle de partie alkyle ayant 1 à 3 atomes de carbone ;
R"ₐ et R"_{b}, identiques ou différents, ayant les mêmes définitions que Rₐ et R_{b};
R₃ et R₄, identiques ou différents, représentent un atome d'hydrogène ou d'halogène, un radical alkyle de 1 à 6 atomes de carbone ou alkényle de 2 à 6 atomes de carbone, un cycle de 3 à 14 atomes, un radical phényle, ou un radical phénylalkyle de partie alkyle ayant 1 à 3 atomes de carbone ;
W représente :
- un radical carboxyle -COOH ; ou
- une fonction dérivée de la fonction acide carboxylique, choisie parmi
- COOR"'ₐ, -COSR"'ₐ, -CONR"'ₐR"'_{b}, -CSNR"'ₐR"'_{b}, -CONH₂ ; ou
- un groupement bioisostère du radical carboxyle, choisi parmi :
- un radical acylsulfonamide (-CONHSO₂R"'ₐ) ;
- un radical hydrazide (-CONHNR"'ₐR"'_{b});
- un radical choisi parmi les cycles thiazolidinedione, oxazolidinedione, tétrazole, oxadiazolone, triazolone, triazole, 3-alkyltriazole, ou imidazolidinedione ;
R"'ₐ et R"'_{b}, identiques ou différents, ayant les mêmes définitions que Rₐ et R_{b}.

2. Composé selon la revendication 1, **caractérisé en ce que** Y₂ se trouve en para ou en méta de Y₁.

3. Composé selon la revendication 1 ou 2, dans lequel :
- X₃ désigne un atome d'hydrogène, et/ou
- X₄ et X₅ désignent indépendamment un atome d'hydrogène, un radical alkyle ayant 1 à 6 atomes de carbone, un groupement -OR"ₐ ou -SR"ₐ ; R"ₐ étant un radical alkyle ayant 1 à 6 atomes de carbone.

4. Composé selon l'une des revendications 1 à 3 dans lequel R₁, R₂, R₃, R₄ désignent indépendamment un atome d'hydrogène ou un radical méthyle, éthyle, propyle, butyle, isopropyle ou tertio-butyle.

5. Composé selon l'une des revendications 1 à 4, dans lequel X₃, R₁ et R₂ désignent des atomes d'hydrogène.

6. Composé selon l'une des revendications 1 à 5, dans lequel X₁ et/ou X₂ désigne(nt) un cycle de 5 à 10 atomes, non substitué ou substitué par un groupement -CF₃.

7. Composé selon l'une des revendications 1 à 6, dans lequel G désigne
- un radical -ORₐ ou -SRₐ, avec Rₐ choisi parmi radical alkyle ayant 1 à 6 atomes de carbone, un cyclohexyle ou un radical phényle ; ou
- un radical -NRₐR_{b}, Rₐ et R_{b} formant ensemble, et avec l'atome d'azote auquel ils sont liés, un hétérocycle de 3 à 8 atomes.

8. Composé selon l'une des revendications 1 à 6, dans lequel G désigne un radical -ORₐ avec Rₐ choisi parmi un radical méthyle, éthyle, propyle, butyle, isopropyle ou tertio-butyle

9. Composé selon la revendication 7 ou 8, **caractérisé en ce que** :
- X₂ et X₃ désignent simultanément un atome d'hydrogène ; ou
- X₃ et X₁ désignent simultanément un atome d'hydrogène.

10. Composé selon l'une des revendications 1 à 9, dans lequel Y₁ désigne un atome d'oxygène ou soufre, et simultanément Y₂ désigne un atome d'oxygène, un atome de soufre, ou un groupement -CR₅R₆.

11. Composé selon l'une des revendications 1 à 9, **caractérisé en ce que** Y₁ désigne un groupe -NR et simultanément Y₂ désigne un atome d'oxygène, un atome de soufre ou un groupement -CR₅R₆.

12. Composé selon la revendication 10 ou 11, dans lequel X₁ désigne un radical phényle non substitué ou un radical phényle substitué par un groupement -CF₃, ledit groupement -CF₃ étant de préférence en para du radical pyridinyle, et/ou G désigne un groupement -OCH₃ ou -OC(CH₃)₃.

13. Composé selon l'une des revendications 1 à 12, **caractérisé en ce qu'**il est choisi parmi :
| | |
|---|---|
| Cpd 1 : | acide 2-(4-((2-méthoxy-6-phénylpyridin-3-yl)méthoxy)phénoxy)-2-méthyl-propanoïque |
| Cpd 2 : | acide 2-(4-((2-méthoxy-6-phénylpyridin-3-yl)méthoxy)phénoxy) éthanoïque |
| Cpd 3 : | acide 2-(4-((2-méthoxy-6-phénylpyridin-3-yl)méthoxy)phénoxy) propanoïque |
| Cpd 4 : | acide 2-(4-(((2-méthoxy-6-phénylpyridin-3yl)méthyl)amino)phénoxy) éthanoïque |
| Cpd 5 : | acide 2-(4-(((2-méthoxy-6-phénylpyridin-3yl)méthyl)amino)phénoxy) propanoïque |
| Cpd 6 : | acide 2-(4-((2-tertio-butyloxy-6-phénylpyridin-3-yl)méthoxy)phénoxy)-2-méthyl-propanoïque |
| Cpd 7 : | acide 2-(4-(((2-tertio-butyloxy-6-phénylpyridin-3-yl)méthoxy)phénoxy) éthanoïque |
| Cpd 8 : | acide 2-(4-((2-tertio-butyloxy-6-phénylpyridin-3-yl)méthyl)amino) phénoxy)éthanoïque |
| Cpd 9 : | acide 2-(4-(((2-tertio-butyloxy-6-phénylpyridin-3-yl)méthyl)amino) phénoxy)-2-méthyl-propanoïque |
| Cpd 10 : | acide 2-(4-(((2-tertio-butyloxy-6-phénylpyridin-3-yl)méthyl)amino) phénoxy)-propanoïque |
| Cpd 11 : | acide 2-(4-(((2-méthoxy-6-phénylpyridin-3-yl)méthyl)amino)phénylthio)-2-méthyl-propanoïque |
| Cpd 12 : | acide 2-(4-(((2-méthoxy-6-phénylpyridin-3-yl)méthyl)amino)phénoxy)-2-méthyl-propanoïque |
| Cpd 13 : | acide 2-(3-(((2-méthoxy-6-phénylpyridin-3-yl)méthyl)amino)phénoxy)-2-méthyl-propanoïque |
| Cpd 14 : | acide 2-(3-(((2-méthoxy-6-phénylpyridin-3-yl)méthyl)amino)phénoxy) éthanoïque |
| Cpd 15 : | acide 2-(4-((2-hexyloxy-6-phénylpyridin-3-yl)méthoxy)phénoxy) éthanoïque |
| Cpd 16 : | acide 2-(4-(((2-méthoxy-6-phénylpyridin-3-yl)méthyl)amino)phénylthio)-éthanoïque |
| Cpd 17 : | acide 2-(4-((2-hexyloxy-6-phénylpyridin-3-yl)méthoxy)phénoxy)-2-méthyl-propanoïque |
| Cpd 18 : | acide 2-(4-((2-cyclohexyloxy-6-phénylpyridin-3-yl)méthoxy)phénoxy) éthanoïque |
| Cpd 19 : | acide 3-(4-(((2-méthoxy-6-phénylpyridin-3-yl)méthyl)amino)phényl) propanoïque |
| Cpd 20 : | acide 2-(4-((6-phényl-2-(pipéridin-1-yl)pyridin-3-yl)méthoxy)phénoxy)-éthanoïque |
| Cpd 21 : | acide 2-(4-((2-méthoxy-6-(4-(trifluorométhyl)phényl)pyridin-3-yl)méthoxy) - phénoxy)-2-méthylpropanoïque |
| Cpd 22 : | acide 2-(4-(((2-méthoxy-6-(4-(trifluorométhyl)phényl)pyridin-3-yl)méthyl) amino)-phénylthio)-2-méthylpropanoïque |
| Cpd 23 : | acide 2-(4-(((2-méthoxy-6-(4-(trifluorométhyl)phényl)pyridin-3-yl)méthyl) amino)-phénylthio)éthanoïque |
| Cpd 24 : | acide 2-(4-((2-méthoxy-6-(4-(trifluorométhyl)phényl)pyridin-3-yl)méthoxy) - phénoxy)éthanoïque |
| Cpd 25 : | acide 2-(4-((2-phénylthio-6-(phényl)pyridin-3-yl)méthoxy)phénoxy) éthanoïque |
| Cpd 26 : | acide 2-(4-(((2-méthoxy-5-phénylpyridin-3-yl)méthyl)amino)phénylthio) éthanoïque |
| Cpd 27 : | acide 2-(4-(((2-méthoxy-6-phénylpyridin-3-yl)méthyl)amino)phénylthio)-2,2-difluoroéthanoïque |
| Cpd 28 : | acide 2-(4-(((2-méthoxy-5,6-diphénylpyridin-3-yl)méthyl)amino) phénylthio)-éthanoïque |
| Cpd 29 : | acide 2-(4-(((2-méthoxy-5-bromo-6-phénylpyridin-3-yl)méthyl)amino) phénylthio)-éthanoïque |
| Cpd 30 : | acide 2-(4-(((2-méthoxy-6-furylpyridin-3-yl)méthyl)amino)phénylthio)-éthanoïque |
| Cpd 31 : | acide 3-(4-(((2-méthoxy-6-furylpyridin-3-yl)méthyl)amino)phényl) propanoïque |
| Cpd 32 : | acide 2-(4-(((2-méthoxy-6-phénylpyridin-3-yl)méthyl)amino)phénylthio)-2-phényl-éthanoïque |
| Cpd 33 : | acide 3-(4-(((2-méthoxy-6-phénylpyridin-3-yl)méthyl)(méthyl)amino) phényl)-propanoïque |
| Cpd 34 : | acide 3-(4-(1-((2-méthoxy-6-phénytpyridin-3-yl)propyl)amino)phényl)-propanoïque |
| Cpd 35 : | acide 2-(4-(((2-méthoxy-6-phénylpyridin-3-yl)méthyl)amino)-2,6-diméthyl-phénoxy)éthanoïque |
| Cpd 36 : | acide 3-(4-(((2-méthoxy-6-(4-(trifluorométhyl)phényl)pyridin-3-yl)méthyl) amino) -phényl)-propanoïque |
| Cpd 37 : | acide 3-(4-((2-méthoxy-6-phénylpyridin-3-yl)méthylthio)phényl) propanoïque |
| Cpd 38 : | acide 3-(4-(((2-(éthylthio)-6-phénylpyridin-3-yl)méthyl)amino)phényl)-propanoïque |
| Cpd 39 : | acide 3-(4-(((2-méthoxy-6-(parabiphényl)pyridin-3-yl)méthyl)amino) phényl)-propanoïque |
| Cpd 40 : | acide 3-(4-(((2-méthoxy-6-(3-(trifluorométhyl)phényl)pyridin-3-yl)méthyl) amino)-phényl)propanoïque |
| Cpd 41 : | acide 3-(4-(((2-méthoxy-5-phénylpyridin-3-yl)méthyl)amino)phényl)-propanoïque |
| Cpd 42 : | acide 3-(4-((2(-méthoxy-6-phénylpyridin-3-yl)méthyl)amino)phényl)-3-phényl-propanoïque |
| Cpd 43 : | acide 3-(2-méthoxy-4-(((2-méthoxy-6-phénylpyridin-3-yl)méthyl)amino)-phényl)-propanoïque |
| Cpd 44 : | acide 3-(3-méthoxy-4-(((2-méthoxy-6-phénylpyridin-3-yl)méthyl)amino)-phényl)-propanoïque |
| Cpd 45 : | acide 3-(4-(((2-méthoxy-6-phénylpyridin-3-yl)méthyl)amino)phényl) butanoïque |
| Cpd 46 : | acide 3-(4-(((2-méthoxy-5-(4-(trifluorométhyl)phényl)pyridin-3-yl)méthyl) amino)phényl)propanoïque |
| Cpd 47 : | acide 3-(4-(((2-méthoxy-5-(3-(trifluorométhyl)phényl)pyridin-3-yl)méthyl) amino)-phényl)propanoïque |
| Cpd 48 : | acide 3-(4-(((2,6-diméthoxy-5-phénylpyridin-3-yl)méthyl)amino)phényl)-propanoïque |
| Cpd 49 : | acide 3-(4-(((5-(4-chlorophényl)-2-méthoxypyridin-3-yl)méthyl)amino) phényl)-propanoïque |
| Cpd 50 : | acide 3-(4-(((2-méthoxy-5-(naphthalèn-2-yl)pyridin-3-yl)méthyl)amino) phényl)-propanoïque |
| Cpd 51 : | acide 3-(4-(((2-éthoxy-6-phénylpyridin-3-yl)méthyl)amino)phényl) propanoïque |
| Cpd 52 : | acide 3-(4-((2-méthoxy-5-phénylpyridin-3-yl)méthoxy)phényl)hex-4-ynoïque |
| Cpd 53 : | acide 3-(4-((2-méthoxy-6-phénylpyridin-3-yl)méthoxy)phényl)hex-4-ynoïque |
| Cpd 54 : | acide 3-(4-(((2-isopropyloxy-6-phénylpyridin-3-yl)méthyl)amino) phényl)-propanoïque. |

14. Composition pharmaceutique comprenant, dans un support acceptable sur le plan pharmaceutique, au moins un composé tel que défini dans l'une des revendications 1 à 13, éventuellement en association avec un ou plusieurs autres actifs thérapeutiques et/ou cosmétiques.

15. Composition pharmaceutique selon la revendication 14 pour le traitement thérapeutique, curatif et/ou prophylactique du diabète, des dyslipidémies, de l'insulino-résistance, des pathologies associées au syndrome métabolique, de l'athérosclérose, des maladies cardiovasculaires, de l'obésité, de l'hypertension et/ou des maladies inflammatoires.

## Patentansprüche

1. Verbindung der allgemeinen Formel 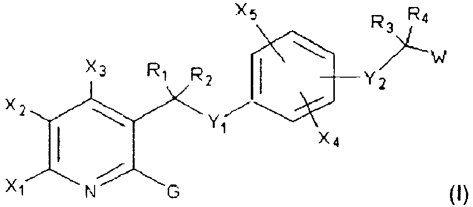 in der
G steht für
- einen -ORₐ-, -SRₐ-Rest oder
- einen -NRₐR_{b}-Rest,
wobei Rₐ aus einem Alkylrest mit 1 bis 6 Kohlenstoffatomen oder einem Alkenylrest mit 2 bis 6 Kohlenstoffatomen, einem Ring mit 3 bis 14 Atomen, einem Phenylrest, einem Phenylalkylrest mit einem Alkylrest, der 1 bis 3 Kohlenstoffatome besitzt, ausgewählt ist;
wobei R_{b} aus einem Wasserstoffatom, einem Alkylrest mit 1 bis 6 Kohlenstoffatomen oder einem Alkenylrest mit 2 bis 6 Kohlenstoffatomen, einem Ring mit 3 bis 14 Atomen, einem Phenylrest oder einem Phenylalkylrest mit einem Alkylrest, der 1 bis 3 Kohlenstoffatome besitzt, ausgewählt ist;
Rₐ und R_{b} gemeinsam und mit dem Stickstoffatom, an das sie gebunden sind, einen Heterocyclus mit 3 bis 8 Atomen bilden können;
R₁ und R₂, die identisch oder verschieden sind, für ein Wasserstoffatom oder einen Alkylrest mit 1 bis 6 Kohlenstoffatomen oder einen Alkenylrest mit 2 bis 6 Kohlenstoffatomen stehen;
wobei R₁ und R₂ gemeinsam und mit dem Kohlenstoffatom, an das sie gebunden sind, einen Kohlenstoffring mit 3 bis 6 Kohlenstoffatomen bilden können;
Y₁ steht für:
- ein Sauerstoff- oder Schwefelatom oder
- eine -NR-Gruppe, in der R wie R_{b} definiert ist;
Y₂ steht für:
- ein Sauerstoff- oder Schwefelatom oder
- einen -CR₅R₆- Rest, wobei R₅ und R₆, die identisch oder verschieden sind, aus einem Wasserstoff- oder Halogenatom, einem Alkylrest mit 1 bis 6 Kohlenstoffatomen oder einem Alkenyl- oder Alkinylrest mit 2 bis 6 Kohlenstoffatomen, einem Ring mit 3 bis 6 Atomen, einem Phenylrest, einem Phenylalkylrest mit einem Alkylrest, der 1 bis 3 Kohlenstoffatome besitzt, ausgewählt sind;
X₁, X₂, X₃ unabhängig voneinander für ein Wasserstoff- oder Halogenatom, einen Alkylrest mit 1 bis 6 Kohlenstoffatomen oder einen Alkenylrest mit 2 bis 6 Kohlenstoffatomen, eine -OR'ₐ-, - SR'ₐ-, -NR'ₐR'_{b-}Gruppe, einen Ring mit 5 bis 14 Atomen oder einen Phenylalkylrest mit einem Alkylrest, der 1 bis 3 Kohlenstoffatome besitzt, stehen;
wobei wenigstens eine der Gruppen X₁, X₂ und X₃ sich von einem Wasserstoffatom und einem Halogenatom unterscheidet;
wobei R'ₐ und R'_{b}, die identisch oder verschieden sind, wie Rₐ und R_{b} definiert sind;
X₄ und X₅ unabhängig voneinander für ein Wasserstoff- oder Halogenatom, einen Alkylrest mit 1 bis 6 Kohlenstoffatomen oder einen Alkenylrest mit 2 bis 6 Kohlenstoffatomen, eine -OR"ₐ-, - SR"ₐ- oder -NR"ₐR"_{b}-Gruppe, einen Ring mit 3 bis 14 Atomen, einen Phenylrest oder einen Phenylalkylrest mit einem Alkylrest, der 1 bis 3 Kohlenstoffatome besitzt, stehen;
wobei R"ₐ und R"_{b}, die identisch oder verschieden sind, wie Rₐ und R_{b} definiert sind;
R₃ und R₄, die identisch oder verschieden sind, für ein Wasserstoff- oder Halogenatom, einen Alkylrest mit 1 bis 6 Kohlenstoffatomen oder einen Alkenylrest mit 2 bis 6 Kohlenstoffatomen, einen Ring mit 3 bis 14 Atomen, einen Phenylrest oder einen Phenylalkylrest mit einem Alkylrest, der 1 bis 3 Kohlenstoffatome besitzt, stehen;
W steht für
- einen Carboxylrest -COOH; oder
- eine Funktion, die von der Carbonsäurefunktion abgeleitet ist und aus -COOR"'ₐ, -COSR"'ₐ, - CONR"'ₐR"'_{b}, -CSNR"'ₐR"'_{b}, -CONH₂ ausgewählt ist; oder
- eine bioisosterische Gruppe des Carboxylrestes, ausgewählt aus:
- einem Acylsulfonamidrest (-CONHSO₂R"'ₐ);
- einem Hydrazidrest (-CONHNR"'ₐR"'_{b});
- einem Rest, der aus Thiazolidindion-, Oxazolidindion-, Tetrazol-, Oxadiazolon-, Triazolon-, Triazol-, 3-Alkyltriazol- oder Imidazolidindionring ausgewählt ist;
wobei R"'ₐ und R"'_{b}, die identisch oder verschieden sind, wie Rₐ und R_{b} definiert sind.

2. Verbindung gemäß Anspruch 1, **dadurch gekennzeichnet, dass** Y₂ para oder meta zu Y₁ steht.

3. Verbindung gemäß Anspruch 1 oder 2, wobei
- X₃ ein Wasserstoffatom bezeichnet, und/oder
X₄ und X₅ unabhängig voneinander ein Wasserstoffatom, einen Alkylrest mit 1 bis 6 Kohlenstoffatomen, eine -OR"ₐ- oder -SR"ₐ-Gruppe bezeichnen; wobei R"ₐ ein Alkylrest mit 1 bis 6 Kohlenstoffatomen ist.

4. Verbindung gemäß einem der Ansprüche 1 bis 3, wobei R₁, R₂, R₃, R₄ unabhängig voneinander ein Wasserstoffatom oder einen Methyl-, Ethyl-, Propyl-, Butyl, Isopropyl- oder tert.-Butylrest bezeichnen.

5. Verbindung gemäß einem der Ansprüche 1 bis 4, wobei X₃, R₁ und R₂ Wasserstoffatome bezeichnen.

6. Verbindung gemäß einem der Ansprüche 1 bis 5, wobei X₁ und/oder X₂ einen nichtsubstituierten oder mit einer -CF₃-Gruppe substituierten Ring mit 5 bis 10 Atomen bezeichnet.

7. Verbindung gemäß einem der Ansprüche 1 bis 6, wobei G bezeichnet
- einen -ORₐ- oder -SRₐ-Rest, wobei Rₐ aus einem Alkylrest mit 1 bis 6 Kohlenstoffatomen, einem Cyclohexyl- oder einem Phenylrest ausgewählt ist; oder
- einen -NRₐR_{b}-Rest, wobei Rₐ und R_{b} gemeinsam und mit dem Stickstoffatom, an das sie gebunden sind, einen Heterocyclus mit 3 bis 8 Atomen bilden können.

8. Verbindung gemäß einem der Ansprüche 1 bis 6, wobei G einen -ORₐ-Rest bezeichnet, wobei Rₐ aus einem Methyl-, Ethyl-, Propyl-, Butyl, Isopropyl- oder tert.-Butylrest ausgewählt ist

9. Verbindung gemäß Anspruch 7 oder 8, **dadurch gekennzeichnet, dass**:
- X₂ und X₃ gleichzeitig ein Wasserstoffatom bezeichnen; oder
- X₃ und X₁ gleichzeitig ein Wasserstoffatom bezeichnen.

10. Verbindung gemäß einem der Ansprüche 1 bis 9, wobei Y₁ ein Sauerstoff- oder Schwefelatom bezeichnet, und gleichzeitig Y₂ ein Sauerstoffatom, ein Schwefelatom oder eine - CR₅R₆-Gruppe bezeichnet.

11. Verbindung gemäß einem der Ansprüche 1 bis 9, **dadurch gekennzeichnet, dass** Y₁ eine -NR-Gruppe bezeichnet, und gleichzeitig Y₂ ein Sauerstoffatom, ein Schwefelatom oder eine -CR₅R₆-Gruppe bezeichnet.

12. Verbindung gemäß Anspruch 10 oder 11, wobei X₁ einen nichtsubstituierten Phenylrest oder einen mit einer -CF₃-Gruppe substituierten Phenylrest bezeichnet, wobei besagte -CF₃-Gruppe vorzugsweise para zum Pyridinylrest steht, und/oder G eine -OCH₃-Gruppe oder -OC(CH₃)₃-Gruppe bezeichnet.

13. Verbindung gemäß einem der Ansprüche 1 bis 12, **dadurch gekennzeichnet, dass** sie ausgewählt ist aus:
| | |
|---|---|
| Cpd 1: | 2-(4-((2-Methoxy-6-phenylpyridin-3-yl)methoxy)phenoxy)-2-methyl-propansäure |
| Cpd 2: | 2-(4-((2-Methoxy-6-phenylpyridin-3-yl)methoxy)phenoxy)ethansäure |
| Cpd 3: | 2-(4-((2-Methoxy-6-phenylpyridin-3-yl)methoxy)phenoxy)propansäure |
| Cpd 4: | 2-(4-(((2-Methoxy-6-phenylpyridin-3-yl)methyl)amino)phenoxy)ethansäure |
| Cpd 5: | 2-(4-(((2-Methoxy-6-phenylpyridin-3-yl)methyl)amino)phenoxy)propansäure |
| Cpd 6: | 2-(4-((2-tert.-Butyloxy-6-phenylpyridin-3-yl)methoxy)phenoxy)-2-methyl-propansäure |
| Cpd 7: | 2-(4-((2-tert.-Butyloxy-6-phenylpyridin-3-yl)methoxy)phenoxy)ethansäure |
| Cpd 8: | 2-(4-(((2-tert.-Butyloxy-6-phenylpyridin-3-yl)methyl)amino)phenoxy)ethansäure |
| Cpd 9: | 2-(4-(((2-tert.-Butyloxy-6-phenylpyridin-3-yl)methyl)amino)phenoxy)-2-methyl-propansäure |
| Cpd 10: | 2-(4-(((2-tert.-Butyloxy-6-phenylpyridin-3-yl)methyl)amino)phenoxy)propansäure |
| Cpd 11: | 2-(4-(((2-Methoxy-6-phenylpyridin-3-yl)methyl)amino)phenylthio)-2-methyl-propansäure |
| Cpd 12: | 2-(4-(((2-Methoxy-6-phenylpyridin-3-yl)methyl)amino)phenoxy)-2-methyl-propansäure |
| Cpd 13: | 2-(3-(((2-Methoxy-6-phenylpyridin-3-yl)methyl)amino)phenoxy)-2-methyl-propansäure |
| Cpd 14: | 2-(3-(((2-Methoxy-6-phenylpyridin-3-yl)methyl)amino)phenoxy)ethansäure |
| Cpd 15: | 2-(4-((2-Hexyloxy-6-phenylpyridin-3-yl)methoxy)phenoxy)ethansäure |
| Cpd 16: | 2-(4-(((2-Methoxy-6-phenylpyridin-3-yl)methyl)amino)phenylthioethansäure |
| Cpd 17: | 2-(4-((2-Hexyloxy-6-phenylpyridin-3-yl)methoxy)phenoxy)-2-methyl-propansäure |
| Cpd 18: | 2-(4-((2-Cyclohexyloxy-6-phenylpyridin-3-yl)methoxy)phenoxy)ethansäure |
| Cpd 19: | 3-(4-(((2-Methoxy-6-phenylpyridin-3-yl)methyl)amino)phenyl)propansäure |
| Cpd 20: | 2-(4-((6-Phenyl-2-(pipendin-1-yl)pyridin3-yl)methoxy)phenoxy)-ethansäure |
| Cpd 21: | 2-(4-((2-Methoxy-6-(4-(trifluormethyl)phenyl)pyridin-3-yl)methoxy)phenoxy)-2-methylpropansäure |
| Cpd 22: | 2-(4-(((2-Methoxy-6-(4-(trifluormethyl)phenyl)pyridin-3-yl)methyl)amino)phenylthio)-2-methylpropansäure |
| Cpd 23: | 2-(4-(((2-Methoxy-6-(4-(trifluormethyl)phenyl)pyridin-3-yl)methyl)amino)phenylthio)ethansäure |
| Cpd 24: | 2-(4-((2-Methoxy-6-(4-(trifluormethyl)phenyl)pyridin-3-yl)methoxy)phenoxy)ethansäure |
| Cpd 25: | 2-(4-((2-Phenylthio-6-(phenyl)pyridin-3-yl)methoxy)phenoxy)ethansäure |
| Cpd 26: | 2-(4-(((2-Methoxy-5-phenylpyridin-3-yl)methyl)amino)phenylthio)ethansäure |
| Cpd 27: | 2-(4-(((2-Methoxy-6-phenylpyridin-3-yl)methyl)amono)phenylthio)-2,2-difluorethansäure |
| Cpd 28: | 2-(4-(((2-Methoxy-5,6-diphenylpyridin-3-yl)methyl)amino)phenylthio)ethansäure |
| Cpd 29: | 2-(4-(((2-Methoxy-5-brom-6-phenylpyridin-3-yl)methyl)amino)phenylthio)ethansäure |
| Cpd 30: | 2-(4-(((2-Methoxy-6-furylpyridin-3-yl)methyl)amino)phenylthio)ethansäure |
| Cpd 31: | 3-(4-(((2-Methoxy-6-furylpyridin-3-yl)methyl)amino)phenyl)propansäure |
| Cpd 32: | 2-(4-(((2-Methoxy-6-phenylpyridin-3-yl)methyl)amino)phenylthio)-2-phenyl-ethansäure |
| Cpd 33: | 3-(4-(((2-Methoxy-6-phenylpyridin-3-yl)methyl)(methyl)amino)phenyl)propansäure |
| Cpd 34: | 3-(4-(1-((2-Methoxy-6-phenylpyridin-3-yl)propyl)amino)phenyl)proansäure |
| Cpd 35: | 2-(4-(((2-Methoxy-6-phenylpyridin-3-yl)methyl)amino)-2,6-dimethyl-phenoxy)ethansäure |
| Cpd 36: | 3-(4-(((2-Methoxy-6-(4-(trifluormethyl)phenyl)pyridin-3-yl)methyl)amino)phenyl)propansäure |
| Cpd 37: | 3-(4-((2-Methoxy-6-phenylpyridin-3-yl)methylthio)phenyl)propansäure |
| Cpd 38: | 3-(4-(((2-(Ethylthio)-6-phenylpyridin-3-yl)methyl)amino)phenyl)propansäure |
| Cpd 39: | 3-(4-(((2-Methoxy-6-(parabiphenyl)pyridin-3-yl)methyl)amino)phenyl)propansäure |
| Cpd 40: | 3-(4-(((2-Methoxy-6-(3-(trifluormethyl)phenyl)pyridin-3-yl)methyl)amino)phenyl)propansäure |
| Cpd 41: | 3-(4-(((2-Methoxy-5-phenylpyridin-3-yl)methyl)amino)phenyl)propansäure |
| Cpd 42: | 3-(4-((2-(Methoxy-6-phenylpyridin-3-yl)methyl)amino)phenyl)-3-phenyl-propansäure |
| Cpd 43: | 3-(2-Methoxy-4-(((2-methoxy-6-phenylpyridin-3-yl)methyl)amino)phenyl)propansäure |
| Cpd 44: | 3-(3-Methoxy-4-(((2-Methoxy-6-phenylpyridin-3-yl)methyl)amino)phenyl)propansäure |
| Cpd 45: | 3-(4-(((2-Methoxy-6-phenylpyridin-3-yl)methyl)amino)phenyl)butansäure |
| Cpd 46: | 3-(4-(((2-Methoxy-5-(4-trifluormethyl)phenyl)pyridin-3-yl)methyl)amino)phenyl)propansäure |
| Cpd 47: | 3-(4-(((2-Methoxy-5-(3-(trifluormethyl)phenyl)pyridin-3-yl)methyl)amino)phenyl)propansäure |
| Cpd 48: | 3-(4-(((2,6-Dimethoxy-5-phenylpyridin-3-yl)methyl)amino)phenyl)propansäure |
| Cpd 49: | 3-(4-(((5-(4-Chlorphenyl)-2-methoxypyridin-3-yl)methyl)amino)phenyl)propansäure |
| Cpd 50: | 3-(4-(((2-Methoxy-5-(naphtalen-2-yl)pyridin-3-yl)methyl)amino)phenyl)propansäure |
| Cpd 51: | 3-(4-(((2-Ethoxy-6-phenylpyridin-3-yl)methyl)amino)phenyl)propansäure |
| Cpd 52: | 3-(4-((2-Methoxy-5-phenylpyridin-3-yl)methoxy)amino)phenyl)hex-4-insäure |
| Cpd 53: | 3-(4-((2-Methoxy-6-phenylpyridin-3-yl)methoxy)amino)phenyl)hex-4-insäure |
| Cpd 54: | 3-(4-(((2-Isopropyloxy-6-phenylpyridin-3-yl)methyl)amino)phenyl)propansäure |

14. Pharmazeutische Zusammensetzung, umfassend in einem pharmazeutisch verträglichen Träger wenigstens eine Verbindung wie in einem der Ansprüche 1 bis 13 definiert, möglicherweise in Kombination mit einem oder mehreren weiteren therapeutischen und/oder kosmetischen Wirkstoffen.

15. Pharmazeutische Zusammensetzung gemäß Anspruch 14 zur therapeutischen, kurativen und/oder prophylaktischen Behandlung von Diabetes, Dyslipidämien, Insulinresistenz, Krankheiten, die mit dem metabolischen Syndrom assoziiert sind, Arteriosklerose, kardiovaskulären Krankheiten, Adipositas, Bluthochdruck und/oder Entzündungskrankheiten.

## Claims

1. A compound of general formula: 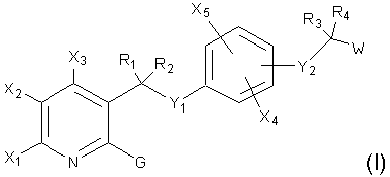 in which,
G represents:
- a radical -ORₐ, -SRₐ, or
- a radical -NRₐR_{b},
Rₐ being selected from an alkyl radical with 1 to 6 carbon atoms or alkenyl radical with 2 to 6 carbon atoms, a ring with 3 to 14 atoms, a phenyl radical, a phenylalkyl radical with the alkyl moiety having 1 to 3 carbon atoms;
R_{b} being selected from a hydrogen atom, an alkyl radical with 1 to 6 carbon atoms or alkenyl radical with 2 to 6 carbon atoms, a ring with 3 to 14 atoms, a phenyl radical, or a phenylalkyl radical with the alkyl moiety having 1 to 3 carbon atoms;
Rₐ and R_{b} can form, together and with the nitrogen atom to which they are bound, a heterocycle with 3 to 8 atoms.
R₁ and R₂, which may be identical or different, represent a hydrogen atom or an alkyl radical with 1 to 6 carbon atoms or alkenyl radical with 2 to 6 carbon atoms;
R₁ and R₂ can form, together and with the carbon atom to which they are bound, a carbocycle with 3 to 6 carbon atoms;
Y₁ represents:
- a oxygen atom or sulfur atom, or
- a group -NR-, in which R has the same definition as R_{b};
Y₂ represents:
- a oxygen atom or sulfur atom, or
- a radical -CR₅R₆-; with R₅ and R₆, which may be identical or different, selected from a hydrogen atom or halogen atom, an alkyl radical with 1 to 6 carbon atoms or an alkenyl or alkynyl radical with 2 to 6 carbon atoms, a ring with 3 to 6 atoms, a phenyl radical, a phenylalkyl radical with the alkyl moiety having 1 to 3 carbon atoms;
X₁, X₂, X₃ represent independently a hydrogen atom or halogen atom, an alkyl radical with 1 to 6 carbon atoms or alkenyl radical with 2 to 6 carbon atoms, a group -OR'ₐ, - SR'ₐ, -NR'ₐR'_{b}, a ring with 5 to 14 atoms, or a phenylalkyl radical with the alkyl moiety having 1 to 3 carbon atoms,
with at least one of the groups X₁, X₂ and X₃ different from a hydrogen atom and from a halogen atom;
R'ₐ and R'_{b}, which may be identical or different, having the same definitions as Rₐ and R_{b};
X₄ and X₅ represent independently a hydrogen atom or halogen atom, an alkyl radical with 1 to 6 carbon atoms or alkenyl radical with 2 to 6 carbon atoms, a group -OR"ₐ, - SR"ₐ or -NR"ₐR"_{b}, a ring with 3 to 14 atoms, a phenyl radical, or a phenylalkyl radical with the alkyl moiety having 1 to 3 carbon atoms;
R"ₐ and R"_{b}, which may be identical or different, having the same definitions as Rₐ and R_{b};
R₃ and R₄, which may be identical or different, represent a hydrogen atom or halogen atom, an alkyl radical with 1 to 6 carbon atoms or alkenyl radical with 2 to 6 carbon atoms, a ring with 3 to 14 atoms, a phenyl radical, or a phenylalkyl radical with the alkyl moiety having 1 to 3 carbon atoms;
W represents:
- a carboxyl radical -COOH; or
- a function derived from the carboxylic acid function, selected from
- COOR"'ₐ, -COSR"'ₐ, -CONR"'ₐR"'_{b}, -CSNR"'ₐR"'_{b}, -CONH₂; or
- a bioisosteric group of the carboxyl radical, selected from:
- an acylsulfonamide radical (-CONHSO₂R"'ₐ);
- a hydrazide radical (-CONHNR"'ₐR"'_{b});
- a radical selected from the thiazolidinedione, oxazolidinedione, tetrazole, oxadiazolone, triazolone, triazole, 3-alkyltriazole, or imidazolidinedione rings;
R"'ₐ and R"'_{b}, which may be identical or different, having the same definitions as Rₐ and R_{b}.

2. The compound as claimed in claim 1, **characterized in that** Y₂ is positioned para or meta of Y₁.

3. The compound as claimed in claim 1 or 2, **characterized in that**:
- X₃ denotes a hydrogen atom, and/or
- X₄ and X₅ denote independently a hydrogen atom, an alkyl radical having 1 to 6 carbon atoms, a group -OR"ₐ or -SR"ₐ; R"ₐ being an alkyl radical having 1 to 6 carbon atoms.

4. The compound as claimed in one of claims 1 to 3, **characterized in that** R₁, R₂, R₃, R₄ denote independently a hydrogen atom or a methyl, ethyl, propyl, butyl, isopropyl or tert-butyl radical.

5. The compound as claimed in one of claims 1 to 4, **characterized in that** X₃, R₁ and R₂ denote hydrogen atoms.

6. The compound as claimed in one of claims 1 to 5, **characterized in that** X₁ and/or X₂ denote a ring with 5 to 10 atoms, unsubstituted or substituted with a -CF₃ group.

7. The compound as claimed in one of claims 1 to 6, **characterized in that** G denotes
- a radical -ORₐ or -SRₐ, with Rₐ selected from alkyl radical having 1 to 6 carbon atoms, a cyclohexyl or a phenyl radical; or
- a radical -NRₐR_{b}, Rₐ and R_{b} forming together, and with the nitrogen atom to which they are bound, a heterocycle with 3 to 8 atoms.

8. The compound as claimed in one of claims 1 to 6, **characterized in that** G denotes a radical -ORₐ with Rₐ selected from a methyl, ethyl, propyl, butyl, isopropyl or tert-butyl radical.

9. The compound as claimed in claim 7 or 8, **characterized in that**:
- X₂ and X₃ denote simultaneously a hydrogen atom; or
- X₃ and X₁ denote simultaneously a hydrogen atom.

10. The compound as claimed in one of claims 1 to 9, **characterized in that** Y₁ denotes an oxygen or sulfur atom, and simultaneously Y₂ denotes an oxygen atom, a sulfur atom, or a group -CR₅R₆.

11. The compound as claimed in one of claims 1 to 9, **characterized in that** Y₁ denotes a group -NR and simultaneously Y₂ denotes an oxygen atom, a sulfur atom or a group - CR₅R₆.

12. The compound as claimed in claim 10 or 11, **characterized in that** X₁ denotes an unsubstituted phenyl radical or a phenyl radical substituted with a group -CF₃, said group -CF₃ being preferably in para of the pyridinyl radical, and/or G denotes a group -OCH₃ or -OC(CH₃)₃.

13. The compound as claimed in one of claims 1 to 12, **characterized in that** it is selected from:
| | |
|---|---|
| Cpd 1: | 2-(4-((2-methoxy-6-phenylpyridin-3-yl)methoxy)phenoxy)-2-methyl-propanoic acid |
| Cpd 2: | 2-(4-((2-methoxy-6-phenylpyridin-3-yl)methoxy)phenoxy)ethanoic acid |
| Cpd 3: | 2-(4-((2-methoxy-6-phenylpyridin-3-yl)methoxy)phenoxy)propanoic acid |
| Cpd 4: | 2-(4-(((2-methoxy-6-phenylpyridin-3yl)methyl)amino)phenoxy)ethanoic acid |
| Cpd 5: | 2-(4-(((2-methoxy-6-phenylpyridin-3yl)methyl)amino)phenoxy)propanoic acid |
| Cpd 6: | 2-(4-((2-tert-butyloxy-6-phenylpyridin-3-yl)methoxy)phenoxy)-2-methyl-propanoic acid |
| Cpd 7: | 2-(4-(((2-tert-butyloxy-6-phenylpyridin-3-yl)methoxy)phenoxy)ethanoic acid |
| Cpd 8: | 2-(4-((2-tert-butyloxy-6-phenylpyridin-3-yl)methyl)amino) phenoxy)ethanoic acid |
| Cpd 9: | 2-(4-(((2-tert-butyloxy-6-phenylpyridin-3-yl)methyl)amino)phenoxy)-2-methyl-propanoic acid |
| Cpd 10: | 2-(4-(((2-tert-butyloxy-6-phenylpyridin-3-yl)methyl)amino) phenoxy)-propanoic acid |
| Cpd 11: | 2-(4-(((2-methoxy-6-phenylpyridin-3-yl)methyl)amino)phenylthio)-2-methyl-propanoic acid |
| Cpd 12: | 2-(4-(((2-methoxy-6-phenylpyridin-3-yl)methyl)amino)phenoxy)-2-methyl-propanoic acid |
| Cpd 13: | 2-(3-(((2-methoxy-6-phenylpyridin-3-yl)methyl)amino)phenoxy)-2-methyl-propanoic acid |
| Cpd 14: | 2-(3-(((2-methoxy-6-phenylpyridin-3-yl)methyl)amino)phenoxy)ethanoic acid |
| Cpd 15: | 2-(4-((2-hexyloxy-6-phenylpyridin-3-yl)methoxy)phenoxy)ethanoic acid |
| Cpd 16: | 2-(4-(((2-methoxy-6-phenylpyridin-3-yl)methyl)amino)phenylthio)-ethanoic acid |
| Cpd 17: | 2-(4-((2-hexyloxy-6-phenylpyridin-3-yl)methoxy)phenoxy)-2-methyl-propanoic acid |
| Cpd 18: | 2-(4-((2-cyclohexyloxy-6-phenylpyridin-3-yl)methoxy)phenoxy)ethanoic acid |
| Cpd 19: | 3-(4-(((2-methoxy-6-phenylpyridin-3-yl)methyl)amino)phenyl)propanoic acid |
| Cpd 20: | 2-(4-((6-phenyl-2-(piperidin-1-yl)pyridin-3-yl)methoxy)phenoxy)-ethanoic acid |
| Cpd 21: | 2-(4-((2-methoxy-6-(4-(trifluoromethyl)phenyl)pyridin-3-yl)methoxy) - phenoxy)-2-methylpropanoic acid |
| Cpd 22: | 2-(4-(((2-methoxy-6-(4-(trifluoromethyl)phenyl)pyridin-3-yl)methyl) amino)-phenylthio)-2-methylpropanoic acid |
| Cpd 23: | 2-(4-(((2-methoxy-6-(4-(trifluoromethyl)phenyl)pyridin-3-yl)methyl) amino)-phenylthio)ethanoic acid |
| Cpd 24: | 2-(4-((2-methoxy-6-(4-(trifluoromethyl)phenyl)pyridin-3-yl)methoxy) - phenoxy)ethanoic acid |
| Cpd 25: | 2-(4-((2-phenylthio-6-(phenyl)pyridin-3-yl)methoxy)phenoxy)ethanoic acid |
| Cpd 26: | 2-(4-(((2-methoxy-5-phenylpyridin-3-yl)methyl)amino)phenylthio)-ethanoic acid |
| Cpd 27: | 2-(4-(((2-methoxy-6-phenylpyridin-3-yl)methyl)amino)phenylthio)-2,2-difluoroethanoic acid |
| Cpd 28: | 2-(4-(((2-methoxy-5,6-diphenylpyridin-3-yl)methyl)amino)phenylthio)-ethanoic acid |
| Cpd 29: | 2-(4-(((2-methoxy-5-bromo-6-phenylpyridin-3-yl)methyl)amino)-phenylthio)-ethanoic acid |
| Cpd 30: | 2-(4-(((2-methoxy-6-furylpyridin-3-yl)methyl)amino)phenylthio)ethanoic acid |
| Cpd 31: | 3-(4-(((2-methoxy-6-furylpyridin-3-yl)methyl)amino)phenyl)propanoic acid |
| Cpd 32: | 2-(4-(((2-methoxy-6-phenylpyridin-3-yl)methyl)amino)phenylthio)-2-phenyl-ethanoic acid |
| Cpd 33: | 3-(4-(((2-methoxy-6-phenylpyridin-3-yl)methyl)(methyl)amino)phenyl)-propanoic acid |
| Cpd 34: | 3-(4-(1-((2-methoxy-6-phenylpyridin-3-yl)propyl)amino)phenyl)propanoic acid |
| Cpd 35: | 2-(4-(((2-methoxy-6-phenylpyridin-3-yl)methyl)amino)-2,6-dimethyl-phenoxy)ethanoic acid |
| Cpd 36: | 3-(4-(((2-methoxy-6-(4-(trifluoromethyl)phenyl)pyridin-3-yl)methyl) amino)-phenyl)-propanoic acid |
| Cpd 37: | 3-(4-((2-methoxy-6-phenylpyridin-3-yl)methylthio)phenyl) propanoic acid |
| Cpd 38: | 3-(4-(((2-(ethylthio)-6-phenylpyridin-3-yl)methyl)amino)phenyl)-propanoic acid |
| Cpd 39: | 3-(4-(((2-methoxy-6-(parabiphenyl)pyridin-3-yl)methyl)amino)phenyl)-propanoic acid |
| Cpd 40: | 3-(4-(((2-methoxy-6-(3-(trifluoromethyl)phenyl)pyridin-3-yl)methyl)-amino)-phenyl)propanoic acid |
| Cpd 41: | 3-(4-(((2-methoxy-5-phenylpyridin-3-yl)methyl)amino)phenyl)propanoic acid |
| Cpd 42: | 3-(4-((2(-methoxy-6-phenylpyridin-3-yl)methyl)amino)phenyl)-3-phenyl-propanoic acid |
| Cpd 43: | 3-(2-methoxy-4-(((2-methoxy-6-phenylpyridin-3-yl)methyl)amino)-phenyl)-propanoic acid |
| Cpd 44: | 3-(3-methoxy-4-(((2-methoxy-6-phenylpyridin-3-yl)methyl)amino)-phenyl)-propanoic acid |
| Cpd 45: | 3-(4-(((2-methoxy-6-phenylpyridin-3-yl)methyl)amino)phenyl)butanoic acid |
| Cpd 46: | 3-(4-(((2-methoxy-5-(4-(trifluoromethyl)phenyl)pyridin-3-yl)methyl)-amino)phenyl)propanoic acid |
| Cpd 47: | 3-(4-(((2-methoxy-5-(3-(trifluoromethyl)phenyl)pyridin-3-yl)methyl)-amino)-phenyl)propanoic acid |
| Cpd 48: | 3-(4-(((2,6-dimethoxy-5-phenylpyridin-3-yl)methyl)amino)phenyl)-propanoic acid |
| Cpd 49: | 3-(4-(((5-(4-chlorophenyl)-2-methoxypyridin-3-yl)methyl)amino)phenyl)-propanoic acid |
| Cpd 50: | 3-(4-(((2-methoxy-5-(naphthalen-2-yl)pyridin-3-yl)methyl)amino)phenyl)-propanoic acid |
| Cpd 51: | 3-(4-(((2-ethoxy-6-phenylpyridin-3-yl)methyl)amino)phenyl)propanoic acid |
| Cpd 52: | 3-(4-((2-methoxy-5-phenylpyridin-3-yl)methoxy)phenyl)hex-4-ynoic acid |
| Cpd 53: | 3-(4-((2-methoxy-6-phenylpyridin-3-yl)methoxy)phenyl)hex-4-ynoic acid |
| Cpd 54: | 3-(4-(((2-isopropyloxy-6-phenylpyridin-3-yl)methyl)amino)phenyl)-propanoic acid. |

14. A pharmaceutical composition comprising, in a pharmaceutically acceptable carrier, at least one compound as defined in one of claims 1 to 13, optionally in combination with one or more other therapeutic and/or cosmetic active substances.

15. The pharmaceutical composition as claimed in claim 14 for the therapeutic, curative and/or prophylactic treatment of diabetes, dyslipidaemias, insulin resistance, pathologies associated with metabolic syndrome, atherosclerosis, cardiovascular diseases, obesity, hypertension and/or inflammatory diseases.


### SEQUENCE LISTING

<110> GENFIT
<120> COMPOSES AGONISTES PPAR, PREPARATION ET UTILISATIONS
<130> B724WO00
<160> 12
<170> PatentIn version 3.3
<210> 1
   <211> 22
   <212> ADN
   <213> Artificielle
<220>
   <223> Amorce sens PDK4
<400> 1
   tactccactg ctccaacacc tg 22
<210> 2
   <211> 20
   <212> ADN
   <213> Artificielle
<220>
   <223> Amorce antisens PDK4
<400> 2
   gttcttcggt tccctgcttg 20
<210> 3
   <211> 20
   <212> ADN
   <213> Artificielle
<220>
   <223> Amorce sens ACO
<400> 3
   gaagccagcg ttacgaggtg 20
<210> 4
   <211> 20
   <212> ADN
   <213> Artificielle
<220>
   <223> Amorce antisens ACO
<400> 4
   tggagttctt gggacgggtg 20
<210> 5
   <211> 20
   <212> ADN
   <213> Artificielle
<220>
   <223> Amorce sens ApoCIII
<400> 5
   ctcttggctc tcctggcatc 20
<210> 6
   <211> 20
   <212> ADN
   <213> Artificielle
<220>
   <223> Amorce antisens ApoCIII
<400> 6
   gcatcctgga ccgtcttgga 20
<210> 7
   <211> 22
   <212> ADN
   <213> Artificielle
<220>
   <223> Amorce sens CPT1b
<400> 7
   ggactgagac tgtgcgttcc tg 22
<210> 8
   <211> 20
   <212> ADN
   <213> Artificielle
<220>
   <223> Amorse antisens CPT1b
<400> 8
   agtgcttggc ggatgtggtt 20
<210> 9
   <211> 24
   <212> ADN
   <213> Artificielle
<220>
   <223> Amorce sens UCP2
<400> 9
   gtcggagata ccagagcact gtcg 24
<210> 10
   <211> 20
   <212> ADN
   <213> Artificielle
<220>
   <223> Amorce antisens UCP2
<400> 10
   cacatcaaca ggggaggcga 20
<210> 11
   <211> 25
   <212> ADN
   <213> Artificielle
<220>
   <223> Amorce sens 36B4
<400> 11
   catgctcaac atctccccct tctcc 25
<210> 12
   <211> 25
   <212> ADN
   <213> Artificielle
<220>
   <223> Amorce antisens 36B4
<400> 12
   gggaaggtgt aatccgtctc cacag 25